# EUROPEAN PATENT SPECIFICATION

(11) **EP 3 620 460 B1**
(45) Date of publication and mention of the grant of the patent: **16.08.2023**
(21) Application number: 19195090.6
(22) Date of filing: 03.09.2019
(51) Int. Cl.: C07F 15/00, H10K 85/30, C09K 11/06

(54) **ORGANOMETALLIC COMPOUND, ORGANIC LIGHT-EMITTING DEVICE INCLUDING THE SAME, AND DIAGNOSTIC COMPOSITION INCLUDING THE ORGANOMETALLIC COMPOUND**
ORGANOMETALLISCHE VERBINDUNG, ORGANISCHE LICHTEMITTIERENDE VORRICHTUNG DAMIT UND DIAGNOSEZUSAMMENSETZUNG MIT DER ORGANOMETALLISCHEN VERBINDUNG
COMPOSÉ ORGANOMÉTALLIQUE, DISPOSITIF ÉLECTROLUMINESCENT ORGANIQUE LE COMPRENANT ET COMPOSITION DE DIAGNOSTIC COMPRENANT LE COMPOSÉ ORGANOMÉTALLIQUE

(30) Priority: 05.09.2018 KR 20180106188
(43) Date of publication of application: 11.03.2020
(73) Proprietor: Samsung Electronics Co., Ltd., Gyeonggi-do 16677 (KR)
(72) Inventor: CHOI, Jongwon, 16678 Gyeonggi-do (KR); CHO, Yongsuk, 16678 Gyeonggi-do (KR); KWAK, Yoonhyun, 16678 Gyeonggi-do (KR); KOO, Hyun, 16678 Gyeonggi-do (KR); KWON, Ohyun, 16678 Gyeonggi-do (KR); KIM, Soyeon, 16678 Gyeonggi-do (KR); ARATANI, Sukekazu, 16678 Gyeonggi-do (KR); LEE, Jiyoun, 16678 Gyeonggi-do (KR); IM, Kyuhyun, 16678 Gyeonggi-do (KR); KRAVCHUK, Dmitry, 16678 Gyeonggi-do (KR)
(74) Representative: Elkington and Fife LLP

(56) References cited:
- WO-A1-2018/124697

## Description

### FIELD OF THE INVENTION

One or more embodiments relate to an organometallic compound, an organic light-emitting device including the organometallic compound, and a diagnostic composition including the organometallic compound.

### BACKGROUND OF THE INVENTION

Organic light-emitting devices are self-emission devices, which have better characteristics in terms of a viewing angle, response time, brightness, driving voltage, and response speed, and produce full-color images.

In an example, an organic light-emitting device includes an anode, a cathode, and an organic layer between the anode and the cathode, wherein the organic layer includes an emission layer. A hole transport region may be between the anode and the emission layer, and an electron transport region may be between the emission layer and the cathode. Holes provided from the anode may move toward the emission layer through the hole transport region, and electrons provided from the cathode may move toward the emission layer through the electron transport region. The holes and the electrons recombine in the emission layer to produce excitons. These excitons transit from an excited state to a ground state, thereby generating light.

Meanwhile, luminescent compounds may be used to monitor, sense, or detect a variety of biological materials including cells and proteins. An example of the luminescent compounds is a phosphorescent luminescent compound.

WO 2018/124697 discloses organic electroluminescent compounds of the below formula (1) and devices including the same:

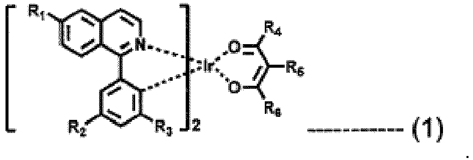

### SUMMARY OF THE INVENTION

One or more embodiments relate to a novel organometallic compound and an organic light-emitting device and a diagnostic composition including the same.

Additional aspects will be set forth in part in the description which follows and, in part, will be apparent from the description, or may be learned by practice of the presented embodiments.

An aspect of the present disclosure provides an organometallic compound represented by Formula 1 below: 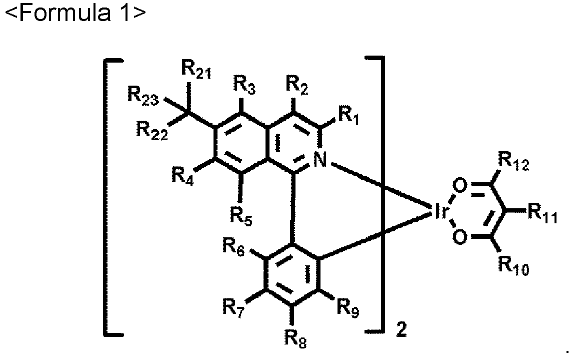 In Formula 1,
R₁ to R₁₂ and R₂₃ may each independently be hydrogen, deuterium, -F, -Cl, -Br, -I, - SF₅, a hydroxyl group, a cyano group, a nitro group, an amino group, an amidino group, a hydrazino group, a hydrazono group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -N(Q₁)(Q₂), -Si(Q₃)(Q₄)(Q₅), -Ge(Q₃)(Q₄)(Q₅), -B(Q₆)(Q₇), -P(=O)(Q₈)(Q₉), or -P(Q₈)(Q₉),
at least one of R₁ to R₅ may each independently be deuterium, -F, -Cl, -Br, -I, -SF₅, a hydroxyl group, a cyano group, a nitro group, an amino group, an amidino group, a hydrazino group, a hydrazono group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -N(Q₁)(Q₂), -Si(Q₃)(Q₄)(Q₅), -Ge(Q₃)(Q₄)(Q₅), -B(Q₆)(Q₇), -P(=O)(Q₈)(Q₉), or -P(Q₈)(Q₉),
R₂₁ may be a C₂-C₂₀ alkyl group, a deuterium-containing C₂-C₂₀ alkyl group, a C₃-C₁₀ cycloalkyl group, or a deuterium-containing C₃-C₁₀ cycloalkyl group,
R₂₂ may be a C₁-C₂₀ alkyl group, a deuterium-containing C₁-C₂₀ alkyl group, a C₃-C₁₀ cycloalkyl group, or a deuterium-containing C₃-C₁₀ cycloalkyl group,
two or more of R₁ to R₉ may optionally be linked to each other to form a C₅-C₃₀ carbocyclic group unsubstituted or substituted with at least one R₁ₐ or a C₂-C₃₀ heterocyclic group unsubstituted or substituted with at least one R₁ₐ,
two or more of R₁₀ to R₁₂ may optionally be linked to each other to form a C₅-C₃₀ carbocyclic group unsubstituted or substituted with at least one R₁ₐ or a C₂-C₃₀ heterocyclic group unsubstituted or substituted with at least one R₁ₐ,
R₁ₐ is the same as defined in connection with R₁,
a substituent(s) of the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₁-C₆₀ heteroaryl group, the substituted monovalent non-aromatic condensed polycyclic group, and the substituted monovalent non-aromatic condensed heteropolycyclic group may be each independently:
   deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazino group, a hydrazono group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, or a C₁-C₆₀ alkoxy group;
   a C₁-C₆₀ alkyl group, a C₂-C₆₀ alkenyl group, a C₂-C₆₀ alkynyl group, or a C₁-C₆₀ alkoxy group, each substituted with deuterium, -F, -Cl, -Br, -I, -CD₃, -CD₂H, -CDH₂, -CF₃, - CF₂H, -CFH₂, a hydroxyl group, a cyano group, a nitro group, an amino group, an amidino group, a hydrazino group, a hydrazono group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₁₁)(Q₁₂), - Si(Q₁₃)(Q₁₄)(Q₁₅), -Ge(Q₁₃)(Q₁₄)(Q₁₅), -B(Q₁₆)(Q₁₇), -P(=O)(Q₁₈)(Q₁₉), -P(Q₁₈)(Q₁₉), or any combination thereof;
   a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, or a monovalent non-aromatic condensed heteropolycyclic group, each unsubstituted or substituted with deuterium, -F, -Cl, -Br, -I, -CD₃, -CD₂H, -CDH₂, -CF₃, - CF₂H, -CFH₂, a hydroxyl group, a cyano group, a nitro group, an amino group, an amidino group, a hydrazino group, a hydrazono group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₂₁)(Q₂₂), - Si(Q₂₃)(Q₂₄)(Q₂₅), -Ge(Q₂₃)(Q₂₄)(Q₂₅), -B(Q₂₆)(Q₂₇), -P(=O)(Q₂₈)(Q₂₉), -P(Q₂₈)(Q₂₉), or any combination thereof;
   -N(Q₃₁)(Q₃₂), -Si(Q₃₃)(Q₃₄)(Q₃₅), -Ge(Q₃₃)(Q₃₄)(Q₃₅), -B(Q₃₆)(Q₃₇), -P(=O)(Q₃₈)(Q₃₉) or -P(Q₃₈)(Q₃₉); or
   any combination thereof; and
   Q₁ to Q₉, Q₁₁ to Q₁₉, Q₂₁ to Q₂₉, and Q₃₁ to Q₃₉ may each independently be hydrogen; deuterium; -F; -Cl; -Br; -I; a hydroxyl group; a cyano group; a nitro group; an amino group; an amidino group; a hydrazine group; a hydrazone group; a carboxylic acid group or a salt thereof; a sulfonic acid group or a salt thereof; a phosphoric acid group or a salt thereof; a C₁-C₆₀ alkyl group unsubstituted or substituted with deuterium, a C₁-C₆₀ alkyl group, a C₆-C₆₀ aryl group, or any combination thereof; a C₂-C₆₀ alkenyl group; a C₂-C₆₀ alkynyl group; a C₁-C₆₀ alkoxy group; a C₃-C₁₀ cycloalkyl group; a C₁-C₁₀ heterocycloalkyl group; a C₃-C₁₀ cycloalkenyl group; a C₁-C₁₀ heterocycloalkenyl group; a C₆-C₆₀ aryl group unsubstituted or substituted with a C₁-C₆₀ alkyl group, a C₆-C₆₀ aryl group, or any combination thereof; a C₆-C₆₀ aryloxy group; a C₆-C₆₀ arylthio group; a C₁-C₆₀ heteroaryl group; a monovalent non-aromatic condensed polycyclic group; or a monovalent non-aromatic condensed heteropolycyclic group.

Another aspect of the present disclosure provides an organic light-emitting device including: a first electrode, a second electrode, and an organic layer disposed between the first electrode and the second electrode and including an emission layer, wherein the organic layer includes at least one the organometallic compound represented by Formula 1.

The organometallic compound represented by Formula 1 included in the emission layer in the organic layer may act as a dopant.

### BRIEF DESCRIPTION OF THE DRAWING

These and/or other aspects will become apparent and more readily appreciated from the following description of the embodiments, taken in conjunction with FIGURE which is a schematic view of an organic light-emitting device according to an embodiment.

### DETAILED DESCRIPTION OF THE EMBODIMENTS

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. As used herein, the term "and/or" includes any and all combinations of one or more of the associated listed items. Expressions such as "at least one of," when preceding a list of elements, modify the entire list of elements and do not modify the individual elements of the list.
It will be understood that when an element is referred to as being "on" another element, it can be directly on the other element or intervening elements may be present therebetween. In contrast, when an element is referred to as being "directly on" another element, there are no intervening elements present.
It will be understood that, although the terms "first," "second," "third" etc. may be used herein to describe various elements, components, regions, layers and/or sections, these elements, components, regions, layers and/or sections should not be limited by these terms. These terms are only used to distinguish one element, component, region, layer, or section from another element, component, region, layer, or section. Thus, "a first element," "component," "region," "layer," or "section" discussed below could be termed a second element, component, region, layer, or section without departing from the teachings herein. The terminology used herein is for the purpose of describing particular embodiments only and is not intended to be limiting. As used herein, the singular forms "a," "an," and "the" are intended to include the plural forms, including "at least one," unless the content clearly indicates otherwise. "Or" means "and/or." It will be further understood that the terms "comprises" and/or "comprising," or "includes" and/or "including" when used in this specification, specify the presence of stated features, regions, integers, steps, operations, elements, and/or components, but do not preclude the presence or addition of one or more other features, regions, integers, steps, operations, elements, components, and/or groups thereof.
Furthermore, relative terms, such as "lower" or "bottom" and "upper" or "top," may be used herein to describe one element's relationship to another element as illustrated in the
Figures. It will be understood that relative terms are intended to encompass different orientations of the device in addition to the orientation depicted in the Figures. For example, if the device in one of the figures is turned over, elements described as being on the "lower" side of other elements would then be oriented on "upper" sides of the other elements. The exemplary term "lower," can therefore, encompasses both an orientation of "lower" and "upper," depending on the particular orientation of the figure. Similarly, if the device in one of the figures is turned over, elements described as "below" or "beneath" other elements would then be oriented "above" the other elements. The exemplary terms "below" or "beneath" can, therefore, encompass both an orientation of above and below.
"About" or "approximately" as used herein is inclusive of the stated value and means within an acceptable range of deviation for the particular value as determined by one of ordinary skill in the art, considering the measurement in question and the error associated with measurement of the particular quantity (i.e., the limitations of the measurement system). For example, "about" can mean within one or more standard deviations, or within ± 30%, 20%, 10%, or 5% of the stated value.
Unless otherwise defined, all terms (including technical and scientific terms) used herein have the same meaning as commonly understood by one of ordinary skill in the art to which this disclosure belongs. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

Exemplary embodiments are described herein with reference to cross section illustrations that are schematic illustrations of idealized embodiments. As such, variations from the shapes of the illustrations as a result, for example, of manufacturing techniques and/or tolerances, are to be expected. Thus, embodiments described herein should not be construed as limited to the particular shapes of regions as illustrated herein but are to include deviations in shapes that result, for example, from manufacturing. For example, a region illustrated or described as flat may, typically, have rough and/or nonlinear features. Moreover, sharp angles that are illustrated may be rounded. Thus, the regions illustrated in the figures are schematic in nature and their shapes are not intended to illustrate the precise shape of a region and are not intended to limit the scope of the present claims.

An aspect of the present disclosure provides an organometallic compound represented by Formula 1 below:

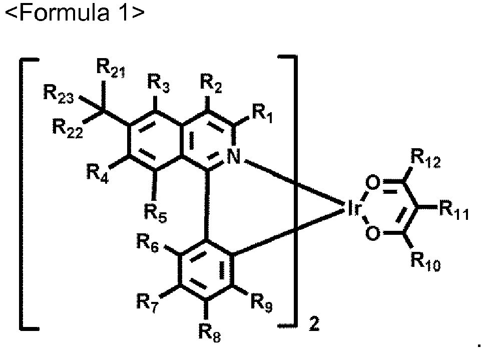

In Formula 1, R₁ to R₁₂ and R₂₃ may each independently be hydrogen, deuterium, - F, -Cl, -Br, -I, -SF₅, a hydroxyl group, a cyano group, a nitro group, an amino group, an amidino group, a hydrazino group, a hydrazono group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -N(Q₁)(Q₂), -Si(Q₃)(Q₄)(Q₅), -Ge(Q₃)(Q₄)(Q₅), -B(Q₆)(Q₇), -P(=O)(Q₈)(Q₉), or -P(Q₈)(Q₉), wherein Q₁ to Q₉ will be each understood by referring to a detailed description thereof provided below.

In Formula 1, at least one of R₁ to R₅ (for example, one, two, three, four, or five of R₁ to R₅) may not be hydrogen. For example, at least one of R₁ to R₅ in Formula 1 (for example, one, two, three, four, or five of R₁ to R₅) may each independently be deuterium, - F, -Cl, -Br, -I, -SF₅, a hydroxyl group, a cyano group, a nitro group, an amino group, an amidino group, a hydrazino group, a hydrazono group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -N(Q₁)(Q₂), -Si(Q₃)(Q₄)(Q₅), -Ge(Q₃)(Q₄)(Q₅), -B(Q₆)(Q₇), -P(=O)(Q₈)(Q₉), or -P(Q₈)(Q₉).

In one embodiment, in Formula 1,
R₁ may not be hydrogen and R₂, R₃, R₄ and R₅ may each independently hydrogen or deuterium;
R₂ may not be hydrogen and R₁, R₂, R₄ and R₅ may each independently hydrogen or deuterium;
R₃ may not be hydrogen and R₁, R₂, R₄ and R₅ may each independently hydrogen or deuterium;
R₄ may not be hydrogen and R₁, R₂, R₃ and R₅ may each independently hydrogen or deuterium; or
R₅ may not be hydrogen and R₁, R₂, R₃ and R₄ may each independently hydrogen or deuterium.

For example, in Formula 1,
R₁ to R₁₂ and R₂₃ may each independently be:hydrogen, deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, -SF₅, a C₁-C₂₀ alkyl group, or a C₁-C₂₀ alkoxy group;
a C₁-C₂₀ alkyl group or a C₁-C₂₀ alkoxy group, each substituted with deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, a C₁-C₂₀ alkoxy group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a phenyl group, a (C₁-C₂₀ alkyl)phenyl group, a biphenyl group, a terphenyl group, a naphthyl group, a pyridinyl group, a pyrimidinyl group, or a combination thereof;
a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a phenyl group, a (C₁-C₂₀ alkyl)phenyl group, a biphenyl group, a terphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group. or an imidazopyrimidinyl group, each unsubstituted or substituted with deuterium, -F, -Cl, -Br, -I, -CD₃, -CD₂H, - CDH₂, -CF₃, -CF₂H, -CFH₂, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, a C₁-C₂₀ alkoxy group, a cyclopentyl group, a cyclohexyl group, a cycloheptyl group, a cycloctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a phenyl group, a (C₁-C₂₀ alkyl)phenyl group, a biphenyl group, a terphenyl group, a naphthyl group, a fluorenyl group, a phenanthrenyl group, an anthracenyl group, a fluoranthenyl group, a triphenylenyl group, a pyrenyl group, a chrysenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a quinoxalinyl group, a quinazolinyl group, a cinnolinyl group, a carbazolyl group, a phenanthrolinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a benzocarbazolyl group, a dibenzocarbazolyl group, an imidazopyridinyl group, an imidazopyrimidinyl group, or any combination thereof; or
-N(Q₁)(Q₂), -Si(Q₃)(Q₄)(Q₅), -Ge(Q₃)(Q₄)(Q₅), -B(Q₆)(Q₇), or -P(=O)(Q₈)(Q₉);
at least one of R₁ to R₅ may not be hydrogen, and
Q₁ to Q₉ may each independently be:
   -CH₃, -CD₃, -CD₂H, -CDH₂, -CH₂CH₃, -CH₂CD₃, -CH₂CD₂H, -CH₂CDH₂, -CHDCH₃, -CHDCD₂H, -CHDCDH₂, -CHDCD₃, -CD₂CD₃, -CD₂CD₂H, or -CD₂CDH₂; or
   an n-propyl group, an isopropyl group, an n-butyl group, a sec-butyl group, an isobutyl group, a tert-butyl group, an n-pentyl group, a tert-pentyl group, a neopentyl group, an isopentyl group, a sec-pentyl group, a 3-pentyl group, a sec-isopentyl group, a phenyl group, a biphenyl group, or a naphthyl group, each unsubstituted or substituted with deuterium, a C₁-C₁₀ alkyl group, a phenyl group, or any combination thereof.

In one embodiment, in Formula 1,
R₁ to R₁₂ and R₂₃ may each independently be:
hydrogen, deuterium, -F, a cyano group, or a C₁-C₂₀ alkyl group;
a C₁-C₂₀ alkyl group substituted with deuterium, -F, -CD₃, -CD₂H, -CDH₂, -CF₃, - CF₂H, -CFH₂, a cyano group, a C₁-C₁₀ alkyl group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a phenyl group, a (C₁-C₂₀ alkyl)phenyl group, a biphenyl group, a terphenyl group, or a combination thereof;
a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a phenyl group, a (C₁-C₂₀ alkyl)phenyl group, a biphenyl group, or a terphenyl group, each unsubstituted or substituted with deuterium, -F, -CD₃, -CD₂H, -CDH₂, -CF₃, -CF₂H, -CFH₂, a cyano group, a C₁-C₁₀ alkyl group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a phenyl group, a (C₁-C₂₀ alkyl)phenyl group, a biphenyl group, a terphenyl group, or any combination thereof; or
-Si(Q₃)(Q₄)(Q₅), or -Ge(Q₃)(Q₄)(Q₅); and/or
each of at least one of R₁ to R₅ may each independently be:
   deuterium, -F, a cyano group, or a C₁-C₂₀ alkyl group;
   a C₁-C₂₀ alkyl group substituted with deuterium, -F, -CD₃, -CD₂H, -CDH₂, -CF₃, - CF₂H, -CFH₂, a cyano group, a C₁-C₁₀ alkyl group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a phenyl group, a (C₁-C₂₀ alkyl)phenyl group, a biphenyl group, a terphenyl group, or a combination thereof;
   a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a phenyl group, a (C₁-C₂₀ alkyl)phenyl group, a biphenyl group, or a terphenyl group, each unsubstituted or substituted with deuterium, -F, -CD₃, -CD₂H, -CDH₂, -CF₃, -CF₂H, -CFH₂, a cyano group, a C₁-C₁₀ alkyl group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a phenyl group, a (C₁-C₂₀ alkyl)phenyl group, a biphenyl group, a terphenyl group, or any combination thereof; or
   -Si(Q₃)(Q₄)(Q₅), or -Ge(Q₃)(Q₄)(Q₅), wherein Q₃ to Q₅ may be each understood by referring to a detailed description therein.

In Formula 1, R₂₁ may be a C₂-C₂₀ alkyl group, a deuterium-containing C₂-C₂₀ alkyl group, a C₃-C₁₀ cycloalkyl group, or a deuterium-containing C₃-C₁₀ cycloalkyl group, and R₂₂ may be a C₁-C₂₀ alkyl group, a deuterium-containing C₁-C₂₀ alkyl group, a C₃-C₁₀ cycloalkyl group, or a deuterium-containing C₃-C₁₀ cycloalkyl group.

For example, R₂₁ in Formula 1 may be an ethyl group, an n-propyl group, an isopropyl group, an n-butyl group, a sec-butyl group, an isobutyl group, a tert-butyl group, an n-pentyl group, a tert-pentyl group, a neopentyl group, an isopentyl group, a sec-pentyl group, a 3-pentyl group, a sec-isopentyl group, an n-hexyl group, an isohexyl group, a sec-hexyl group, a tert-hexyl group, an n-heptyl group, an isoheptyl group, a sec-heptyl group, a tert-heptyl group, an n-octyl group, an isooctyl group, a sec-octyl group, a tert-octyl group, an n-nonyl group, an isononyl group, a sec-nonyl group, a tert-nonyl group, an n-decyl group, an isodecyl group, a sec-decyl group, a tert-decyl group, a cyclopentyl group, a cyclohexyl group, a cycloheptyl group, a cyclooctyl group, a bicyclo[1.1.1]pentyl group, a bicyclo[2.1.1]hexyl group, a bicyclo[2.2.1]heptyl group, or a bicyclo[2.2.2]octyl group, each unsubstituted or substituted with at least one deuterium.

For example, R₂₂ in Formula 1 may be a methyl group, an ethyl group, an n-propyl group, an isopropyl group, an n-butyl group, a sec-butyl group, an isobutyl group, a tert-butyl group, an n-pentyl group, a tert-pentyl group, a neopentyl group, an isopentyl group, a sec-pentyl group, a 3-pentyl group, a sec-isopentyl group, an n-hexyl group, an isohexyl group, a sec-hexyl group, a tert-hexyl group, an n-heptyl group, an isoheptyl group, a sec-heptyl group, a tert-heptyl group, an n-octyl group, an isooctyl group, a sec-octyl group, a tert-octyl group, an n-nonyl group, an isononyl group, a sec-nonyl group, a tert-nonyl group, an n-decyl group, an isodecyl group, a sec-decyl group, a tert-decyl group, a cyclopentyl group, a cyclohexyl group, a cycloheptyl group, a cyclooctyl group, a bicyclo[1.1.1]pentyl group, a bicyclo[2.1.1]hexyl group, a bicyclo[2.2.1]heptyl group, or a bicyclo[2.2.2]octyl group, each unsubstituted or substituted with at least one deuterium.

In one or more embodiments, one of R₁ to R₅ in Formula 1 may be a substituted or unsubstituted C₁-C₆₀ alkyl group, a substituted or unsubstituted C₃-C₁₀ cycloalkyl - Si(Q₃)(Q₄)(Q₅), or -Ge(Q₃)(Q₄)(Q₅).

In one or more embodiments, in Formula 1, R₆ and R₈ may each independently be hydrogen or deuterium, R₇ and R₉ may each independently be a substituted or unsubstituted C₁-C₆₀ alkyl group, a substituted or unsubstituted C₃-C₁₀ cycloalkyl group, - Si(Q₃)(Q₄)(Q₅), or -Ge(Q₃)(Q₄)(Q₅).

In one or more embodiments, in Formula 1, R₇ and R₉ may each independently be a substituted or unsubstituted C₁-C₆₀ alkyl group, and R₇ and R₉ may be identical to each other.

In one or more embodiments, in Formula 1, R₇ and R₉ may each independently be a substituted or unsubstituted C₁-C₆₀ alkyl group, and R₇ and R₉ may be different from each other.

In one or more embodiments, in Formula 1, R₇ and R₉ may each independently be a substituted or unsubstituted C₁-C₆₀ alkyl group, R₇ and R₉ may be different from each other, and the number of carbon atoms included in R₇ may be greater than that of carbon atoms included in R₉.

In one or more embodiments, in Formula 1, R₂₁ may be a C₃-C₁₀ cycloalkyl group, or a deuterium-containing C₃-C₁₀ cycloalkyl group.

In one or more embodiments, in Formula 1, R₂₁ may be a C₃-C₂₀ alkyl group, a deuterium-containing C₃-C₂₀ alkyl group, a C₃-C₁₀ cycloalkyl group, or a deuterium-containing C₃-C₁₀ cycloalkyl group.

In one or more embodiments, in Formula 1, R₂₁ may be a branched C₃-C₂₀ alkyl group, a deuterium-containing branched C₃-C₂₀ alkyl group, a C₃-C₁₀ cycloalkyl group, or a deuterium-containing C₃-C₁₀ cycloalkyl group.

In one or more embodiments, in Formula 1, R₂₂ may be a methyl group.

In one or more embodiments, in Formula 1, R₂₂ may be a C₂-C₂₀ alkyl group, a deuterium-containing C₂-C₂₀ alkyl group, a C₃-C₁₀ cycloalkyl group, or a deuterium-containing C₃-C₁₀ cycloalkyl group.

In one or more embodiments, in Formula 1, R₂₁ and R₂₂ may be identical to each other.

In one or more embodiments, in Formula 1, R₂₁ and R₂₂ may be different from each other.

In one or more embodiments, in Formula 1, R₂₃ may be hydrogen, or deuterium.

In one or more embodiments, the organometallic compound represented by Formula 1 may satisfy <Condition 3>, <Condition 4>, or combination thereof:
<Condition 3>
   R₁₀ in Formula 1 is represented by Formula 3.
<Condition 4>
   R₁₂ in Formula 1 is represented by Formula 4.

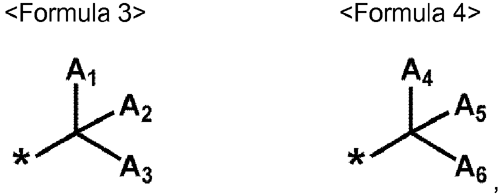

In Formulae 3 and 4,
A₁ to A₆ may each independently be:
hydrogen, or deuterium; or
a C₁-C₂₀ alkyl group, a C₃-C₁₀ cycloalkyl group, a C₆-C₆₀ aryl group, or a C₁-C₆₀ heteroaryl group, each unsubstituted or substituted with deuterium, a C₁-C₂₀ alkyl group, a C₃-C₁₀ cycloalkyl group, or any combination thereof; and
* indicates a binding site to a neighboring atom.

For example, Formula 3 may satisfy one of <Condition 3-1> to <Condition 3-5> and/or Formula 4 may satisfy one of <Condition 4-1> to <Condition 4-5>:
<Condition 3-1>
   A₁ to A₃ are not hydrogen, simultaneously.
<Condition 3-2>
   A₁ to A₃ are each independently a C₁-C₂₀ alkyl group, or a C₃-C₁₀ cycloalkyl group, each unsubstituted or substituted with deuterium, a C₁-C₂₀ alkyl group, a C₃-C₁₀ cycloalkyl group, or any combination thereof.
<Condition 3-3>
   A₁ and A₂ are each independently a C₁-C₂₀ alkyl group, or a C₃-C₁₀ cycloalkyl group, each unsubstituted or substituted with deuterium, a C₁-C₂₀ alkyl group, a C₃-C₁₀ cycloalkyl group, or any combination thereof, and
   A₃ is a C₂-C₂₀ alkyl group, or a C₃-C₁₀ cycloalkyl group, each unsubstituted or substituted with deuterium, a C₁-C₂₀ alkyl group, a C₃-C₁₀ cycloalkyl group, or any combination thereof.
<Condition 3-4>
   A₁ is a C₁-C₂₀ alkyl group, or a C₃-C₁₀ cycloalkyl group, each unsubstituted or substituted with deuterium, a C₁-C₂₀ alkyl group, a C₃-C₁₀ cycloalkyl group, or any combination thereof, and
   A₂ and A₃ are each independently a C₂-C₂₀ alkyl group, or a C₃-C₁₀ cycloalkyl group, each unsubstituted or substituted with deuterium, a C₁-C₂₀ alkyl group, a C₃-C₁₀ cycloalkyl group, or any combination thereof.
<Condition 3-5>
   A₁ is hydrogen, or deuterium,
   A₂ is a C₁-C₂₀ alkyl group, or a C₃-C₁₀ cycloalkyl group, each unsubstituted or substituted with deuterium, a C₁-C₂₀ alkyl group, a C₃-C₁₀ cycloalkyl group, or any combination thereof, and
   A₃ is a C₂-C₂₀ alkyl group, or a C₃-C₁₀ cycloalkyl group, each unsubstituted or substituted with deuterium, a C₁-C₂₀ alkyl group, a C₃-C₁₀ cycloalkyl group, or any combination thereof.
<Condition 4-1>
   A₄ to A₆ are not hydrogen, simultaneously.
<Condition 4-2>
   A₄ to A₆ are each independently a C₁-C₂₀ alkyl group, or a C₃-C₁₀ cycloalkyl group, each unsubstituted or substituted with deuterium, a C₁-C₂₀ alkyl group, a C₃-C₁₀ cycloalkyl group, or any combination thereof.
<Condition 4-3>
   A₄ and A₅ are each independently a C₁-C₂₀ alkyl group, or a C₃-C₁₀ cycloalkyl group, each unsubstituted or substituted with deuterium, a C₁-C₂₀ alkyl group, a C₃-C₁₀ cycloalkyl group, or any combination thereof, and
   A₆ is a C₂-C₂₀ alkyl group, or a C₃-C₁₀ cycloalkyl group, each unsubstituted or substituted with deuterium, a C₁-C₂₀ alkyl group, a C₃-C₁₀ cycloalkyl group, or any combination thereof.
<Condition 4-4>
   A₄ is a C₁-C₂₀ alkyl group, or a C₃-C₁₀ cycloalkyl group, each unsubstituted or substituted with deuterium, a C₁-C₂₀ alkyl group, a C₃-C₁₀ cycloalkyl group, or any combination thereof, and
   A₅ and A₆ are each independently a C₂-C₂₀ alkyl group, or a C₃-C₁₀ cycloalkyl group, each unsubstituted or substituted with deuterium, a C₁-C₂₀ alkyl group, a C₃-C₁₀ cycloalkyl group, or any combination thereof.
<Condition 4-5>
   A₄ is hydrogen, or deuterium,
   A₅ is a C₁-C₂₀ alkyl group, or a C₃-C₁₀ cycloalkyl group, each unsubstituted or substituted with deuterium, a C₁-C₂₀ alkyl group, a C₃-C₁₀ cycloalkyl group, or any combination thereof, and
   A₆ is a C₂-C₂₀ alkyl group, or a C₃-C₁₀ cycloalkyl group, each unsubstituted or substituted with deuterium, a C₁-C₂₀ alkyl group, a C₃-C₁₀ cycloalkyl group, or any combination thereof.

In one or more embodiments, at least one of R₁₀ and R₁₂ (for example, both R₁₀ and R₁₂) in Formula 1 may be a substituted or unsubstituted C₃-C₁₀ cycloalkyl group.

In one or more embodiments, at least one of R₁₀ and R₁₂ (for example, both R₁₀ and R₁₂) in Formula 1 may each independently be a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, or a bicyclo[2.2.2]octyl group, each unsubstituted or substituted with deuterium, -F, -CD₃, - CD₂H, -CDH₂, -CF₃, -CF₂H, -CFH₂, a cyano group, a C₁-C₁₀ alkyl group, or any combination thereof.

In one or more embodiments, R₁₀ and R₁₂ in Formula 1 may be identical to each other.

In one or more embodiments, R₁₀ and R₁₂ in Formula 1 may be different from each other.

In one or more embodiments, R₁₁ in Formula 1 may be hydrogen, deuterium, or a methyl group.

In one or more embodiments, a case in which R₁₀ and R₁₂ in Formula 1 are a methyl group and R₁₁ in Formula 1 is hydrogen is excluded.

In one or more embodiments, in Formulae 1, 3 and 4,
R₁ to R₁₂ and R₂₃ may each independently be hydrogen, deuterium, -F, a cyano group, a nitro group, -SF₅, -CH₃, -CD₃, -CD₂H, -CDH₂, -CF₃, -CF₂H, -CFH₂, a group represented by one of Formulae 9-1 to 9-66, a group represented by one of Formulae 9-1 to 9-66 in which at least one hydrogen is substituted with deuterium, a group represented by one of Formulae 10-1 to 10-249, a group represented by one of Formulae 10-1 to 10-249 in which at least one hydrogen is substituted with deuterium, -Si(Q₃)(Q₄)(Q₅), or - Ge(Q₃)(Q₄)(Q₅) (wherein Q₃ to Q₅ are the same as described herein), and/or
at least one of R₁ to R₅ may each independently be deuterium, -F, a cyano group, a nitro group, -SF₅, -CH₃, -CD₃, -CD₂H, -CDH₂, -CF₃, -CF₂H, -CFH₂, a group represented by one of Formulae 9-1 to 9-66, a group represented by one of Formulae 9-1 to 9-66 in which at least one hydrogen is substituted with deuterium, a group represented by one of Formulae 10-1 to 10-249, a group represented by one of Formulae 10-1 to 10-249 in which at least one hydrogen is substituted with deuterium, -Si(Q₃)(Q₄)(Q₅), or -Ge(Q₃)(Q₄)(Q₅) (wherein Q₃ to Q₅ are the same as described herein), and/or
R₂₁ may be a group represented by one of Formulae 9-1 to 9-33, a group represented by one of Formulae 9-1 to 9-33 in which at least one hydrogen is substituted with deuterium, a group represented by one of Formulae 10-1 to 10-10, or a group represented by one of Formulae 10-1 to 10-10 in which at least one hydrogen is substituted with deuterium, and/or
R₂₂ may be -CH₃, -CD₃, -CD₂H, -CDH₂, a group represented by one of Formulae 9-1 to 9-33, a group represented by one of Formulae 9-1 to 9-33 in which at least one hydrogen is substituted with deuterium, a group represented by one of Formulae 10-1 to 10-10, or a group represented by one of Formulae 10-1 to 10-10 in which at least one hydrogen is substituted with deuterium, and/or
A₁ to A₆ may each independently be hydrogen, deuterium, -CH₃, -CD₃, -CD₂H, - CDH₂, a group represented by one of Formulae 9-1 to 9-63, a group represented by one of Formulae 9-1 to 9-63 in which at least one hydrogen is substituted with deuterium, a group represented by one of Formulae 10-1 to 10-10, or a group represented by one of Formulae 10-1 to 10-10 in which at least one hydrogen is substituted with deuterium:

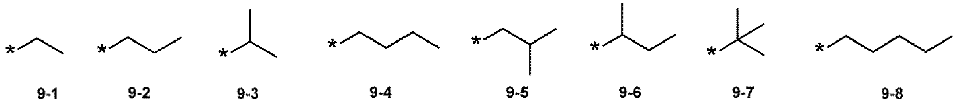

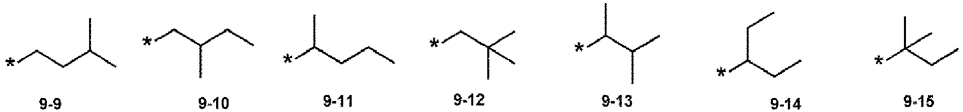

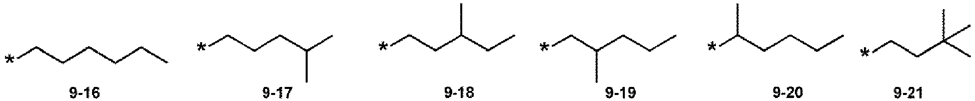

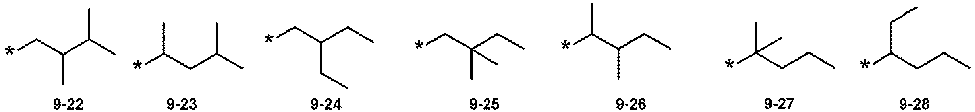

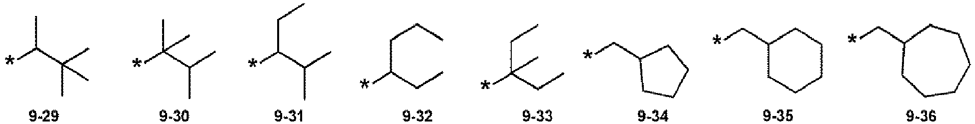

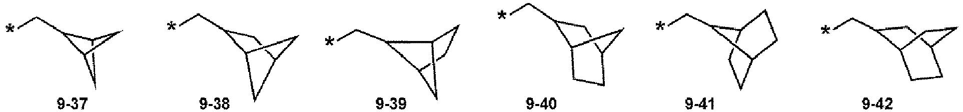

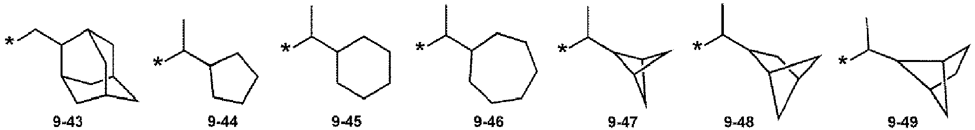

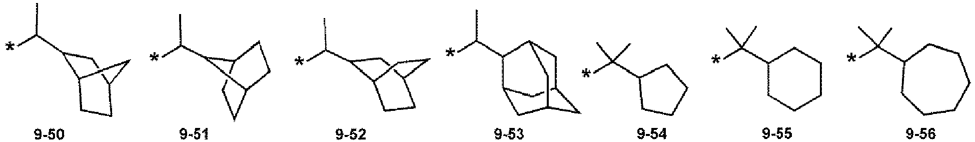

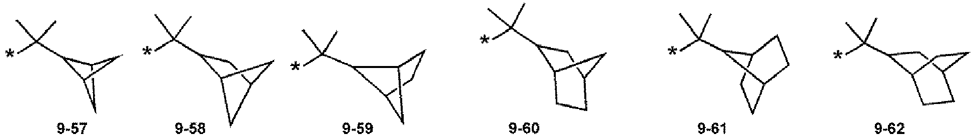

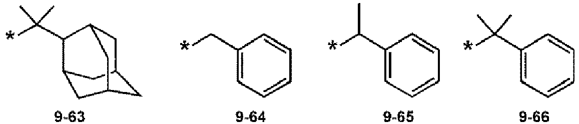

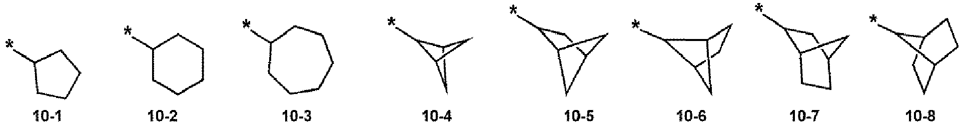

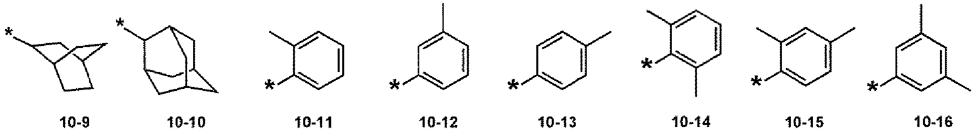

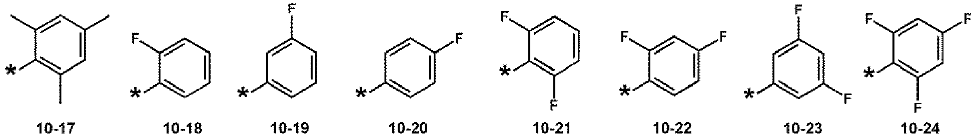

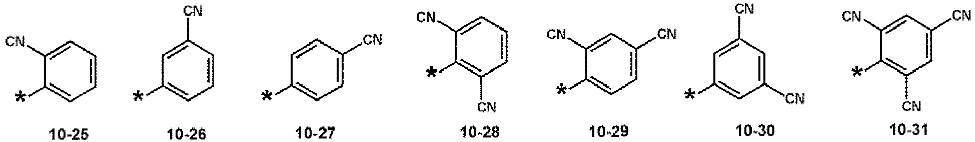

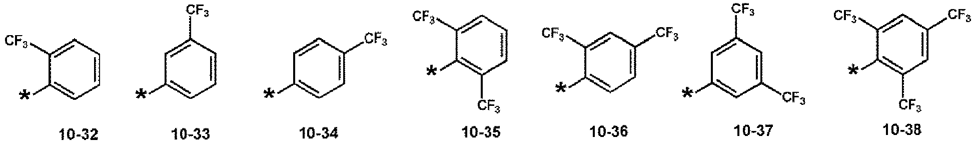

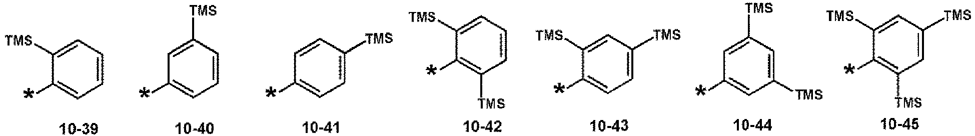

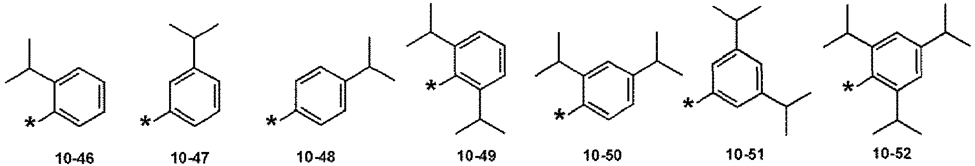

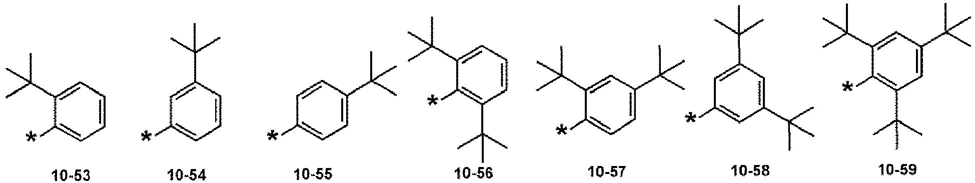

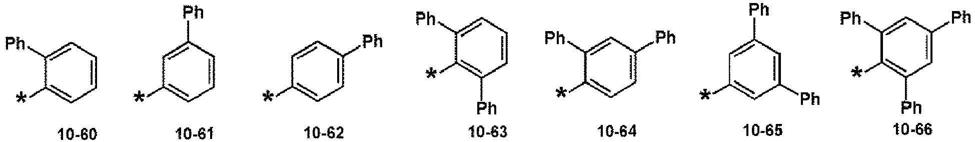

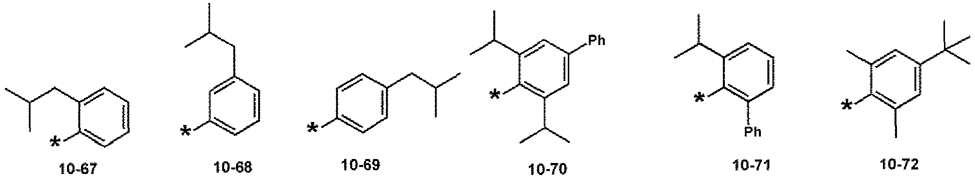

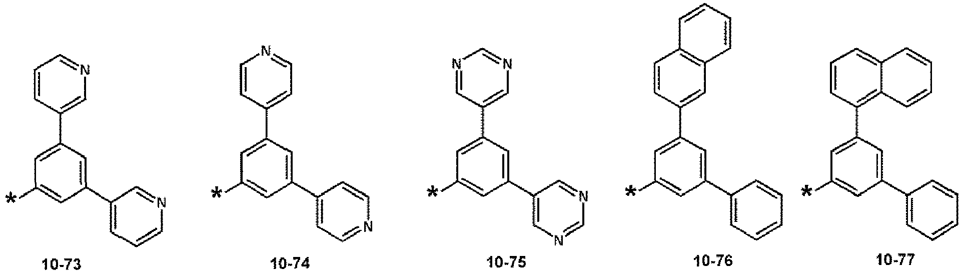

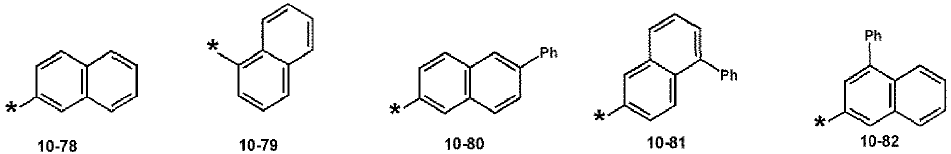

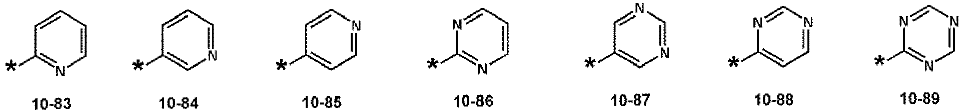

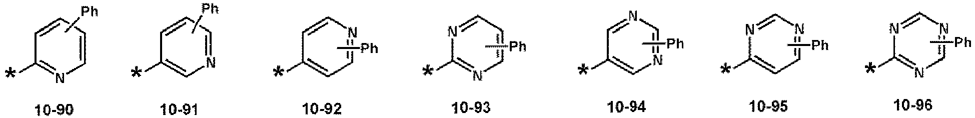

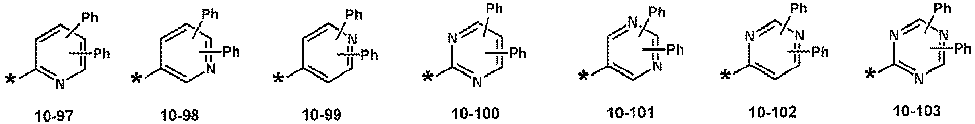

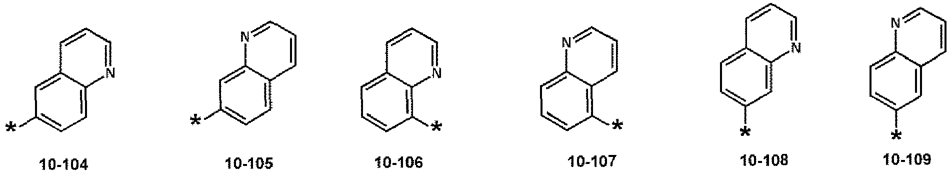

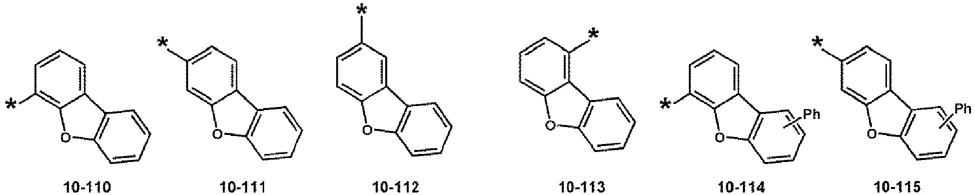

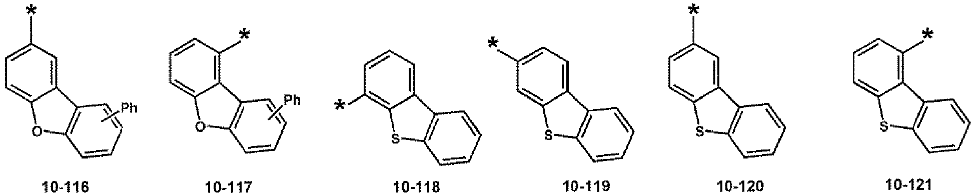

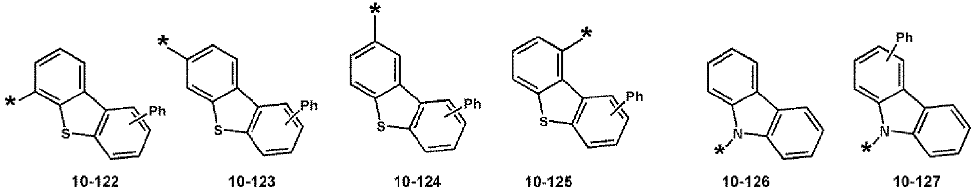

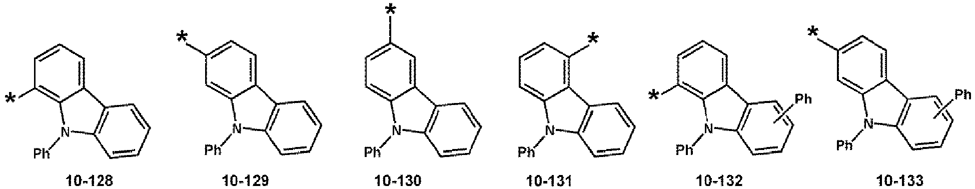

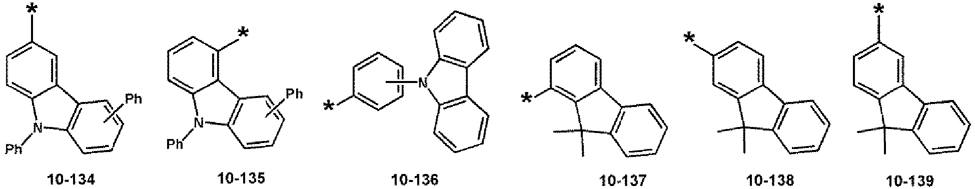

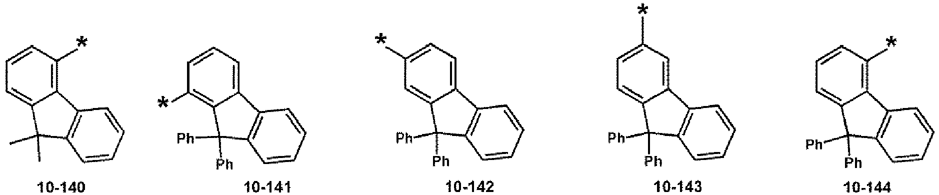

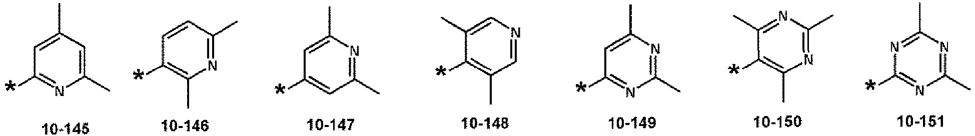

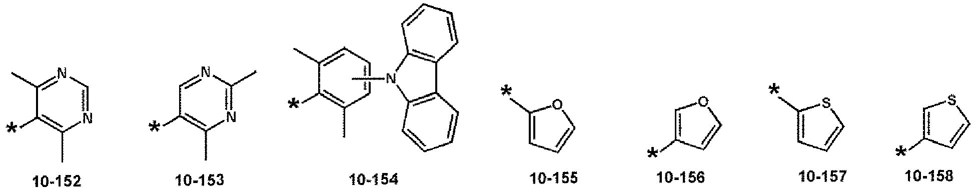

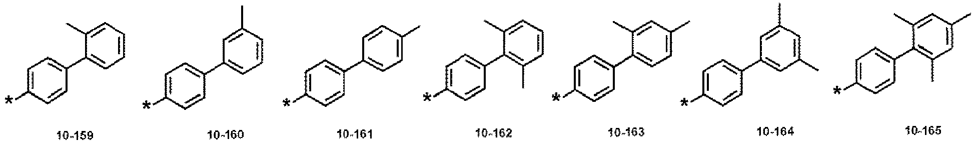

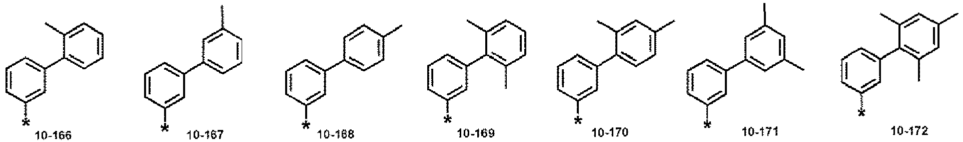

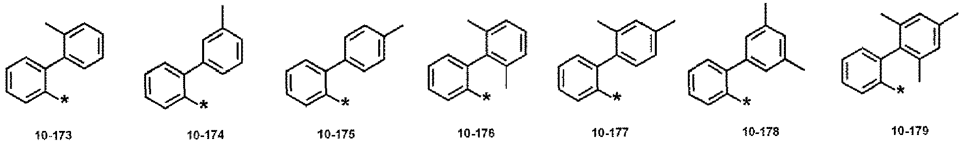

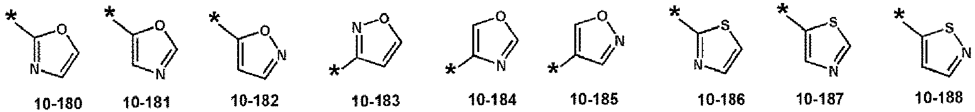

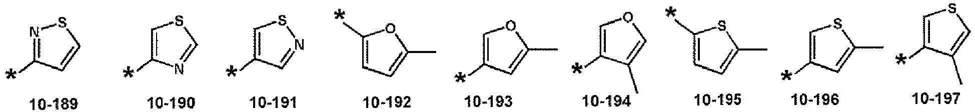

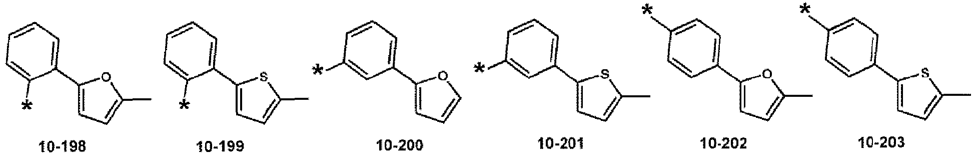

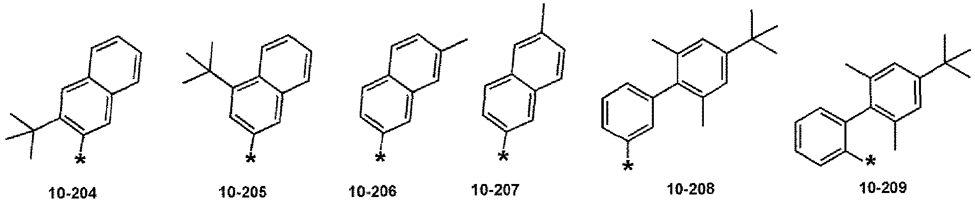

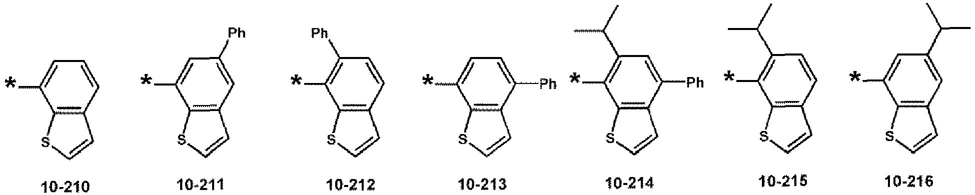

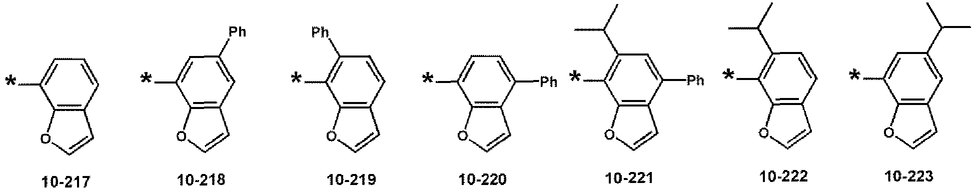

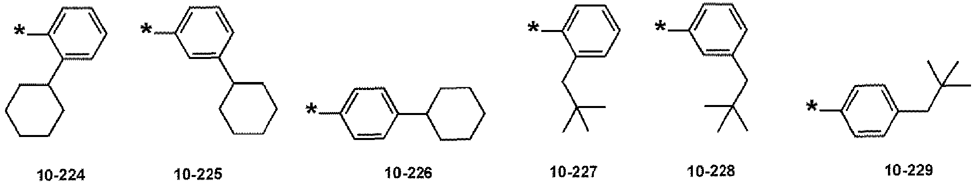

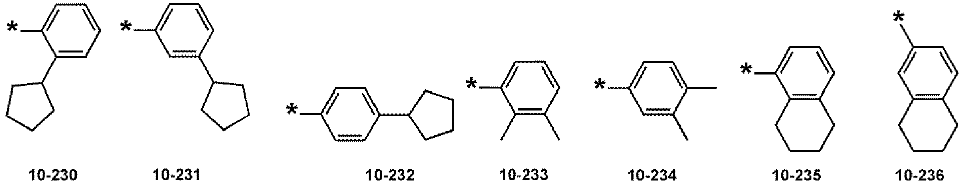

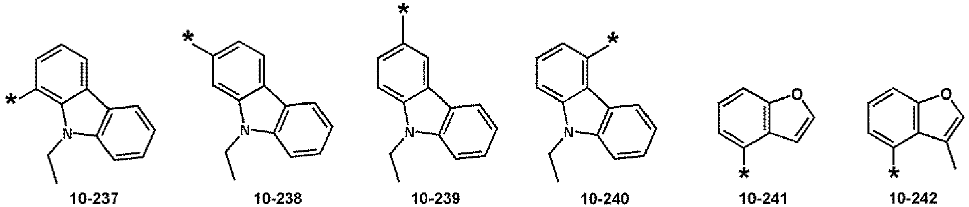

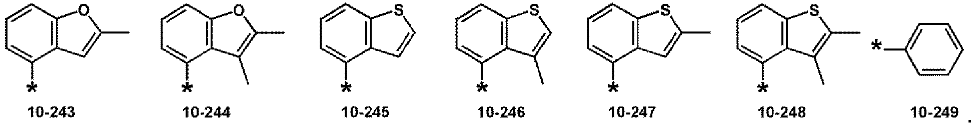

In Formulae 9-1 to 9-66 and 10-1 to 10-249, * indicates a binding site to a neighboring atom, Ph indicates a phenyl group, and TMS indicates a trimethylsilyl group.

The "group represented by one of Formulae 9-1 to 9-66 in which at least one hydrogen is substituted with deuterium" may be, for example, a group represented by one of Formulae 9-501 to 9-552:

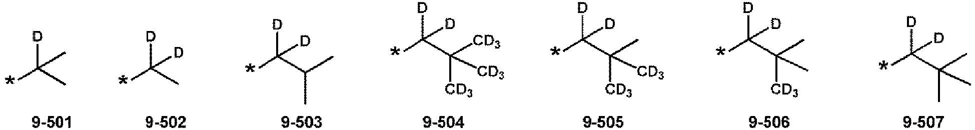

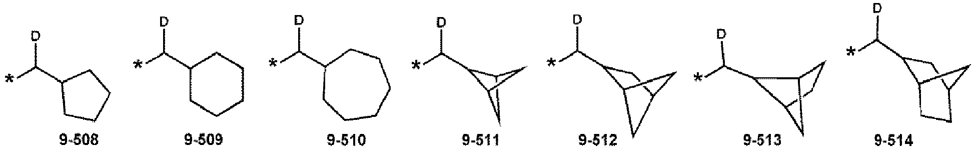

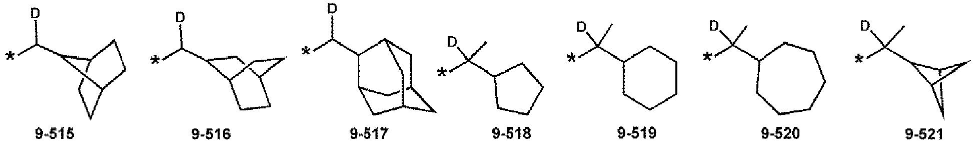

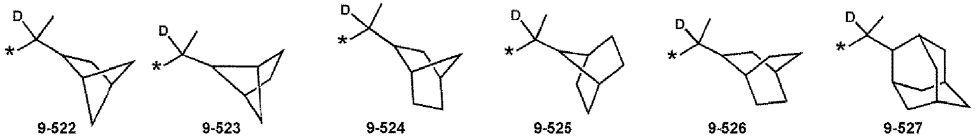

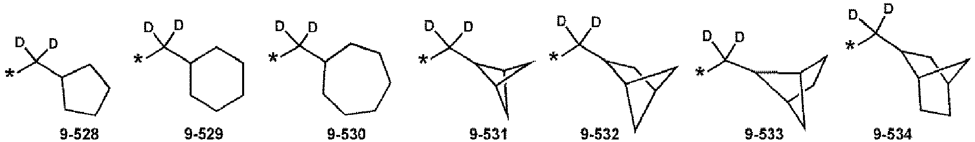

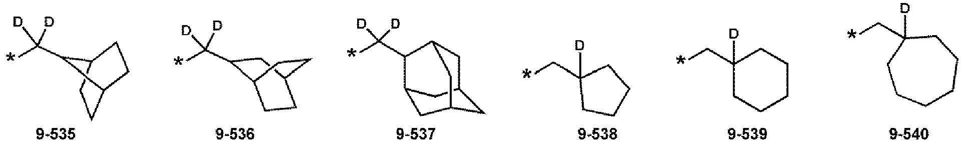

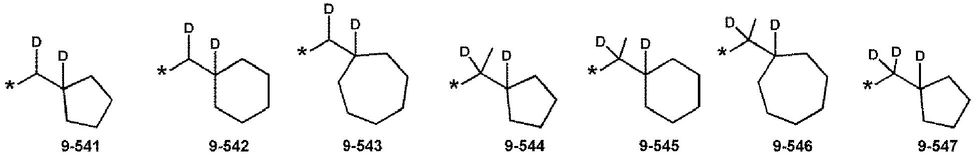

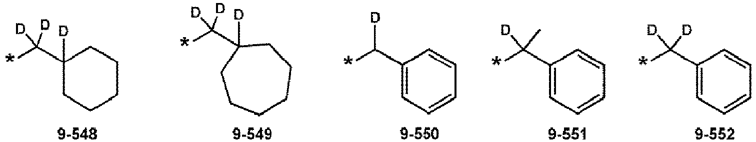

The "group represented by one of Formulae 10-1 to 10-249 in which at least one hydrogen is substituted with deuterium" may be, for example, a group represented by one of Formulae 10-501 to 10-510:

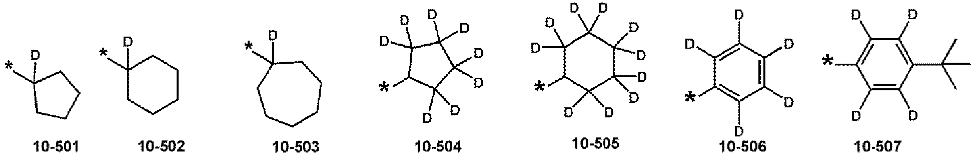

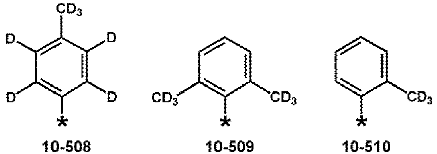

The terms "a deuterium-containing C₁-C₆₀ alkyl group (for example, a deuterium-containing C₁-C₂₀ alkyl group)" and "a deuterium-containing C₃-C₁₀ cycloalkyl group" as used herein each refer to a C₁-C₆₀ alkyl group substituted with at least one deuterium (for example, a C₁-C₂₀ alkyl group substituted with at least one deuterium) and a C₃-C₁₀ cycloalkyl group substituted with at least one deuterium, respectively. For example, a deuterium-containing methyl group may be -CDH₂, -CD₂H, and -CD₃.

Non-limiting examples of the "C₁-C₆₀ alkyl group (for example, C₁-C₂₀ alkyl group)" include a methyl group, an ethyl group, an n-propyl group, an isopropyl group, an n-butyl group, a sec-butyl group, an isobutyl group, a tert-butyl group, an n-pentyl group, a tert-pentyl group, a neopentyl group, an isopentyl group, a sec-pentyl group, a 3-pentyl group, a sec-isopentyl group, an n-hexyl group, an isohexyl group, a sec-hexyl group, a tert-hexyl group, an n-heptyl group, an isoheptyl group, a sec-heptyl group, a tert-heptyl group, an n-octyl group, an isooctyl group, a sec-octyl group, a tert-octyl group, an n-nonyl group, an isononyl group, a sec-nonyl group, a tert-nonyl group, an n-decyl group, an isodecyl group, a sec-decyl group, a tert-decyl group, and the like, but embodiments of the present disclosure are not limited thereto.

Non-limiting examples of the "C₂-C₆₀ alkyl group (for example, C₂-C₂₀ alkyl group)" include groups in which "a methyl group" is excluded from above examples for the "C₁-C₆₀ alkyl group (for example, C₁-C₂₀ alkyl group)".

Non-limiting of the "C₃-C₁₀ cycloalkyl group" include a cyclopentyl group, a cyclohexyl group, a cycloheptyl group, a cycloctyl group, an adamantanyl group, a norbornanyl group, a bicyclo[1.1.1]pentyl group, a bicyclo[2.1.1]hexyl group, a bicyclo[2.2.1]heptyl group, a bicyclo[2.2.2]octyl group, and the like, but embodiments of the present disclosure are not limited thereto.

In Formula 1, two or more of R₁ to R₉ may optionally be linked to each other to form a C₅-C₃₀ carbocyclic group unsubstituted or substituted with at least one R₁ₐ or a C₂-C₃₀ heterocyclic group unsubstituted or substituted with at least one R₁ₐ, and two or more of R₁₀ to R₁₂ may optionally be linked to each other to form a C₅-C₃₀ carbocyclic group unsubstituted or substituted with at least one R₁ₐ or a C₂-C₃₀ heterocyclic group unsubstituted or substituted with at least one R₁ₐ.

For example, the C₅-C₃₀ carbocyclic group unsubstituted or substituted with at least one R₁ₐ and the C₂-C₃₀ heterocyclic group unsubstituted or substituted with at least one R₁ₐ may be a cyclopentane group, a cyclohexane group, a cycloheptane group, a cyclooctane group, an adamantane group, a norbornane group, a norbornene group, a cyclopentene group, a cyclohexene group, a cycloheptene group, a bicyclo[1.1.1]pentane group, a bicyclo[2.1.1]hexane group, a bicyclo[2.2.1]heptane group, a bicyclo[2.2.2]octane group, a benzene group, a naphthalene group, a fluorene group, a phenanthrene group, an anthracene group, a fluoranthene group, a triphenylene group, a pyrene group, a chrysene group, a pyrrole group, a thiophene group, a furan group, an imidazole group, a pyrazole group, a thiazole group, an isothiazole group, an oxazole group, an isoxazole group, a pyridine group, a pyrazine group, a pyrimidine group, a pyridazine group, an isoindole group, an indole group, an indazole group, a purine group, a quinoline group, an isoquinoline group, a benzoquinoline group, a quinoxaline group, a quinazoline group, a cinnoline group, a carbazole group, a phenanthroline group, a benzimidazole group, a benzofuran group, a benzothiophene group, an isobenzothiophene group, a benzoxazole group, an isobenzoxazole group, a triazole group, a tetrazole group, an oxadiazole group, a triazine group, a tetrazine group, a dibenzofuran group, a dibenzothiophene group, a benzocarbazole group, a dibenzocarbazole group, an imidazopyridine group, or an imidazopyrimidine group, each unsubstituted or substituted with at least one R₁ₐ..

R₁ₐ may be the same as defined in connection with R₁.

For example, the organometallic compound may be one of Compounds 1 to 22, but embodiments of the present disclosure are not limited thereto:

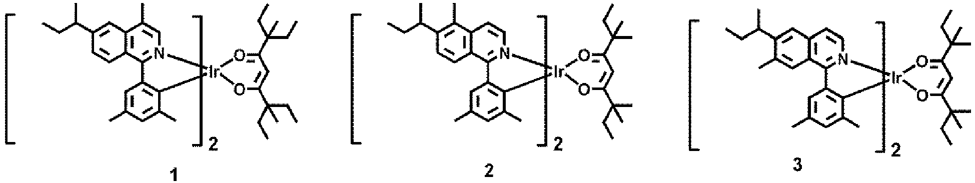

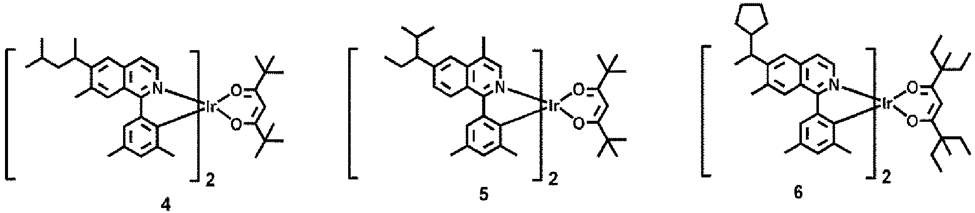

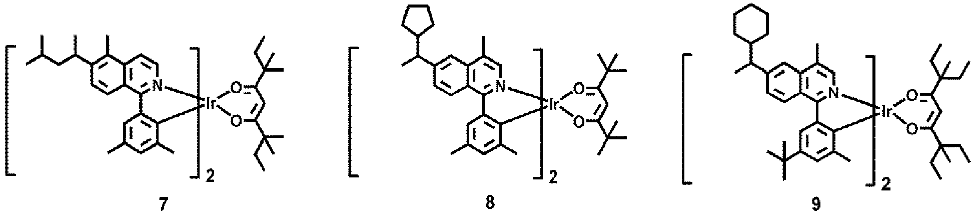

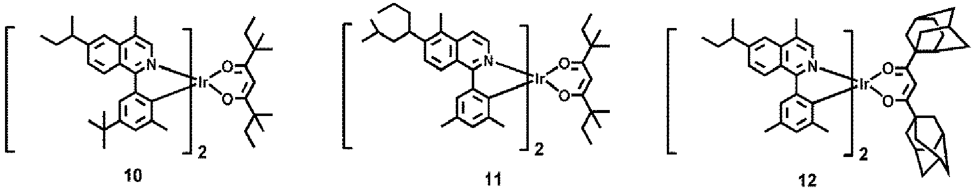

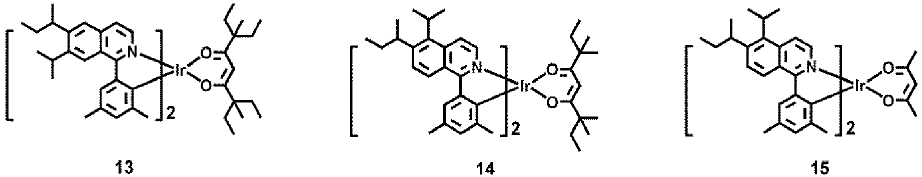

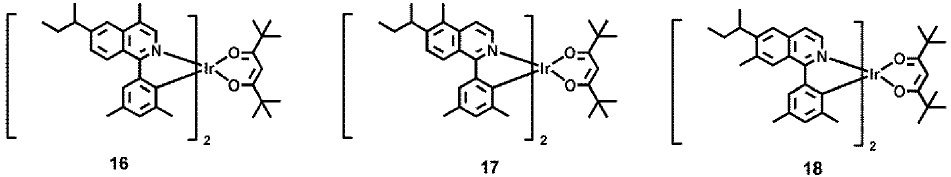

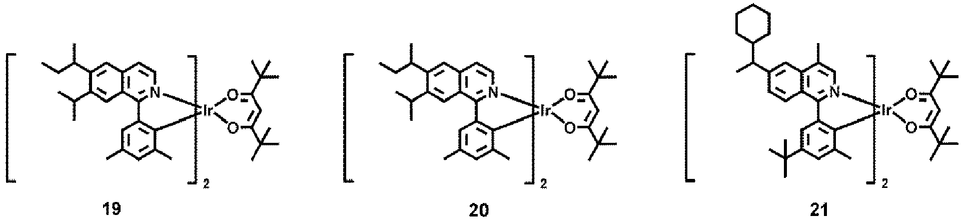

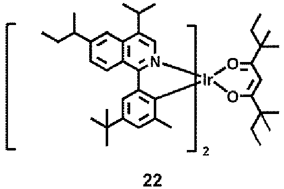

In Formula 1, R₂₁ may be a C₂-C₂₀ alkyl group, a deuterium-containing C₂-C₂₀ alkyl group, a C₃-C₁₀ cycloalkyl group, or a deuterium-containing C₃-C₁₀ cycloalkyl group. In this regard, the organometallic compound represented by Formula 1 may have increased anisotropy so that the horizontal orientation ratio may be increased. Therefore, an electronic device, for example, an organic light-emitting device, including the organometallic compound represented by Formula 1 may have improved luminescence efficiency.

In addition, R₂₂ in Formula 1 may be a C₁-C₂₀ alkyl group, a deuterium-containing C₁-C₂₀ alkyl group, a C₃-C₁₀ cycloalkyl group, or a deuterium-containing C₃-C₁₀ cycloalkyl group. In this regard, the organometallic compound represented by Formula 1 may have improved structural rigidity. Therefore, the organometallic compound represented by Formula 1 may have reduced non-radiative transition. In addition, molecules of the organometallic compound may be structurally elongated along the molecular axis direction of the organometallic compound so that the horizontal orientation ratio may be increased. Therefore, an electronic device, for example, an organic light-emitting device, including the organometallic compound represented by Formula 1 may have improved luminescence efficiency.

Furthermore, at least one of R₁ to R₅ in Formula 1 may not be hydrogen. For example, at least one of R₁ to R₅ in Formula 1 may each independently be deuterium, -F, - Cl, -Br, -I, -SF₅, a hydroxyl group, a cyano group, a nitro group, an amino group, an amidino group, a hydrazino group, a hydrazono group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -N(Q₁)(Q₂), -Si(Q₃)(Q₄)(Q₅), -Ge(Q₃)(Q₄)(Q₅), -B(Q₆)(Q₇), -P(=O)(Q₈)(Q₉), or -P(Q₈)(Q₉). Accordingly, an isoquinoline ring, i.e., a lowest unoccupied molecular orbital (LUMO) part, of the organometallic compound represented by Formula 1 may have secured stability, and therefore, an electronic device, for example, an organic light-emitting device, including the organometallic compound may have improved lifespan. In addition, although not limited to a particular theory, the horizontal orientation ratio of the organometallic compound may be increased while full width at half maximum (FWHM) of the organometallic compound may be reduced, when at least one of R₁ to R₅ in Formula 1 satisfies the definition as described above.

In the organometallic compound represented by Formula 1, the horizontal orientation ratio of a transition dipole moment may be about 90 % to about 100 %.

For example, the horizontal orientation ratio of the transition dipole moment in the organometallic compound may be, for example, about 90 % to about 100 %, about 91% to about 100 %, about 92 % to about 100 %, about 93 % to about 100 %, about 94 % to about 100 %, about 95 % to about 100 %, about 96 % to about 100 %, about 9 7% to about 100 %, about 98 % to about 100 %, about 99 % to about 100 %, or 100 %, but embodiments of the present disclosure are not limited thereto.

Here, the horizontal orientation ratio of the transition dipole moment refers to a ratio of an organometallic compound having dipole moment that is perpendicular to a film relative to the the total organometallic compound included in the films.

The horizontal orientation ratio of the transition dipole moment may be evaluated using an angle-dependent PL measuring device. Such an angle-dependent PL measuring device may be described by referring to, for example, the angle-dependent PL measuring device described in KR Application No. 2013-0150834.

Since the organometallic compound has a high horizontal orientation ratio of the transition dipole moment as described above, the organometallic compound is also considered to have a large horizontal orientation transition dipole moment (i.e., a large horizontal orientation photo-orientation). Accordingly, a large amount (substantial) of electrical fields traveling in a direction perpendicular to the film including the organometallic compound may be emitted. The light emitted according to such a mechanism may result in high external purge efficiency (i.e., efficiency at which the light emitted from the organometallic compound is purged to the outside from a device (for example, an organic light-emitting device) including a film (for example, an emission layer to be described later) including the organometallic compound. Therefore, an electronic device, for example, an organic light-emitting device, including the organometallic compound may achieve high luminescence efficiency.

Synthesis methods of the organometallic compound represented by Formula 1 may be recognizable by one of ordinary skill in the art by referring to Synthesis Examples provided below.

The organometallic compound represented by Formula 1 is suitable for use in an organic layer of an organic light-emitting device, for example, for use as a dopant in an emission layer of the organic layer. Thus, another aspect of the present disclosure provides an organic light-emitting device including: a first electrode; a second electrode; and an organic layer disposed between the first electrode and the second electrode and including an emission layer, wherein the organic layer includes at least one organometallic compound represented by Formula 1.

The organic light-emitting device may have, due to the inclusion of an organic layer including the organometallic compound represented by Formula 1, a low driving voltage, relatively reduced FWHM, a long lifespan and/or high luminescent efficiency.

The organometallic compound represented by Formula 1 may be used between a pair of electrodes of an organic light-emitting device. For example, the organometallic compound represented by Formula 1 may be included in the emission layer. In this regard, the organometallic compound may act as a dopant, and the emission layer may further include a host (that is, in the emission layer, an amount of the organometallic compound represented by Formula 1 is smaller than an amount of the host).

In one embodiment, the emission layer may emit red light.

The expression "(an organic layer) includes at least one organometallic compounds" as used herein may include a case in which "(an organic layer) includes identical organometallic compounds represented by Formula 1" and a case in which "(an organic layer) includes two or more different organometallic compounds represented by Formula 1".

For example, the organic layer may include, as the organometallic compound, only Compound 1. In this regard, Compound 1 may exist only in the emission layer of the organic light-emitting device. In one or more embodiments, the organic layer may include, as the organometallic compound, Compound 1 and Compound 2. In this regard, Compound 1 and Compound 2 may exist in an identical layer (for example, Compound 1 and Compound 2 all may exist in an emission layer).

The first electrode may be an anode, which is a hole injection electrode, and the second electrode may be a cathode, which is an electron injection electrode; or the first electrode may be a cathode, which is an electron injection electrode, and the second electrode may be an anode, which is a hole injection electrode.

In one embodiment, in the organic light-emitting device, the first electrode is an anode, and the second electrode is a cathode, and the organic layer further includes a hole transport region between the first electrode and the emission layer and an electron transport region between the emission layer and the second electrode, and the hole transport region includes a hole injection layer, a hole transport layer, an electron blocking layer, a buffer layer, or any combination thereof, and the electron transport region includes a hole blocking layer, an electron transport layer, an electron injection layer, or any combination thereof.

The term "organic layer" as used herein refers to a single layer and/or a plurality of layers disposed between the first electrode and the second electrode of an organic light-emitting device. The "organic layer" may include, in addition to an organic compound, an organometallic complex including metal.

FIGURE is a schematic cross-sectional view of an organic light-emitting device 10 according to an embodiment. Hereinafter, the structure of an organic light-emitting device according to an embodiment and a method of manufacturing an organic light-emitting device according to an embodiment will be described in connection with the FIGURE. The organic light-emitting device 10 includes a first electrode 11, an organic layer 15, and a second electrode 19, which are sequentially stacked.

A substrate may be additionally disposed under the first electrode 11 or above the second electrode 19. For use as the substrate, any substrate that is used in general organic light-emitting devices may be used, and the substrate may be a glass substrate or a transparent plastic substrate, each having excellent mechanical strength, thermal stability, transparency, surface smoothness, ease of handling, and water resistance.

The first electrode 11 may be formed by depositing or sputtering a material for forming the first electrode 11 on the substrate. The first electrode 11 may be an anode. The material for forming the first electrode 11 may include a material with a high work function to facilitate hole injection. The first electrode 11 may be a reflective electrode, a semi-transmissive electrode, or a transmissive electrode. The material for forming the first electrode 11 may be, for example, indium tin oxide (ITO), indium zinc oxide (IZO), tin oxide (SnO₂), zinc oxide (ZnO), or any combination thereof. In one or more embodiments, the material for forming the first electrode 11 may be metal, such as magnesium (Mg), aluminum (Al), aluminum-lithium (Al-Li), calcium (Ca), magnesium-indium (Mg-In), or magnesium-silver (Mg-Ag).

The first electrode 11 may have a single-layered structure or a multi-layered structure including two or more layers. For example, the first electrode 11 may have a three-layered structure of ITO/Ag/ITO, but the structure of the first electrode 11 is not limited thereto.

The organic layer 15 is disposed on the first electrode 11.

The organic layer 15 may include a hole transport region, an emission layer, and an electron transport region.

The hole transport region may be disposed between the first electrode 11 and the emission layer.

The hole transport region may include a hole injection layer, a hole transport layer, an electron blocking layer, a buffer layer, or any combination thereof.

The hole transport region may include only either a hole injection layer or a hole transport layer. In one or more embodiments, the hole transport region may have a hole injection layer/hole transport layer structure or a hole injection layer/hole transport layer/electron blocking layer structure, which are sequentially stacked in this stated order from the first electrode 11.

When the hole transport region includes a hole injection layer, the hole injection layer may be formed on the first electrode 11 by using one or more suitable methods, for example, vacuum deposition, spin coating, casting, and/or Langmuir-Blodgett (LB) deposition.

When a hole injection layer is formed by vacuum deposition, the deposition conditions may vary according to a material that is used to form the hole injection layer, and the structure and thermal characteristics of the hole injection layer. For example, the deposition conditions may include a deposition temperature of about 100 °C to about 500 °C, a vacuum pressure of about 1.33 × 10⁻⁶ to about 0.133 Pa (about 10⁻⁸ to about 10⁻³ torr), and a deposition rate of about 0.01 Å/sec to about 100 Å/sec. However, the deposition conditions are not limited thereto.

When the hole injection layer is formed using spin coating, coating conditions may vary according to the material used to form the hole injection layer, and the structure and thermal properties of the hole injection layer. For example, a coating speed may be from about 2,000 rpm to about 5,000 rpm, and a temperature at which a heat treatment is performed to remove a solvent after coating may be from about 80 °C to about 200 °C. However, the coating conditions are not limited thereto.

Conditions for forming a hole transport layer and an electron blocking layer may be understood by referring to conditions for forming the hole injection layer.

The hole transport region may include m-MTDATA, TDATA, 2-TNATA, NPB, β-NPB, TPD, Spiro-TPD, Spiro-NPB, methylated-NPB, TAPC, HMTPD, 4,4',4"-tris(N-carbazolyl)triphenylamine (TCTA), polyaniline/dodecylbenzenesulfonic acid (PANI/DBSA), poly(3,4-ethylenedioxythiophene)/poly(4-styrenesulfonate) (PEDOT/PSS), polyaniline/camphor sulfonicacid (PANI/CSA), polyaniline/poly(4-styrenesulfonate) (PANI/PSS), a compound represented by Formula 201 below, a compound represented by Formula 202 below, or any combination thereof:

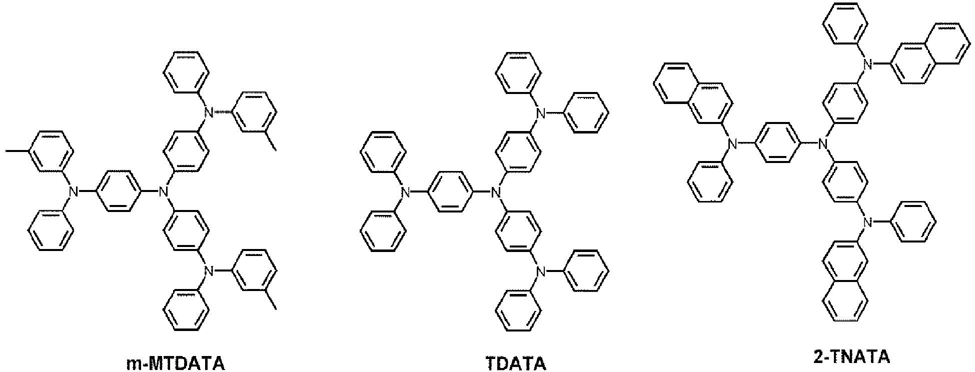

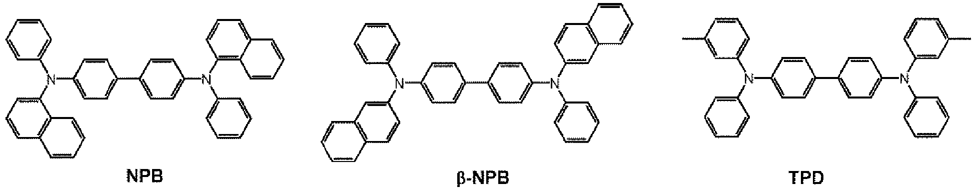

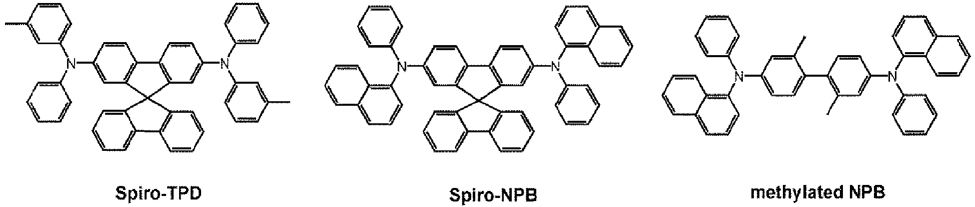

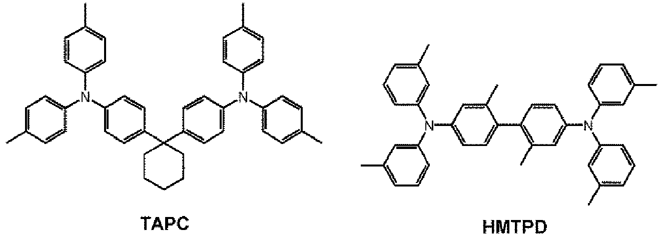

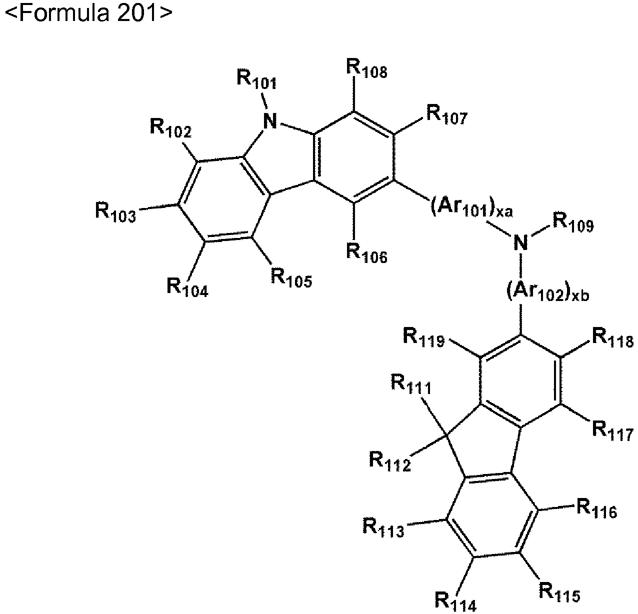

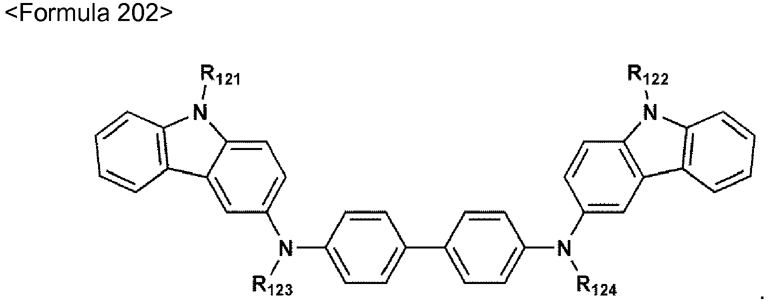

In Formula 201, Ar₁₀₁ and Ar₁₀₂ may each independently be a phenylene group, a pentalenylene group, an indenylene group, a naphthylene group, an azulenylene group, a heptalenylene group, an acenaphthylene group, a fluorenylene group, a phenalenylene group, a phenanthrenylene group, an anthracenylene group, a fluoranthenylene group, a triphenylenylene group, a pyrenylene group, a chrysenylenylene group, a naphthacenylene group, a picenylene group, a perylenylene group, or a pentacenylene group, each unsubstituted or substituted with deuterium, -F, -Cl, -Br, -I, a hydroxyl group, a cyano group, a nitro group, an amino group, an amidino group, a hydrazino group, a hydrazono group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₃-C₁₀ cycloalkenyl group, a C₁-C₁₀ heterocycloalkyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, or any combination thereof.

In Formula 201, xa and xb may each independently be an integer from 0 to 5, or may be 0, 1, or 2. For example, xa may be 1 and xb may be 0, but xa and xb are not limited thereto.

In Formulae 201 and 202, R₁₀₁ to R₁₀₈, R₁₁₁ to R₁₁₉, and R₁₂₁ to R₁₂₄ may each independently be:
hydrogen, deuterium, -F, -Cl, -Br, -I, a hydroxyl group, a cyano group, a nitro group, an amino group, an amidino group, a hydrazino group, a hydrazono group, a carboxylic acid group or a salt thereof, a sulfonic acid group or a salt thereof, a phosphoric acid group or a salt thereof, a C₁-C₁₀ alkyl group (for example, a methyl group, an ethyl group, a propyl group, a butyl group, pentyl group, a hexyl group, or the like), or a C₁-C₁₀ alkoxy group (for example, a methoxy group, an ethoxy group, a propoxy group, a butoxy group, a pentoxy group, or the like);
a C₁-C₁₀ alkyl group or a C₁-C₁₀ alkoxy group, each substituted with deuterium, -F, -Cl, -Br, -I, a hydroxyl group, a cyano group, a nitro group, an amino group, an amidino group, a hydrazino group, a hydrazono group, a carboxylic acid group or a salt thereof, a sulfonic acid group or a salt thereof, a phosphoric acid group or a salt thereof, or any combination thereof; or
a phenyl group, a naphthyl group, an anthracenyl group, a fluorenyl group, or a pyrenyl group, each unsubstituted or substituted with deuterium, -F, -Cl, -Br, -I, a hydroxyl group, a cyano group, a nitro group, an amino group, an amidino group, a hydrazino group, a hydrazono group, a carboxylic acid group or a salt thereof, a sulfonic acid group or a salt thereof, a phosphoric acid group or a salt thereof, a C₁-C₁₀ alkyl group, a C₁-C₁₀ alkoxy group, or any combination thereof;
but embodiments of the present disclosure are not limited thereto.

In Formula 201, R₁₀₉ may be a phenyl group, a naphthyl group, an anthracenyl group, or a pyridinyl group, each unsubstituted or substituted with deuterium, -F, -Cl, -Br, -I, a hydroxyl group, a cyano group, a nitro group, an amino group, an amidino group, a hydrazino group, a hydrazono group, a carboxylic acid group or a salt thereof, a sulfonic acid group or a salt thereof, a phosphoric acid group or a salt thereof, a C₁-C₂₀ alkyl group, a C₁-C₂₀ alkoxy group, a phenyl group, a naphthyl group, an anthracenyl group, a pyridinyl group, or any combination thereof.

In one embodiment, the compound represented by Formula 201 may be represented by Formula 201A below, but embodiments of the present disclosure are not limited thereto:

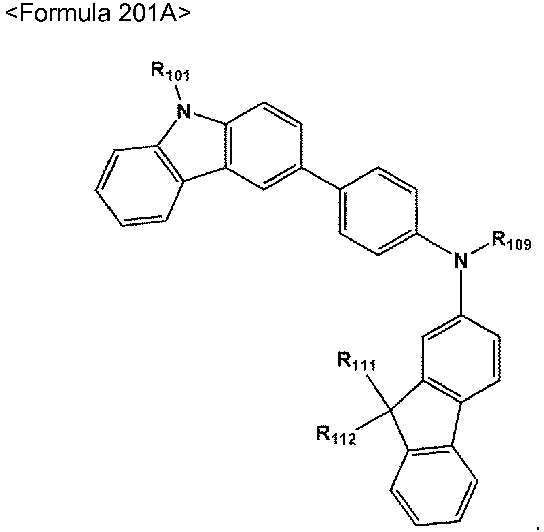

Detailed descriptions about R₁₀₁, R₁₁₁, R₁₁₂, and R₁₀₉ in Formula 201A are already described above.

For example, the compound represented by Formula 201 and the compound represented by Formula 202 may each include Compounds HT1 to HT20 illustrated below, but are not limited thereto:

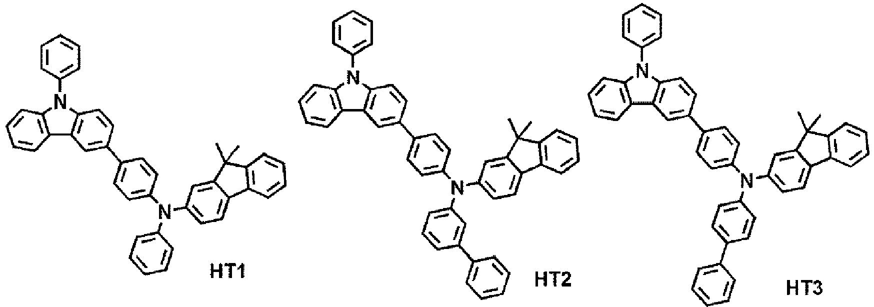

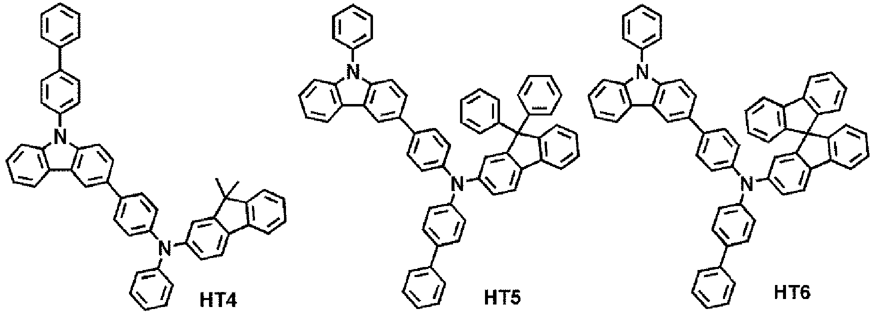

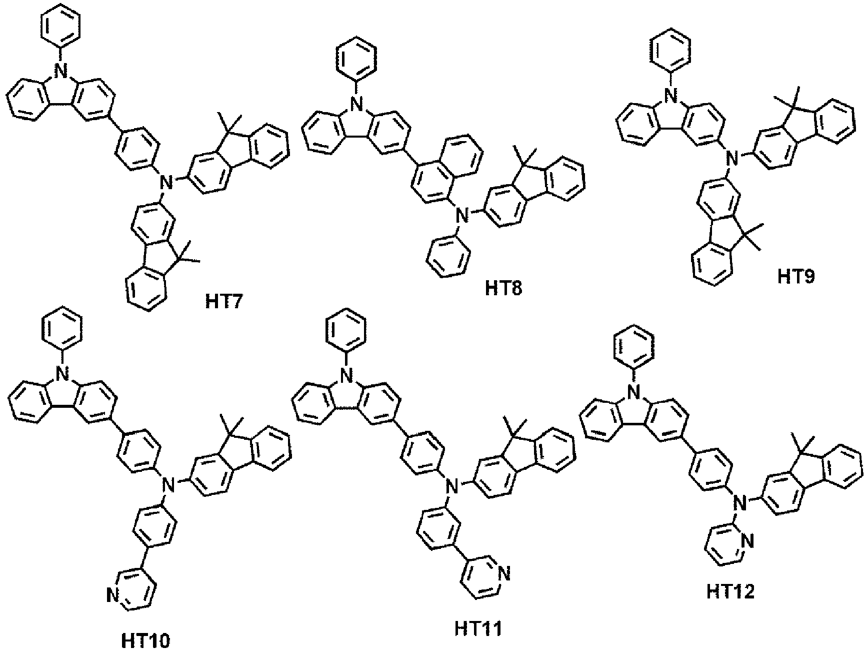

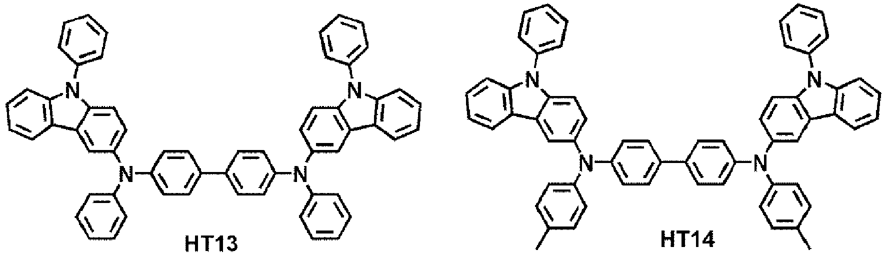

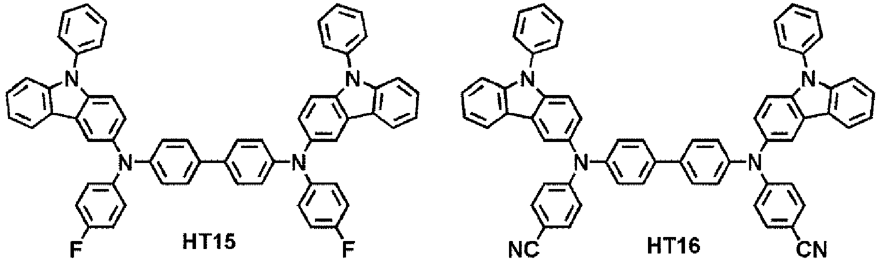

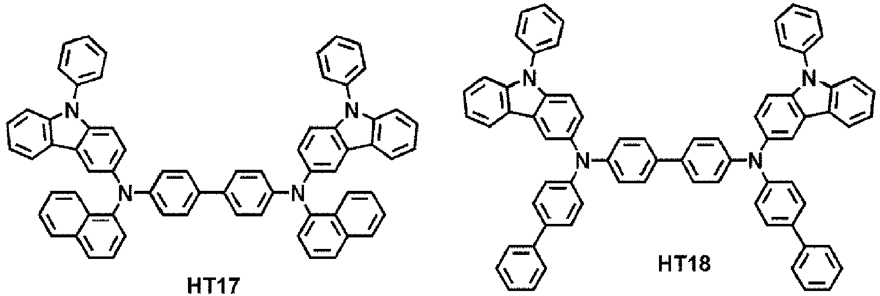

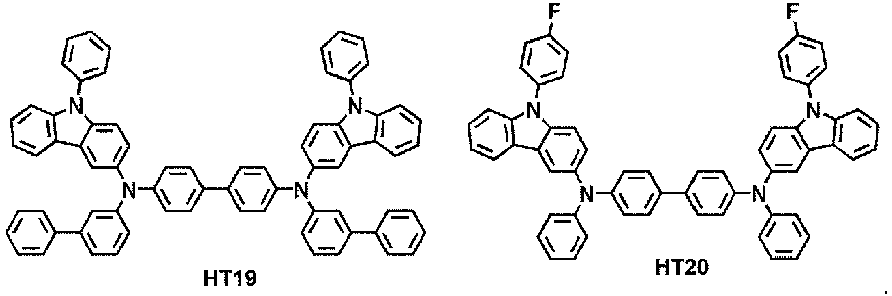

A thickness of the hole transport region may be from about 100 Å to about 10,000 Å, for example, about 100 Å to about 1,000 Å. When the hole transport region includes a hole injection layer, a hole transport layer, or any combination thereof, a thickness of the hole injection layer may be in a range of about 100 Å to about 10,000 Å, for example, about 100 Å to about 1,000 Å, and a thickness of the hole transport layer may be in a range of about 50 Å to about 2,000 Å, for example about 100 Å to about 1,500 Å. When the thicknesses of the hole transport region, the hole injection layer, and the hole transport layer are within these ranges, satisfactory hole transporting characteristics may be obtained without a substantial increase in driving voltage.

The hole transport region may further include, in addition to these materials, a charge-generation material for the improvement of conductive properties. The charge-generation material may be homogeneously or non-homogeneously dispersed in the hole transport region.

The charge-generation material may be, for example, a p-dopant. The p-dopant may be one selected from a quinone derivative, a metal oxide, and a cyano group-containing compound, but embodiments of the present disclosure are not limited thereto. Non-limiting examples of the p-dopant are a quinone derivative, such as tetracyanoquinonedimethane (TCNQ) or 2,3,5,6-tetrafluoro-tetracyano-1,4-benzoquinonedimethane (F4-TCNQ); a metal oxide, such as a tungsten oxide or a molybdenium oxide; and a cyano group-containing compound, such as Compound HT-D1 below, but are not limited thereto:

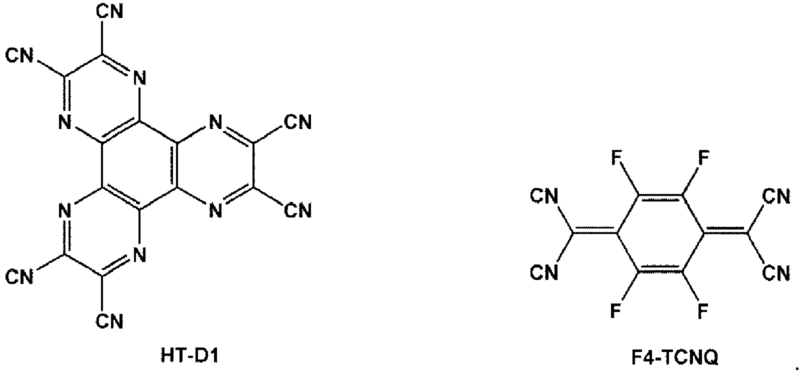

The hole transport region may include a buffer layer.

Also, the buffer layer may compensate for an optical resonance distance according to a wavelength of light emitted from the emission layer, and thus, efficiency of a formed organic light-emitting device may be improved.

Meanwhile, when the hole transport region includes an electron blocking layer, a material for the electron blocking layer may be a material(s) for the hole transport region described above, a material(s) for a host to be explained later, or any combination thereof. However, the material for the electron blocking layer is not limited thereto. For example, when the hole transport region includes an electron blocking layer, a material for the electron blocking layer may be mCP, which will be explained later.

Then, an emission layer may be formed on the hole transport region by vacuum deposition, spin coating, casting, LB deposition, or the like. When the emission layer is formed by vacuum deposition or spin coating, the deposition or coating conditions may be similar to those applied in forming the hole injection layer although the deposition or coating conditions may vary according to a compound that is used to form the emission layer.

The emission layer may include a host and a dopant, and the dopant may include the organometallic compound represented by Formula 1.

The host may include TPBi, TBADN, ADN (also referred to as "DNA"), CBP, CDBP, TCP, mCP, Compound H50, Compound H51, Compound 52, or any combination thereof:

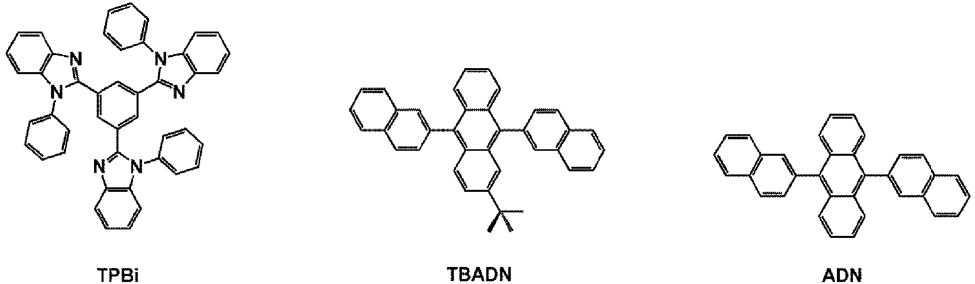

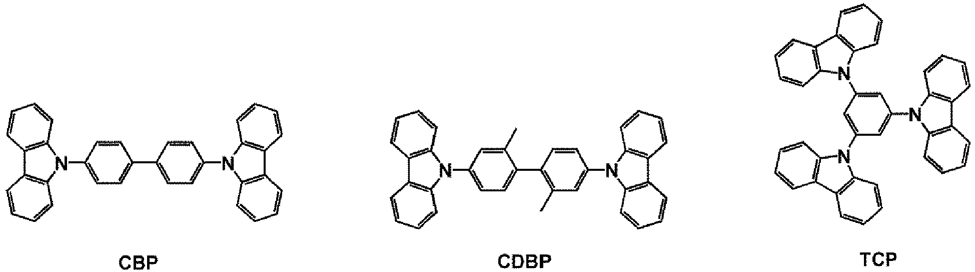

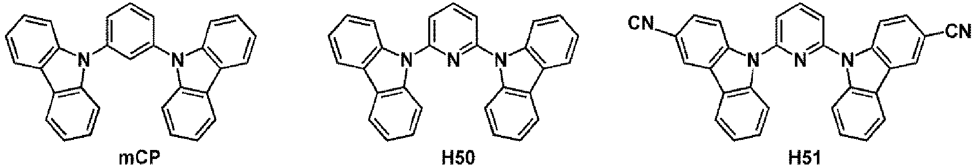

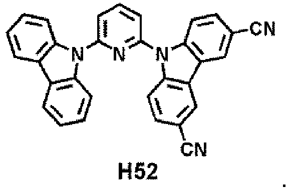

When the organic light-emitting device is a full-color organic light-emitting device, the emission layer may be patterned into a red emission layer, a green emission layer, and/or a blue emission layer. In one or more embodiments, due to a stacked structure including a red emission layer, a green emission layer, and/or a blue emission layer, the emission layer may emit white light.

When the emission layer includes a host and a dopant, an amount of the dopant may be in a range of about 0.01 parts by weight to about 15 parts by weight based on 100 parts by weight of the host, but embodiments of the present disclosure are not limited thereto.

A thickness of the emission layer may be in a range of about 100 Å to about 1,000 Å, for example, about 200 Å to about 600 Å. When the thickness of the emission layer is within this range, excellent light-emission characteristics may be obtained without a substantial increase in driving voltage.

Then, an electron transport region may be disposed on the emission layer.

The electron transport region may include a hole blocking layer, an electron transport layer, an electron injection layer, or any combination thereof.

For example, the electron transport region may have a hole blocking layer/electron transport layer/electron injection layer structure or an electron transport layer/electron injection layer structure, but the structure of the electron transport region is not limited thereto. The electron transport layer may have a single-layered structure or a multi-layered structure including two or more different materials.

Conditions for forming the hole blocking layer, the electron transport layer, and the electron injection layer which constitute the electron transport region may be understood by referring to the conditions for forming the hole injection layer.

When the electron transport region includes a hole blocking layer, the hole blocking layer may include, for example, BCP, Bphen, BAlq, or any combination thereof, but embodiments of the present disclosure are not limited thereto:

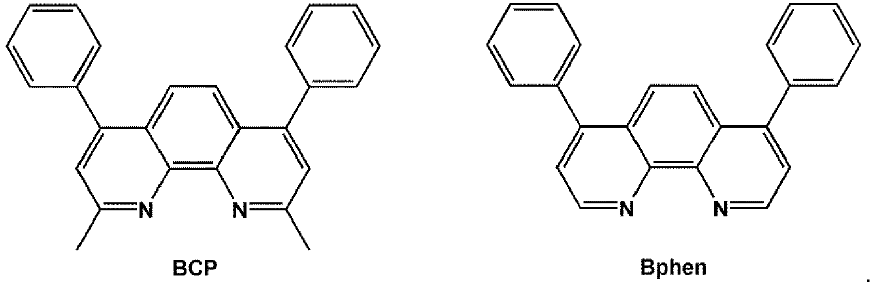

A thickness of the hole blocking layer may be from about 20 Å to about 1,000 Å, for example, about 30 Å to about 300 Å. When the thickness of the hole blocking layer is within these ranges, the hole blocking layer may have excellent hole blocking characteristics without a substantial increase in driving voltage.

The electron transport layer may include BCP, Bphen, Alq₃, BAlq, TAZ, NTAZ, or any combination thereof:

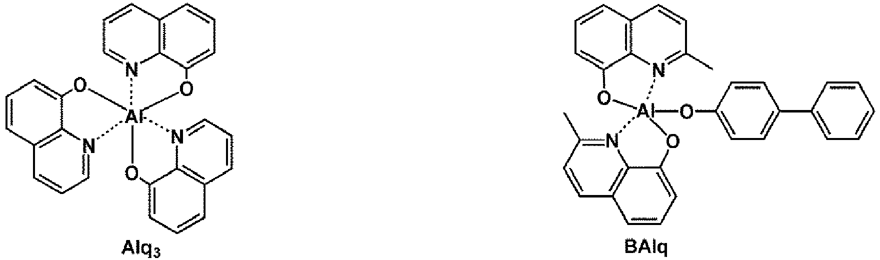

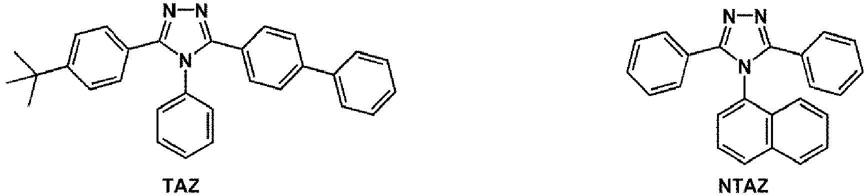

In one or more embodiments, the electron transport layer may include one of ET1 and ET25 or any combination thereof, but are not limited thereto:

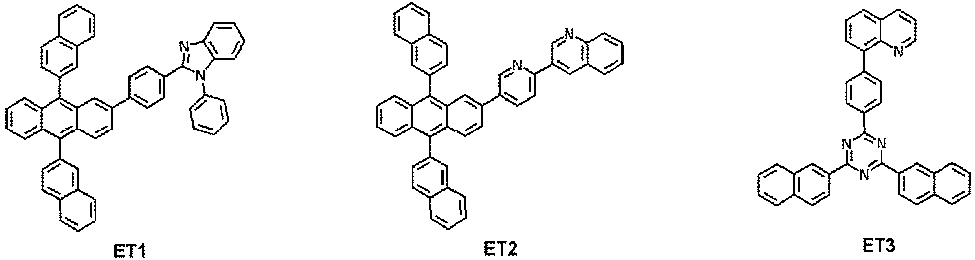

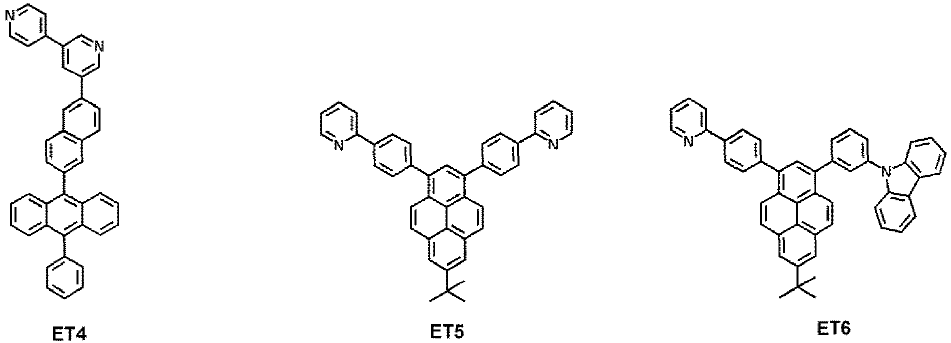

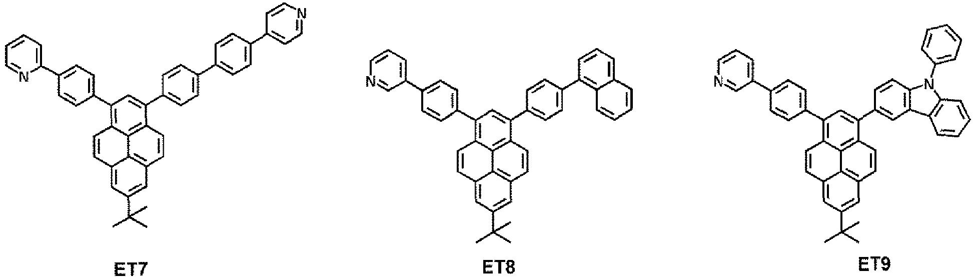

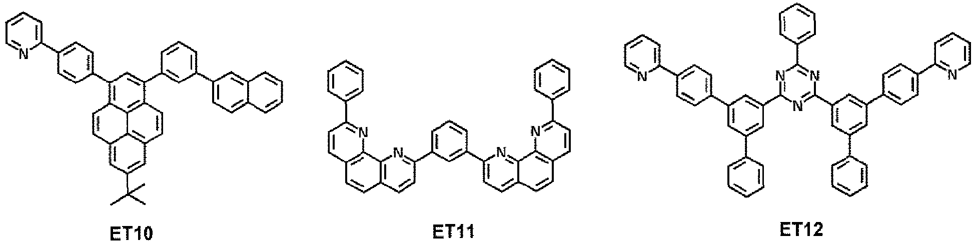

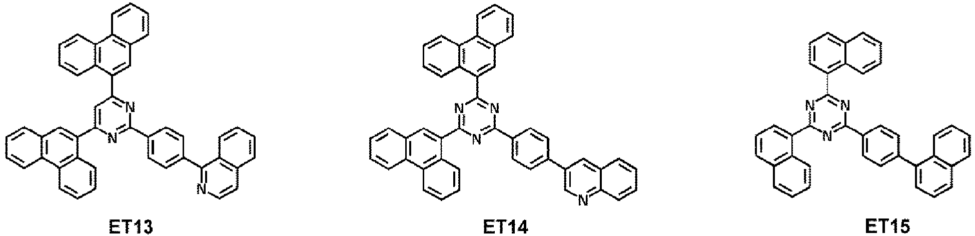

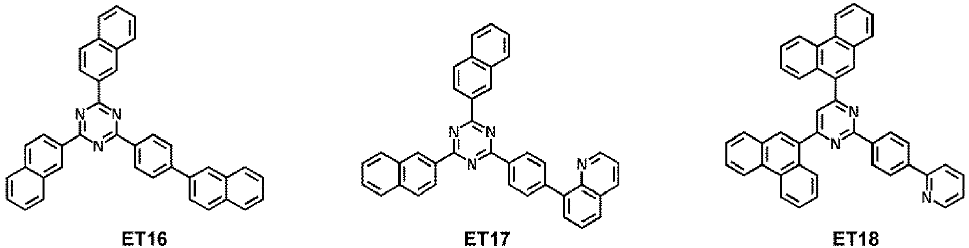

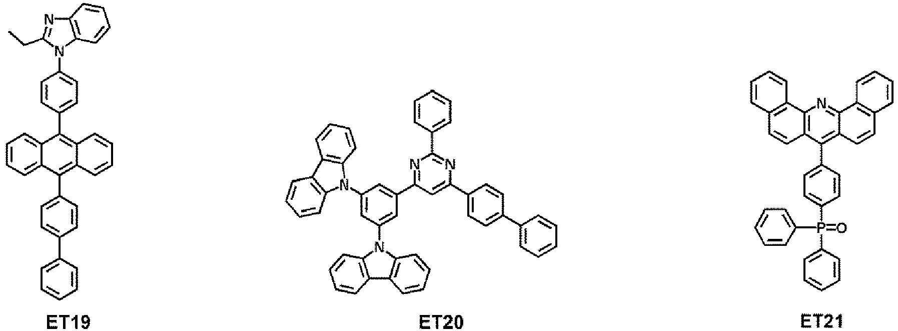

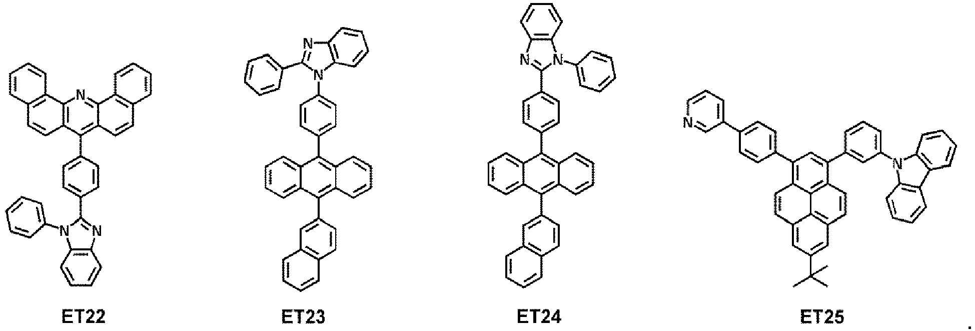

A thickness of the electron transport layer may be from about 100 Å to about 1,000 Å, for example, about 150 Å to about 500 Å. When the thickness of the electron transport layer is within the range described above, the electron transport layer may have satisfactory electron transport characteristics without a substantial increase in driving voltage.

Also, the electron transport layer may further include, in addition to the materials described above, a metal-containing material.

The metal-containing material may include a Li complex. The Li complex may include, for example, Compound ET-D1 (LiQ) or ET-D2:

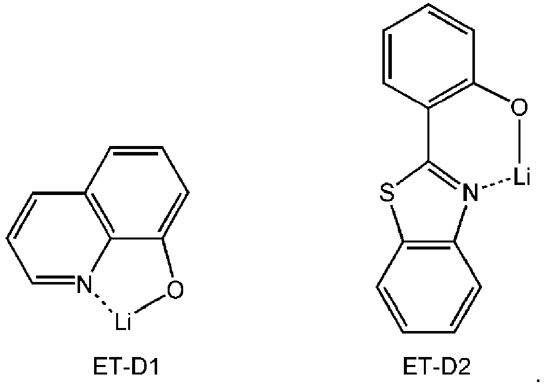

The electron transport region may include an electron injection layer that promotes flow of electrons from the second electrode 19 thereinto.

The electron injection layer may include LiF, NaCl, CsF, Li₂O, BaO, or any combination thereof.

A thickness of the electron transport layer may be from about 1 Å to about 100 Å, for example, about 3 Å to about 90 Å. When a thickness of the electron injection layer is within these ranges, satisfactory electron injection characteristics may be obtained without substantial increase in driving voltage.

The second electrode 19 is disposed on the organic layer 15. The second electrode 19 may be a cathode. A material for forming the second electrode 19 may be selected from metal, an alloy, an electrically conductive compound, or any combination thereof, which have a relatively low work function. For example, lithium (Li), magnesium (Mg), aluminum (Al), aluminum-lithium (Al-Li), calcium (Ca), magnesium-indium (Mg-In), or magnesium-silver (Mg-Ag) may be formed as the material for forming the second electrode 19. To manufacture a top-emission type light-emitting device, a transmissive electrode formed using ITO or IZO may be used as the second electrode 19.

Hereinbefore, the organic light-emitting device according to an embodiment has been described in connection with the FIGURE.

Another aspect of the present disclosure provides a diagnostic composition including at least one organometallic compound represented by Formula 1.

The organometallic compound represented by Formula 1 provides high luminescent efficiency. Accordingly, a diagnostic composition including the organometallic compound may have high diagnostic efficiency.

The diagnostic composition may be used in various applications including a diagnosis kit, a diagnosis reagent, a biosensor, and a biomarker.

The term "C₁-C₆₀ alkyl group" as used herein refers to a linear or branched saturated aliphatic hydrocarbon monovalent group having 1 to 60 carbon atoms, and examples thereof include a methyl group, an ethyl group, a propyl group, an isobutyl group, a sec-butyl group, a tert-butyl group, a pentyl group, an isoamyl group, and a hexyl group. The term "C₁-C₆₀ alkylene group" as used herein refers to a divalent group having the same structure as the C₁-C₆₀ alkyl group.

The term "C₁-C₆₀ alkoxy group" as used herein refers to a monovalent group represented by -OA₁₀₁ (wherein A₁₀₁ is the C₁-C₆₀ alkyl group), and examples thereof include a methoxy group, an ethoxy group, and an isopropyloxy group.

The term "C₂-C₆₀ alkenyl group" as used herein refers to a hydrocarbon group having at least one carbon-carbon double bond in the middle or at the terminus of the C₂-C₆₀ alkyl group, and examples thereof include an ethenyl group, a propenyl group, and a butenyl group. The term "C₂-C₆₀ alkenylene group" as used herein refers to a divalent group having the same structure as the C₂-C₆₀ alkenyl group.

The term "C₂-C₆₀ alkynyl group" as used herein refers to a hydrocarbon group having at least one carbon-carbon triple bond in the middle or at the terminus of the C₂-C₆₀ alkyl group, and examples thereof include an ethynyl group, and a propynyl group. The term "C₂-C₆₀ alkynylene group" as used herein refers to a divalent group having the same structure as the C₂-C₆₀ alkynyl group.

The term "C₃-C₁₀ cycloalkyl group" as used herein refers to a monovalent saturated hydrocarbon monocyclic group having 3 to 10 carbon atoms, and non-limiting examples thereof include a cyclopropyl group, a cyclobutyl group, a cyclopentyl group, a cyclohexyl group, and a cycloheptyl group. The term "C₃-C₁₀ cycloalkylene group" as used herein refers to a divalent group having the same structure as the C₃-C₁₀ cycloalkyl group.

The term "C₁-C₁₀ heterocycloalkyl group" as used herein refers to a monovalent saturated monocyclic group having at least one heteroatom selected from N, O, P, Si and S as a ring-forming atom and 1 to 10 carbon atoms, and non-limiting examples thereof include a tetrahydrofuranyl group, and a tetrahydrothiophenyl group. The term "C₁-C₁₀ heterocycloalkylene group" as used herein refers to a divalent group having the same structure as the C₁-C₁₀ heterocycloalkyl group.

The term "C₃-C₁₀ cycloalkenyl group" as used herein refers to a monovalent monocyclic group that has 3 to 10 carbon atoms and at least one carbon-carbon double bond in the ring thereof and no aromaticity, and non-limiting examples thereof include a cyclopentenyl group, a cyclohexenyl group, and a cycloheptenyl group. The term "C₃-C₁₀ cycloalkenylene group" as used herein refers to a divalent group having the same structure as the C₃-C₁₀ cycloalkenyl group.

The term "C₁-C₁₀ heterocycloalkenyl group" as used herein refers to a monovalent monocyclic group that has at least one heteroatom selected from N, O, P, Si, and S as a ring-forming atom, 1 to 10 carbon atoms, and at least one double bond in its ring. Examples of the C₁-C₁₀ heterocycloalkenyl group are a 2,3-dihydrofuranyl group, and a 2,3-dihydrothiophenyl group. The term "C₁-C₁₀ heterocycloalkenylene group" as used herein refers to a divalent group having the same structure as the C₁-C₁₀ heterocycloalkenyl group.

The term "C₆-C₆₀ aryl group" as used herein refers to a monovalent group having a carbocyclic aromatic system having 6 to 60 carbon atoms, and the term "C₆-C₆₀ arylene group" as used herein refers to a divalent group having a carbocyclic aromatic system having 6 to 60 carbon atoms. Non-limiting examples of the C₆-C₆₀ aryl group include a phenyl group, a naphthyl group, an anthracenyl group, a phenanthrenyl group, a pyrenyl group, and a chrysenyl group. When the C₆-C₆₀ aryl group and the C₆-C₆₀ arylene group each include two or more rings, the rings may be fused to each other.

The term "C₁-C₆₀ heteroaryl group" as used herein refers to a monovalent group having a cyclic aromatic system that has at least one heteroatom selected from N, O, P, Si, and S as a ring-forming atom, in addition to 1 to 60 carbon atoms. The term "C₁-C₆₀ heteroarylene group" as used herein refers to a divalent group having a cyclic aromatic system that has at least one heteroatom selected from N, O, P, Si, and S as a ring-forming atom, in addition to 1 to 60 carbon atoms. Non-limiting examples of the C₁-C₆₀ heteroaryl group include a pyridinyl group, a pyrimidinyl group, a pyrazinyl group, a pyridazinyl group, a triazinyl group, a quinolinyl group, and an isoquinolinyl group. When the C₁-C₆₀ heteroaryl group and the C₁-C₆₀ heteroarylene group each include two or more rings, the rings may be fused to each other.

The term "C₆-C₆₀ aryloxy group" used herein indicates -OA₁₀₂ (wherein A₁₀₂ is the C₆-C₆₀ aryl group), and a C₆-C₆₀ arylthio group used herein indicates -SA₁₀₃ (wherein A₁₀₃ is the C₆-C₆₀ aryl group).

The term "monovalent non-aromatic condensed polycyclic group" as used herein refers to a monovalent group (for example, having 8 to 60 carbon atoms) having two or more rings condensed to each other, only carbon atoms as ring-forming atoms, and no aromaticity in its entire molecular structure. Examples of the monovalent non-aromatic condensed polycyclic group include a fluorenyl group. The term "divalent non-aromatic condensed polycyclic group" as used herein refers to a divalent group having the same structure as the monovalent non-aromatic condensed polycyclic group.

The term "monovalent non-aromatic condensed heteropolycyclic group" as used herein refers to a monovalent group (for example, having 2 to 60 carbon atoms) having two or more rings condensed to each other, a heteroatom selected from N, O, P, Si, and S, other than carbon atoms, as a ring-forming atom, and no aromaticity in its entire molecular structure. Non-limiting examples of the monovalent non-aromatic condensed heteropolycyclic group include a carbazolyl group. The term "divalent non-aromatic condensed heteropolycyclic group" as used herein refers to a divalent group having the same structure as the monovalent non-aromatic condensed heteropolycyclic group.

The term "C₅-C₆₀ carbocyclic group (or, C₅-C₃₀ carbocyclic group)" as used herein refers to a saturated or unsaturated cyclic group having, as a ring-forming atom, 5 to 60 carbon atoms (or, 5 to 30 carbon atoms) only. The C₅-C₆₀ carbocyclic group (or, C₅-C₃₀ carbocyclic group) may be a monocyclic group or a polycyclic group.

The term "C₁-C₆₀ heterocyclic group (or, C₁-C₃₀ heterocyclic group)" as used herein refers to a saturated or unsaturated cyclic group having, as a ring-forming atom, at least one heteroatom selected from N, O, Si, P, and S other than 1 to 60 carbon atoms (or, 1 to 30 carbon atoms). The C₁-C₆₀ heterocyclic group (or, C₁-C₃₀ heterocyclic group) may be a monocyclic group or a polycyclic group.

A substituent(s) of the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₁-C₆₀ heteroaryl group, the substituted monovalent non-aromatic condensed polycyclic group and substituted monovalent non-aromatic condensed heteropolycyclic group may each independently be:
deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazino group, a hydrazono group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, or a C₁-C₆₀ alkoxy group;
a C₁-C₆₀ alkyl group, a C₂-C₆₀ alkenyl group, a C₂-C₆₀ alkynyl group, or a C₁-C₆₀ alkoxy group, each substituted with deuterium, -F, -Cl, -Br, -I, -CD₃, -CD₂H, -CDH₂, -CF₃, - CF₂H, -CFH₂, a hydroxyl group, a cyano group, a nitro group, an amino group, an amidino group, a hydrazino group, a hydrazono group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₁₁)(Q₁₂), - Si(Q₁₃)(Q₁₄)(Q₁₅), -Ge(Q₁₃)(Q₁₄)(Q₁₅), -B(Q₁₆)(Q₁₇), -P(=O)(Q₁₈)(Q₁₉), -P(Q₁₈)(Q₁₉), or any combination thereof;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, or a monovalent non-aromatic condensed heteropolycyclic group, each unsubstituted or substituted with deuterium, -F, -Cl, -Br, -I, -CD₃, -CD₂H, -CDH₂, -CF₃, - CF₂H, -CFH₂, a hydroxyl group, a cyano group, a nitro group, an amino group, an amidino group, a hydrazino group, a hydrazono group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₂₁)(Q₂₂), - Si(Q₂₃)(Q₂₄)(Q₂₅), -Ge(Q₂₃)(Q₂₄)(Q₂₅), -B(Q₂₆)(Q₂₇), -P(=O)(Q₂₈)(Q₂₉), -P(Q₂₈)(Q₂₉), or any combination thereof;
-N(Q₃₁)(Q₃₂), -Si(Q₃₃)(Q₃₄)(Q₃₅), -Ge(Q₃₃)(Q₃₄)(Q₃₅), -B(Q₃₆)(Q₃₇), -P(=O)(Q₃₈)(Q₃₉) or -P(Q₃₈)(Q₃₉); or
any combination thereof.
Q₁ to Q₉, Q₁₁ to Q19, Q21 to Q29, and Q31 to Q39 may each independently be hydrogen; deuterium; -F; -Cl; -Br; -I; a hydroxyl group; a cyano group; a nitro group; an amino group; an amidino group; a hydrazine group; a hydrazone group; a carboxylic acid group or a salt thereof; a sulfonic acid group or a salt thereof; a phosphoric acid group or a salt thereof; a C₁-C₆₀ alkyl group unsubstituted or substituted with deuterium, a C₁-C₆₀ alkyl group, a C₆-C₆₀ aryl group, or any combination thereof; a C₂-C₆₀ alkenyl group; a C₂-C₆₀ alkynyl group; a C₁-C₆₀ alkoxy group; a C₃-C₁₀ cycloalkyl group; a C₁-C₁₀ heterocycloalkyl group; a C₃-C₁₀ cycloalkenyl group; a C₁-C₁₀ heterocycloalkenyl group; a C₆-C₆₀ aryl group unsubstituted or substituted with a C₁-C₆₀ alkyl group, a C₆-C₆₀ aryl group, or any combination thereof; a C₆-C₆₀ aryloxy group; a C₆-C₆₀ arylthio group; a C₁-C₆₀ heteroaryl group; a monovalent non-aromatic condensed polycyclic group; or a monovalent non-aromatic condensed heteropolycyclic group.

Hereinafter, a compound and an organic light-emitting device according to embodiments are described in detail with reference to Synthesis Example and Examples. However, the organic light-emitting device is not limited thereto. The wording "B was used instead of A" used in describing Synthesis Examples means that an amount of B used was identical to an amount of A used, in terms of a molar equivalent.

### [Examples]

### Synthesis Example 1 (Compound 1)

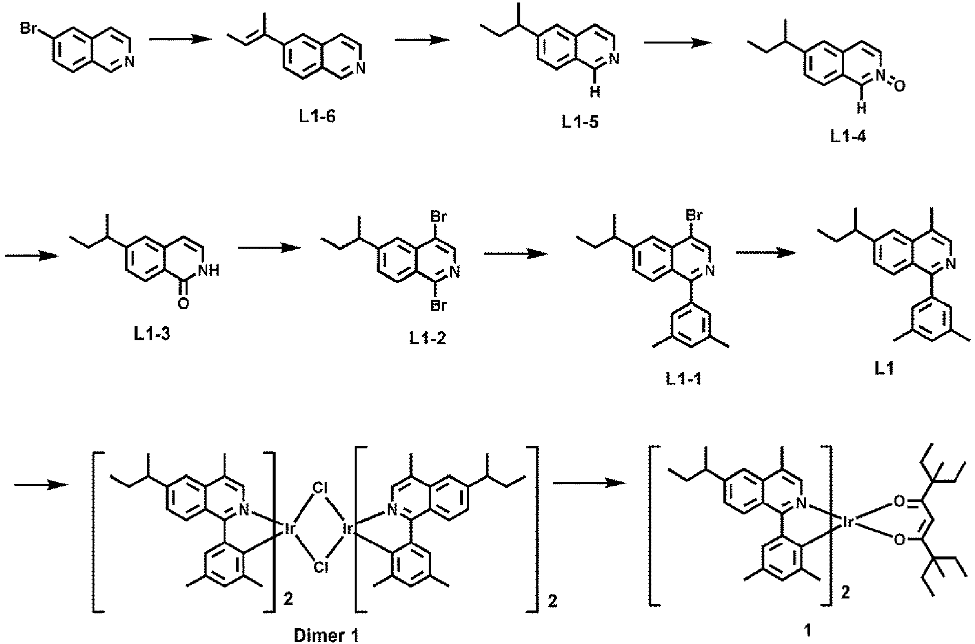

### Synthesis of Intermediate L1-6

44 g (181.4 mmol) of 6-bromoisoquinoline was mixed with 400 ml of tetrahydrofuran (THF) and 100 ml of water, 33.0 g (181.4 mmol) of sec-butenylboronic acid, 14.7 g (12.7 mmol) of a Pd(PPh₃)₄, and 63.0 g (453.0 mmol) of K₂CO₃ were added thereto, and the mixture was heated under reflux at a temperature of 80 °C for 18 hours. After the completion of the reaction, the reaction mixture was concentrated under reduced pressure, and an extraction process was performed thereon using 200 ml of ethylacetate. An organic layer obtained therefrom was then dried with magnesium sulfate, and then, subjected to distillation under reduced pressure. The resulting product was purified by liquid chromatography, thereby obtaining 18.0 g (yield: 46 %) of Intermediate L1-6.

### Synthesis of Intermediate L1-5

10 g (55.3 mmol) of Intermediate L1-6 was mixed with 200 ml of methanol, and 1.0 g (10 wt%) of a Pd(PPh₃)₄ was added thereto. By injecting hydrogen thereto, the mixed solution was stirred for 18 hours. After the completion of the reaction, the reaction mixture was passed through a celite pad, and then, concentrated under reduced pressure. The resulting product was purified by liquid chromatography, thereby obtaining 9.7 g (yield: 95 %) of Intermediate L1-5.

### Synthesis of Intermediate L1-4

9.2 g (50.0 mmol) of Intermediate L1-5 was mixed with 60 ml of CH₂Cl₂, and 100.0 mmol of 3-chlorobenzoic acid (mCPBA) was slowly added thereto dropwise at a temperature of 0 °C. The resulting product was stirred for one day. After the completion of the reaction, an extraction process was performed on the reaction mixture by using 6N KOH, and an organic layer obtained therefrom was dried with magnesium sulfate. The resulting product was purified by liquid chromatography, thereby obtaining 9.9 g (yield: 99 %) of Intermediate L1-4.

### Synthesis of Intermediate L1-3

9.5 g (47.2 mmol) of Intermediate L1-4 was mixed with acetic anhydride, and the mixed solution was heated under reflux. After the completion of the reaction, an extraction process was performed on the reaction mixture by using saturated sodium bicarbonate. An organic layer obtained therefrom was dried with magnesium sulfate, and then, was subjected to distillation under reduced pressure. The resulting product was purified by liquid chromatography, thereby obtaining 7.1 g (yield: 75 %) of Intermediate L1-3.

### Synthesis of Intermediate L1-2

7.0 g (34.8 mmol) of Intermediate L1-3 was mixed with 350 ml of CH₂Cl₂, and 24 g (87.4 mmol) of POBr₃ was slowly added thereto dropwise at a temperature of 0 °C. Then, 5.0 ml of DMF was added thereto, and the resulting solution was stirred for one day at room temperature. After the completion of the reaction, an extraction process was performed on the reaction mixture by using saturated sodium bicarbonate. An organic layer obtained therefrom was dried with magnesium sulfate, and then, was subjected to distillation under reduced pressure. The resulting product was purified by liquid chromatography, thereby obtaining 7.7 g (yield: 65 %) of Intermediate L1-2.

### Synthesis of Intermediate L1-1

1.7 g (4.9 mmol) of Intermediate L1-2 was mixed with 40 ml of toluene and 8 ml of ethanol. 0.8 g (5.23 mmol) of 3,5-dimethylphenylboronic acid, 0.4 g (0.4 mmol) of a Pd(PPh₃)₄, 2N Na₂CO₃ in H₂O were added thereto, and the mixture was heated under reflux at a temperature of 80 °C for 18 hours. After the completion of the reaction, the reaction mixture was concentrated under reduced pressure, and an extraction process was performed thereon using 100 ml of ethylacetate. An organic layer obtained therefrom was then dried with magnesium sulfate, and then, subjected to distillation under reduced pressure. The resulting product was purified by liquid chromatography, thereby obtaining 1.5 g (yield: 70 %) of Intermediate L1-1.

### Synthesis of Intermediate L1

1.5 g (4.1 mmol) of Intermediate L1-1 was mixed with 80 ml of THF. 2.1 g (8.2 mmol) of DABAL-Me₃ (triethylenediaminine bis(trimethylaluminum)), 0.4 g (0.4 mmol) of a Pd(PPh₃)₄, and 0.4 g (0.4 mmol) of Xphos (2-dicyclohexylphosphino-2',4',6'-triisopropylbiphenyl) were added thereto, the mixture was heated under reflux at a temperature of 80 °C for 18 hours. After the completion of the reaction, the reaction mixture was neutralized with HCl, and then, an extraction process was performed thereon using 100 ml of ethylacetate. An organic layer obtained therefrom was then dried with magnesium sulfate, and then, subjected to distillation under reduced pressure. The resulting product was purified by liquid chromatography, thereby obtaining 0.9 g (yield: 70 %) of Intermediate L1.

### Synthesis of Dimer 1

0.9 g (2.96 mmol) of Intermediate L1 and 0.5 g (1.41 mmol) of iridium chloride were mixed with 45 mL of 2-ethoxyethanol and 15 mL of distilled water, and the resulting solution was stirred under reflux for 24 hours. When the reaction procedded, the temperature was lowered to room temperature. A solid produced therefrom was separated by filtration, and then, sufficiently washed using water/methanol/hexane in the stated order. The resulting solid was dried in a vacuum oven, thereby obtaining 0.95 g (yield: 77 %) of Dimer 1.

### Synthesis of Compound 1

0.95 g (0.57 mmol) of Dimer 1, 0.34 g (1.43 mmol) of 3,7-diethyl-3,7-dimethylnonane-4,6-dione, and 0.18 g (1.71 mmol) of Na₂CO₃ were mixed with 15 mL of ethoxyethanol, and the mixed solution was stirred for 24 hours. When the reaction procedded, a solid obtained by filtering the reaction mixture was sufficiently with ethanol and hexane, and then, was subjected to column chromatography under conditions of dichloromethane:n-hexane=1:1(v/v), thereby obtaining 0.5 g (yield: 45 %) of Compound 1.

LC-MS m/z = 1037(M+H)⁺

### Synthesis Example 2 (Compound 3)

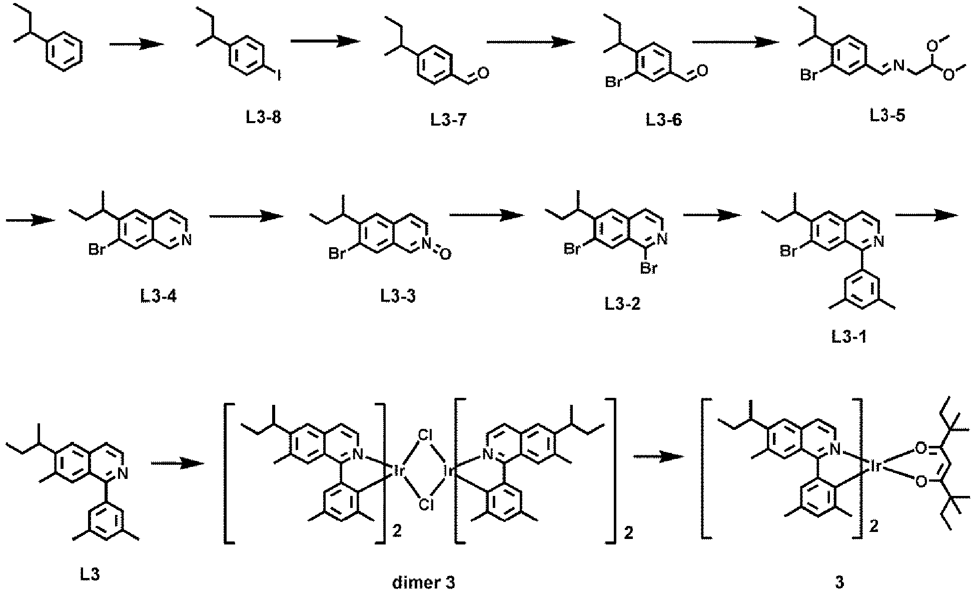

### Synthesis of Intermediate L3-8

72 ml (461.3 mmol) of sec-butylbenzene was mixed with 450 ml of acetonitrile, and 88.0 g (346.0 mmol) of iodine and 122.6 g (346.0 mmol) of Selectfluor (1-chloromethyl-4-fluoro-1,4-diazoniabicyclo[2.2.2]octane bis(tetrafluoroborate)) were added thereto. The mixture was then stirred at a temperature of 60 °C for 4 hours. After the completion of the reaction, the reaction mixture was mixed with dichloromethane, and an extraction process was performed thereon using saturated sodium thiosulfate. An organic layer obtained therefrom was then dried with magnesium sulfate, and subjected to distillation under reduced pressure. The resulting product was purified by liquid chromatography, thereby obtaining 75.0 g (yield: 63 %) of Intermediate L3-8.

LC-MS m/z = 261 (M+H)⁺

### Synthesis of Intermediate L3-7

148 g (512.9 mmol) of Intermediate L3-8 was mixed with 500 ml of tetrahydrofuran, and 205 ml (205 mmol) of 1.0 M isopropylmagnesium chloride solution was slowly added thereto. The mixture was then stirred at a temperature of 0 °C. After 10 minutes, 256 ml (410 mmol) of a 1.6 M n-butyl lithium (BuLi) solution was slowly added thereto, and then, the mixed solution was stirred for 1 hour. Afterwards, 51 ml (666.7 mmol) of DMF was slowly added thereto dropwise at a temperature of -10 °C, and the resulting solution was stirred at room temperature for 1 hour. After the completion of the reaction, an extraction process was performed on the reaction mixture by using ethylacetate and an aqueous hydrochloric acid solution. An organic layer obtained therefrom was dried with dried with magnesium sulfate, and subjected to distillation under reduced pressure. The resulting product was purified by liquid chromatography, thereby obtaining 83.2 g (yield: 100 %) of Intermediate L3-7.

LC-MS m/z = 163(M+H)⁺

### Synthesis of Intermediate L3-6

44g (326.6mmol) of AlCl₃ was added to 100 ml of dichloromethane, and the mixture was heated at a temperature of 40 °C. 30 g (186.6 mmol) of Intermediate L3-7 mixed with 6 ml of dichloromethane was slowly added thereto, and the resulting mixture was then stirred for 30 minutes. 10 ml (205.3 mmol) of bromine was added thereto for 1 hour. After the completion of the reaction, the reaction mixture was slowly added to iced water, and an organic layer obtained therefrom was then dried with magnesium sulfate, and subjected to distillation under reduced pressure. The resulting product was purified by liquid chromatography, thereby obtaining 27 g (yield: 60 %) of Intermediate L3-6.

LC-MS m/z = 241(M+H)⁺

### Synthesis of Intermediate L3-5

27 g (112.7 mmol) of Intermediate L3-6 was mixed with 100 ml of chloroform, and 18 ml (169 mmol) of aminoacetaldehyde dimethyl acetal was slowly added thereto at room temperature. After 1 hour, the mixture was heated at a temperature of 100 °C to remove a solvent therefrom. The resulting reaction mixture was then cooled to room temperature, and without a separate purification process, Intermediate L3-5 was obtained.

### Synthesis of Intermediate L3-4

37 g (113.6 mmol) of Intermediate L3-5 was mixed with 100 ml of chloroform, and 11 ml (113.6 mmol) of ethyl chloroformate and 16 ml (136.3 mmol) of trimethylphosphite were slowly added dropwise thereto at a temperature of 0 °C sequentially in the stated order. The reaction mixture obtained therefrom was then stirred at room temperature for 48 hours, and 454 ml (454 mmol) of 1.0 M TiCl₄ was slowly added dropwise thereto at a temperature of 0 °C. The resulting mixture was then heated under reflux for about 48 hours. After the completion of the reaction, the reaction mixture was cooled to room temperature, and then, added to iced water. A water layer obtained therefrom was washed using dichloromethane, and added to 256 g (908.5 mmol) of tartrate solution. The mixed solution was neutralized with saturated NaHCOs solution, and an extraction process was performed thereon using dichloromethane. An organic layer obtained therefrom was purified by liquid chromatography, thereby obtaining 11 g (yield: 38 %) of Intermediate L3-4.

LC-MS m/z = 264(M+H)⁺

### Synthesis of Intermediate L3-3

11 g (43.2 mmol) of Intermediate L3-4 was mixed with 90 ml of dichloromethane, and 15 g (86.4 mmol) of mCPBA was slowly added dropwise thereto at a temperature of 0 °C. The reaction mixture was stirred for about 18 hours at room temperature, and then, an extraction process was performed thereon using 6 N KOH solution. An organic layer obtained therefrom was dried with magnesium sulfate, and without a separate purification process, 12 g (yield: 100 %) of Intermediate L3-3 was obtained.

### Synthesis of Intermediate L3-2

12 g (43.2 mmol) of Intermediate L3-3 was mixed with 90 ml of dichloromethane, and 15 g (52 mmol) of POBr₃ was slowly added dropwise thereto at a temperature of 0 °C. Then, 1.7 ml (22 mmol) of DMF was slowly added thereto, and the mixture was stirred for about 18 hours at room temperature. After the completion of the reaction, the reaction solution was neutralized with a saturated NaHCOs solution, and an extraction process was performed thereon using dichloromethane. An organic layer obtained therefrom was dried with magnesium sulfate, and purified by liquid chromatography, thereby obtaining 3.8 g (yield: 26 %) of Intermediate L3-2.

LC-MS m/z = 342(M+H)⁺

### Synthesis of Intermediate L3-1

3.8 g (10.9 mmol) of Intermediate L3-2 was mixed with 40 ml of toluene and 8 ml of EtOH, and 1.5 g (9.8 mmol) of 3,5-dimethylphenyl boronic acid, 0.6 g (0.5 mmol) of a Pd(PPh₃)₄, and 8 ml of 2.0 N Na₂CO₃ solution were added thereto. The mixture was then heated under reflux at a temperature of 85 °C for 18 hours. After the completion of the reaction, an extraction process was performed thereon using 50 ml of ethylacetate. An organic layer obtained therefrom was dried with magnesium sulfate, and purified by liquid chromatography, thereby obtaining 3.6 g (yield: 90 %) of Intermediate L3-1.

LC-MS m/z = 368(M+H)⁺

### Synthesis of Intermediate L3

0.9 g (yield: 90 %) of Intermediate L3 was synthesized in the same manner as in the synthesis of Intermediate L1 according to Synthesis Example 1, except that Intermediate L3-1 was used instead of Intermediate L1-1. The synthesized compound was identified by LCMS.

LC-MS m/z = 304(M+H)⁺

### Synthesis of Dimer 3

Dimer 3 (yield: 70 %) was synthesized in the same manner as in the synthesis of Dimer 1 according to Synthesis Example 1, except that Intermediate L3 was used instead of Intermediate L1.

### Synthesis of Compound 3

Compound 3 (yield: 45 %) was synthesized in the same manner as in the synthesis of Compound 1 according to Synthesis Example 1, except that Dimer 3 was used instead of Dimer 1 and 3,3,7,7-tetramethylnonane-4,6-dione was used instead of 3,7-diethyl-3,7-dimethylnonane-4,6-dione. The synthesized compound was identified by LCMS.

LC-MS m/z = 1009(M+H)⁺

### Synthesis Example 3 (Compound 7)

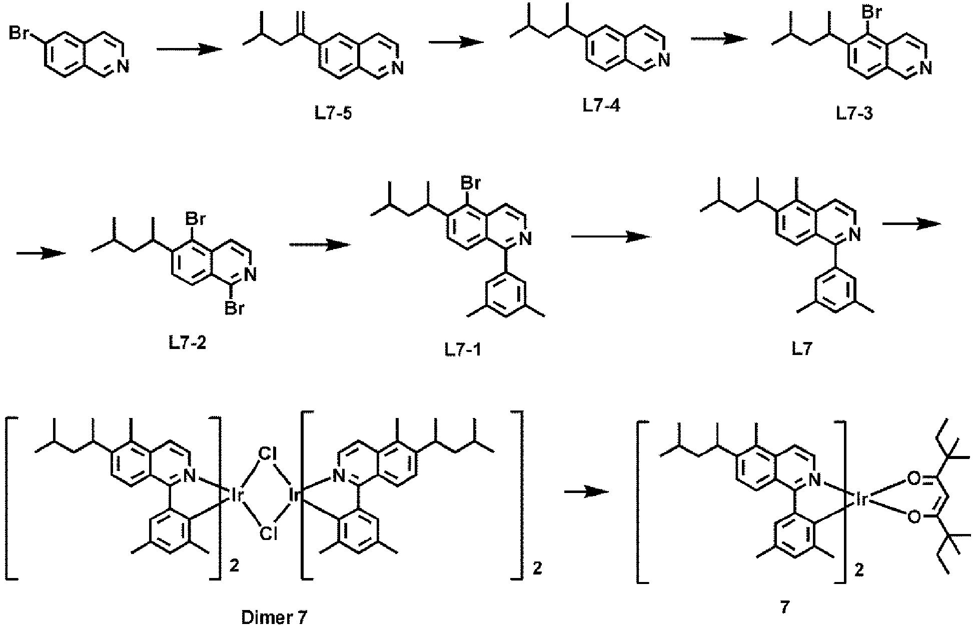

### Synthesis of Intermediate L7-5

20 g (96.1 mmol) of 6-bromoisoquinoline was mixed with 120 ml of tetrahydrofuran, and 11 g (9.6 mmol) of Pd(PPh₃)₄, 18 g (144.2 mmol) of (4-methylpent-1-en-2-yl)boronic acid, 33.2 g (240.5 mmol) of K₂CO₃, and 30 ml of water were added thereto. The mixture was then heated under reflux at a temperature of 80 °C for about 18 hours. After the completion of the reaction, an extraction process was performed thereon using ethylacetate. An organic layer obtained therefrom was dried with magnesium sulfate, and purified by liquid chromatography, thereby obtaining 16 g (yield: 80 %) of Intermediate L7-5.

LC-MS m/z = 212(M+H)⁺

### Synthesis of Intermediate L7-4

Intermediate L7-4 (yield: 95 %) was synthesized in the same manner as in the synthesis of Intermediate L1-5 according to Synthesis Example 1, except that Intermediate L7-5 was used instead of Intermediate L1-6.

LC-MS m/z = 214(M+H)⁺

### Synthesis of Intermediate L7-3

5.8 g (43.2 mmol) of AlCl₃ was mixed with 120 ml of chloroform, and the mixture was stirred at a temperature of 55 °C for 30 minutes. Then, 4.6 g (21.6 mmol) of Intermediate L7-4 mixed with a small amount of chloroform was slowing added dropwise thereto, and the resulting mixture was stirred at a temperature of 55 °C for 1 hour. Subsequently, 1.2 ml (32.4 mmol) of bromine mixed with 30 ml of chloroform was slowly added dropwise thereto, and the resulting mixture was stirred for 3 hours. After the completion of the reaction, an extraction process was performed thereon using iced water and saturated sodium thiosulfate. An organic layer obtained therefrom was dried with magnesium sulfate, and purified by liquid chromatography, thereby obtaining 2.3 g (yield: 40 %) of Intermediate L7-3.

LC-MS m/z = 292(M+H)⁺

### Synthesis of Intermediate L7-2

1.7 g (yield: 70 %) of Intermediate L7-2 was synthesized in the same manner as in the synthesis of Intermediate L3-2 according to Synthesis Example 2, except that Intermediate L7-3 was used instead of Intermediate L3-3.

LC-MS m/z = 370(M+H)⁺

### Synthesis of Intermediate L7-1

Intermediate L7-1 was synthesized in the same manner as in the synthesis of Intermediate L3-1 according to Synthesis Example 2, except that Intermediate L7-2 was used instead of Intermediate L3-2.

LC-MS m/z = 396(M+H)⁺

### Synthesis of Intermediate L7

0.8 g (yield: 85 %) of Intermediate L7 was synthesized in the same manner as in the synthesis of Intermediate L1 according to Synthesis Example 1, except that Intermediate L7-1 was used instead of Intermediate L1-1. The synthesized compound was identified by LCMS.

LC-MS m/z = 332(M+H)⁺

### Synthesis of Dimer 7

Dimer 7 was synthesized in the same manner as in the synthesis of Dimer 1 according to Synthesis Example 1, except that Intermediate L7 was used instead of Intermediate L1

### Synthesis of Compound 7

0.5 g (yield: 46 %) of Compound 7 was synthesized in the same manner as in the synthesis of Compound 1 according to Synthesis Example 1, except that Dimer 7 and 3,3,7,7-tetramethylnonane-4,6-dione were used instead of Dimer 1 and 3,7-diethyl-3,7-dimethylnonane-4,6-dione, respectively. The synthesized compound was identified by LCMS.

LC-MS m/z = 1065(M+H)⁺

### Synthesis Example 4 (Compound 8)

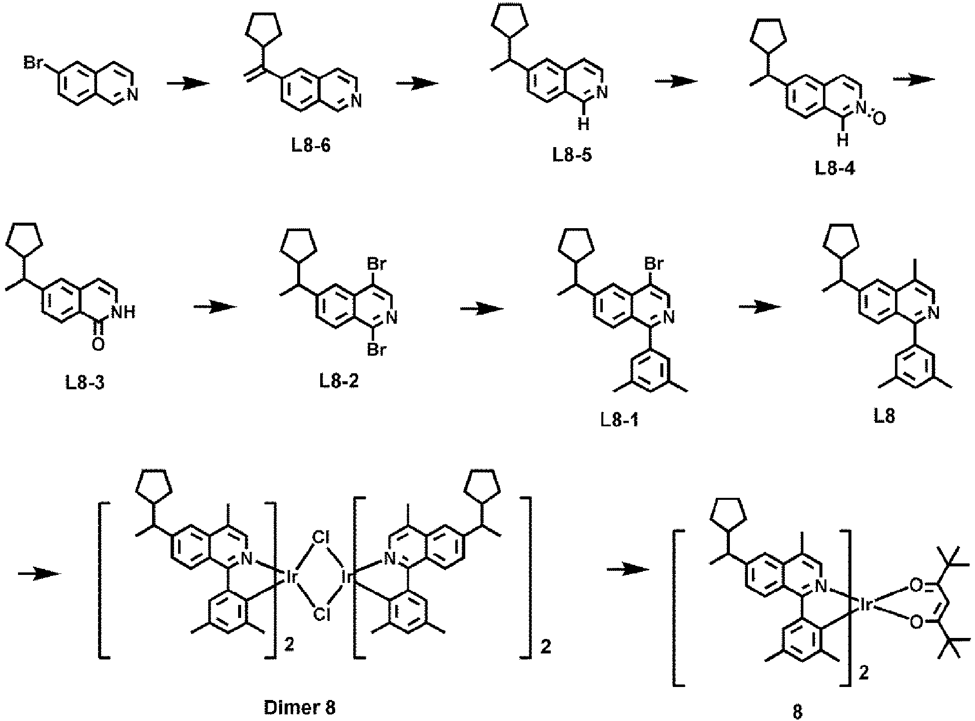

### Synthesis of Intermediate L8-6

Intermediate L8-6 was synthesized in the same manner as in the synthesis of Intermediate L1-6 according to Synthesis Example 1, except that 2-(1-cyclopentylvinyl)-4,4,5,5-tetramethyl-1,3,2-dioxaborolane was used instead of sec-butenylboronic acid.

### Synthesis of Intermediate L8-5

Intermediate L8-5 was synthesized in the same manner as in the synthesis of Intermediate L1-5 according to Synthesis Example 1, except that Intermediate L8-6 was used instead of Intermediate L1-6.

### Synthesis of Intermediate L8-4

Intermediate L8-4 was synthesized in the same manner as in the synthesis of Intermediate L1-4 according to Synthesis Example 1, except that Intermediate L8-5 was used instead of Intermediate L1-5.

### Synthesis of Intermediate L8-3

Intermediate L8-3 was synthesized in the same manner as in the synthesis of Intermediate L1-3 according to Synthesis Example 1, except that Intermediate L8-4 was used instead of Intermediate L1-4.

### Synthesis of Intermediate L8-2

Intermediate L8-2 was synthesized in the same manner as in the synthesis of Intermediate L1-2 according to Synthesis Example 1, except that Intermediate L8-3 was used instead of Intermediate L1-3.

### Synthesis of Intermediate L8-1

Intermediate L8-1 was synthesized in the same manner as in the synthesis of Intermediate L1-1 according to Synthesis Example 1, except that Intermediate L8-2 was used instead of Intermediate L1-2.

### Synthesis of Intermediate L8

Intermediate L8 was synthesized in the same manner as in the synthesis of Intermediate L1 according to Synthesis Example 1, except that Intermediate L8-1 was used instead of Intermediate L1-1.

LC-MS m/z = 344(M+H)⁺

### Synthesis of Dimer 8

Dimer 8 was synthesized in the same manner as in the synthesis of Dimer 1 according to Synthesis Example 1, except that Intermediate L8 was used instead of Intermediate L1.

### Synthesis of Compound 8

1.5 g (0.82 mmol) of Dimer 8, 0.38 g (2.05 mmol) of 2,2,6,6-tetramethylheptane-3,5-dione, and 0.34 g (2.46 mmol) of K₂CO₃ were mixed with 30 ml of ethoxyethanol, and the mixture was stirred for 48 hours. When the reaction proceeded, a solid obtained by filtering the reaction mixture was sufficiently washed with methanol and hexane, and then, was subjected to column chromatography under conditions of dichloromethane:n-hexane=1 :2(v/v), thereby obtaining 0.8 g (yield: 46 %) of Compound 8. The synthesized compound was identified by LCMS.

LC-MS m/z = 1061 (M+H)⁺

### Synthesis Example 5 (Compound 13)

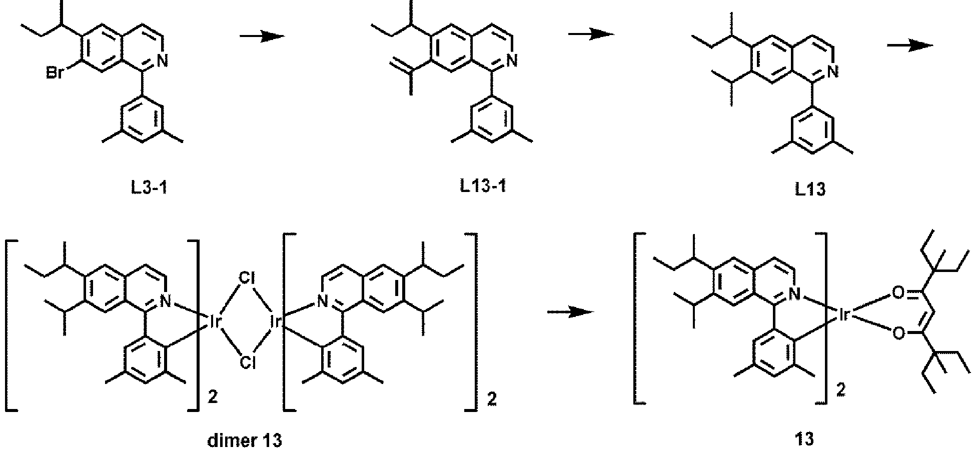

### Synthesis of Intermediate L13-1

1.52 g (4.12 mmol) of Intermediate L3-1 was mixed with 400 ml of THF and 60 ml of water, and 1.0 g (6.2 mmol) of 4,4,5,5-tetramethyl-2-(prop-1-en-2-yl)-1,3,2-dioxaborolane, 0.3 g (0.3 mmol) of Pd(PPh₃)₄, and 1.4 g (10.3 mmol) of K₂CO₃ were added thereto. The mixture was then heated under reflux at a temperature of 80 °C for 18 hours. After the completion of the reaction, the reaction mixture was concentrated under reduced pressure, and an extraction process was performed thereon using 30 ml of ethylacetate. An organic layer obtained therefrom was then dried with magnesium sulfate, and then, subjected to distillation under reduced pressure. The resulting product was purified by liquid chromatography, thereby obtaining 1.1 g (yield: 81 %) of Intermediate L13-1.

LC-MS m/z = 330(M+H)⁺

### Synthesis of Intermediate L13

1.0 g (3.0 mmol) of Intermediate L3-1 was mixed with 50 ml of MeOH, and 19.0 g (30.0 mmol) of ammonium formate and 0.15 g of Pd/C were added thereto. The mixture was then heated under reflux at a temperature of 70 °C for 6 hours. After the completion of the reaction, the reaction mixture was filtered through a celite pad, and then, concentrated under reduced pressure. The resulting product was purified by liquid chromatography, thereby obtaining 0.9 g (yield: 95 %) of Intermediate L13.

LC-MS m/z = 332(M+H)⁺

### Synthesis of Dimer 13

Dimer 13 was synthesized in the same manner as in the synthesis of Dimer 1 according to Synthesis Example 1, except that Intermediate L13 was used instead of Intermediate L1.

### Synthesis of Compound 13

0.6 g (yield: 47 %) of Compound 13 was synthesized in the same manner as in the synthesis of Compound 1 according to Synthesis Example 1, except that Dimer 13 was used instead of Dimer 1.

LC-MS m/z = 1093(M+H)⁺

### Synthesis Example 6(Compound 14)

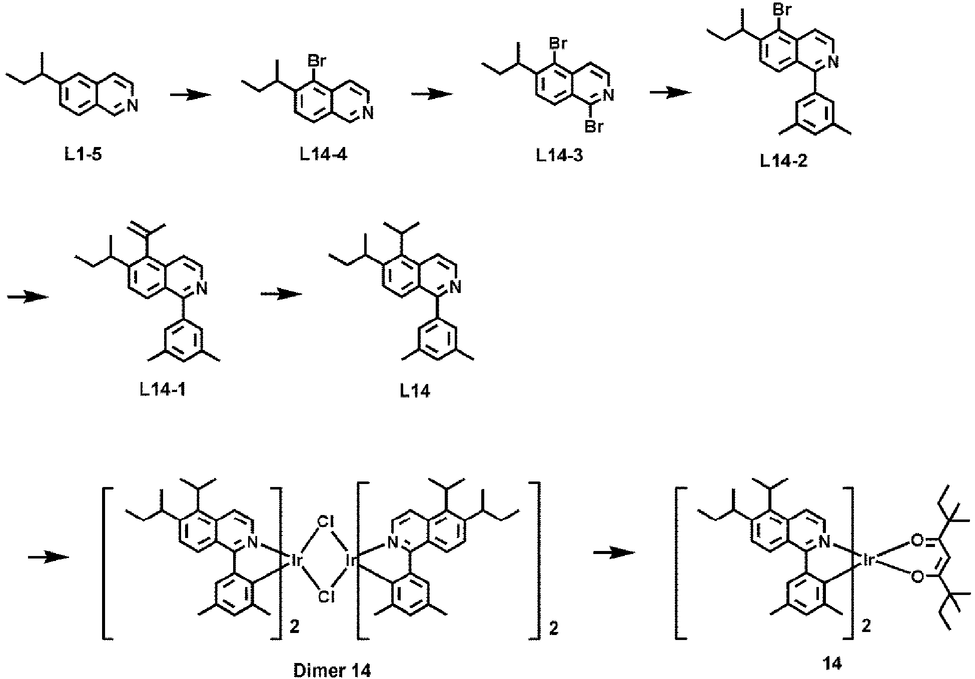

### Synthesis of Intermediate L14-4

Intermediate L14-4 was synthesized in the same manner as in the synthesis of Intermediate L7-3 according to Synthesis Example 3, except that Intermediate L1-5 was used instead of Intermediate L7-4.

### Synthesis of Intermediate L14-3

Intermediate L14-3 was synthesized in the same manner as in the synthesis of Intermediate L7-2 according to Synthesis Example 3, except that Intermediate L14-4 was used instead of Intermediate L7-3.

### Synthesis of Intermediate L14-2

Intermediate L14-2 was synthesized in the same manner as in the synthesis of Intermediate L7-1 according to Synthesis Example 3, except that Intermediate L14-3 was used instead of Intermediate L7-2.

LC-MS m/z = 369(M+H)⁺

### Synthesis of Intermediate L14-1

Intermediate L14-1 was synthesized in the same manner as in the synthesis of Intermediate L13-1 according to Synthesis Example 5, except that Intermediate L14-2 was used instead of Intermediate L3-1.

LC-MS m/z = 330(M+H)⁺

### Synthesis of Intermediate L14

Intermediate L14 was synthesized in the same manner as in the synthesis of Intermediate L13 according to Synthesis Example 5, except that Intermediate L14-1 was used instead of Intermediate L13-1.

LC-MS m/z = 332(M+H)⁺

### Synthesis of Dimer 14

Dimer 14 was synthesized in the same manner as in the synthesis of Dimer 3 according to Synthesis Example 2, except that Intermediate L14 was used instead of Intermediate L3.

### Synthesis of Compound 14

0.9 g (yield: 46 %) of Compound 14 was synthesized in the same manner as in the synthesis of Compound 3 according to Synthesis Example 2, except that Dimer 14 was used instead of Dimer 3.

LC-MS m/z = 1065(M+H)⁺

### Synthesis Example 7 (Compound 15)

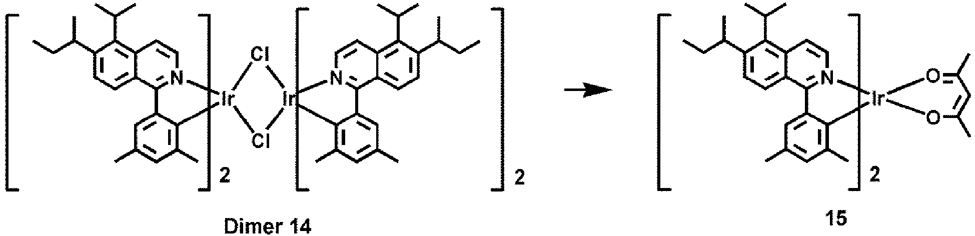

0.7 g (yield: 45 %) of Compound 15 was synthesized in the same manner as in the synthesis of Compound 14 according to Synthesis Example 6, except that pentane-2,4-dione was used instead of 3,3,7,7-tetramethylnonane-4,6-dione.

LC-MS m/z = 953(M+H)⁺

Compounds 17, 18 and 21 can be synthesized by those skilled in the art by referring to the Synthesis Examples 1 to 7 mentioned above and MS spectrum of Compounds 17, 18 and 21 are as follow:
Compound 17 : LC-MS m/z = 981.49(M+H)⁺
Compound 18 : LC-MS m/z = 981.49(M+H)⁺
Compound 21 : LC-MS m/z = 1173.67(M+H)⁺

### Evaluation Example 1: Evaluation of thermal characteristics

Regarding Compounds 1, 3, 7, 8, 13, 14 and A to C, the thermo gravimetric analysis (TGA) and the differential scanning calorimetry (DSC) were performed for thermal analysis (N₂ atmosphere, temperature section: room temperature to 800 °C (10 °C/min)-TGA, room temperature to 400 °C-DSC, Pan Type : Pt Pan in disposable Al Pan (TGA) , disposable Al pan (DSC)), and results thereof are summarized in Table 1.

**[Table 1]**

| Compound No. | Ts_10% (°C) |
|---|---|
| 1 | 192 |
| 3 | 203 |
| 7 | 200 |
| 8 | 213 |
| 13 | 205 |
| 14 | 202 |
| A | 238 |
| B | 235 |
| C | 253 |

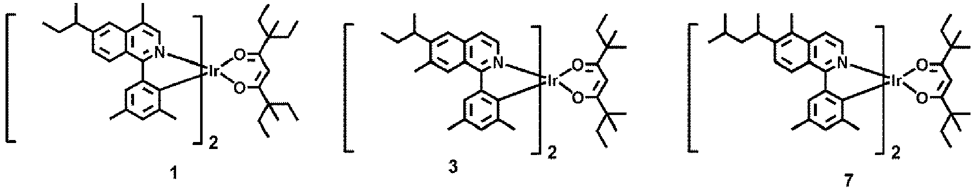

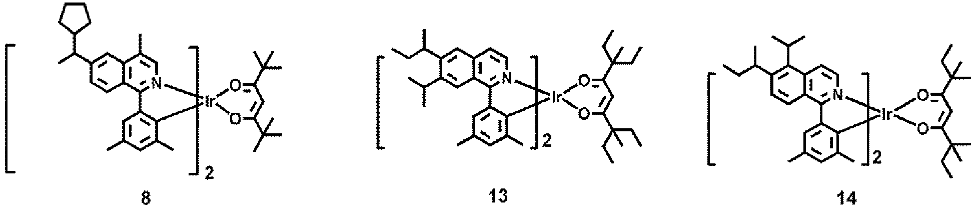

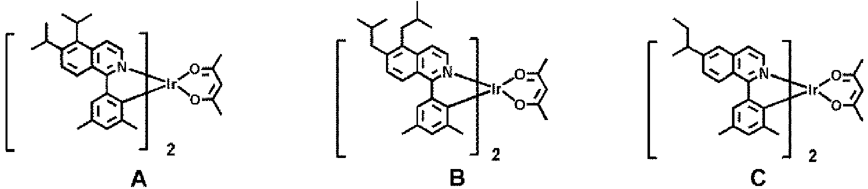

Referring to Table 1, Compounds 1, 3, 7, 8, 13 and 14 were found to have relatively low sublimation temperatures compared to Compounds A to C, and in this regard, a thin film having excellent performance may be prepared by using Compound 1, 3, 7, 8, 13 and 14.

### Evaluation Example 2: Evaluation of horizontal orientation ratio

In a vacuum deposition apparatus having a vacuum level of 1.33 × 10⁻⁵ Pa (1 × 10⁻⁷ Torr), mCP and Compound 1 were co-deposited at a weight ratio of 97 : 3 on a fused silica substrate layer (having a thickness of 1 mm) to form Sample 1 having a thickness of 30 nm (3 wt%). Then, Sample 1 was sealed using glass and glue in a nitrogen atmosphere. The same process was repeated for each of the compounds shown in Table 2 to prepare the remaining samples.

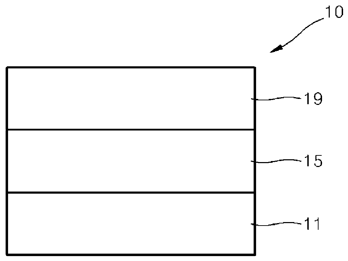

Meanwhile, an angle-dependent PL measuring device having the structure shown in FIG. 3 in KR Application No. 2013-0150834 was prepared. Specifications are as follows:
- Wavelength of excited light: 325 nm
- Source of excited light: He-Cd laser, Melles Griot Company
- Measuring means of excited light : Light fiber, diameter of 1 mm, Thorlabs Company
- Semi-cylindrical prism: Fused silica, diameter of 100 mm, length of 30 mm,
- Detection means of emitted light : Photomultiplier tube, Acton Company
- Polarizer mounted on detection means of emitted light: Linear polarizer, Thorlabs Company
- Recording device: SpectraSense, Acton Company
- Incident angle of excited light: OP = 45°, OH = 0°
- Distance between sample to detection means of emitted light (or, radius of movement path of detection means of emitted light) : 900 mm

Next, each sample was fixed on a semi-cylindrical lens, and then, irradiated with a laser having a wavelength of 325 nm to emit light. The light emitted therefrom passes through a polarizing film, and an intensity of p-polarized light emission was measured for the light having a wavelength of 530 nm by rotating the semi-cylindrical lens fixed with the samples using a charge-coupled device (CCD) at an angle of 1 degree with respect to the axis of the lens.

The intensity of p-polarized light when each compound had a vertical orientation (No.1: Intensity of p-polarized light emission) and the intensity of p-polarized light when each compound had a horizontal orientation (No. 2: Intensity of p-polarized light emission) were each calculated at an angle from 0 degree to 90 degree. Then, weightings that match the p-polarized light, which is obtained by multiplying the intensities of p-polarized light of No. 1 and No. 2 by each weighting thereof were obtained. Then, the horizontal orientation ratios of the compounds of Table 2 were also measured, and results of the measurements are shown in Table 2. Here, the angle-dependent photoluminescence spectra were analyzed using a classical dipole model which regards the emission from the excitons as the dissipated power from the oscillating dipoles.

**[Table 2]**

| Sample No. | Materials for co-deposition | Horizontal orientation ratio (%) |
|---|---|---|
| 1 | mCP : Compound 1 (3 wt%) | 90% |
| 3 | mCP : Compound 3 (3 wt%) | 92% |
| 7 | mCP : Compound 7 (3 wt%) | 93% |
| 8 | mCP : Compound 8 (3 wt%) | 94% |
| 13 | mCP : Compound 13 (3 wt%) | 93% |
| 14 | mCP : Compound 14 (3 wt%) | 92% |
| 15 | mCP : Compound 15 (3 wt%) | 92% |
| A | mCP : Compound A (3 wt%) | 89% |
| B | mCP : Compound B (3 wt%) | 88% |
| C | mCP : Compound C (3 wt%) | 89% |

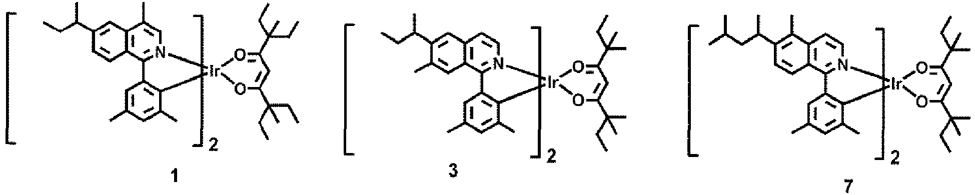

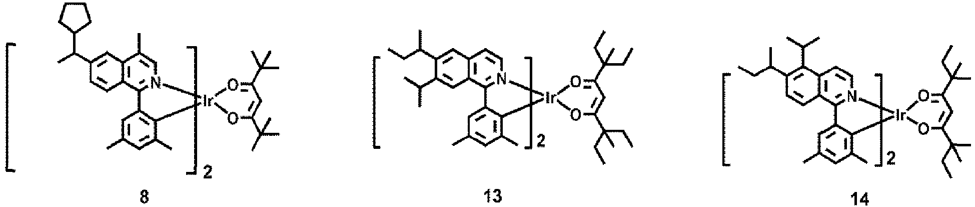

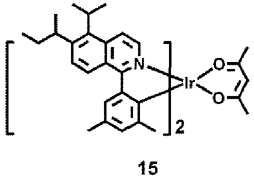

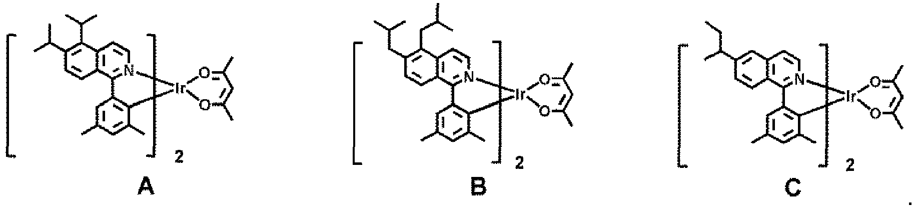

Referring to Table 2, it was confirmed that Compound 1, 3, 7, 8, 13, 14 and 15 had superior horizontal orientation ratios, i.e., excellent directional photoalignment in a horizontal direction, than Compounds A to C.

### Example 1

As an anode, a glass substrate, on which ITO/Ag/ITO were deposited to thicknesses of 70 Å /1,000 Å /70 Å, was cut to a size of 50 mm × 50 mm × 0.5 mm, sonicated with isopropyl alcohol and pure water each for 5 minutes, and then cleaned by exposure to ultraviolet rays and ozone for 30 minutes. Then, the glass substrate was provided to a vacuum deposition apparatus.

2-TNATA was vacuum-deposited on the anode to form a hole injection layer having a thickness of 600 Å, and 4,4'-bis[N-(1-naphthyl)-N-phenylamino]biphenyl (NPB) was vacuum-deposited on the hole injection layer to form a hole transport layer having a thickness of 1,350 Å.

Then, CBP(host) and Compound 1(dopant) were co-deposited in a weight ratio of 97 : 3 on the hole transport layer to form an emission layer having a thickness of 400 Å.

Then, BCP was vacuum deposited on the emission layer to form a hole blocking layer having a thickness of 50 Å, and Alq₃ was vacuum deposited on the hole blocking layer to form an electron transport layer having a thickness of 350 Å. LiF was then vacuum deposited on the electron transport layer to form an electron injection layer having a thickness of 10 Å, and Mg and Ag were co-deposited on a weight ratio of 90:10 on the electron injection layer to form a cathode having a thickness of 120 Å, thereby completing the manufacture of an organic light-emitting device (front red light-emitting device).

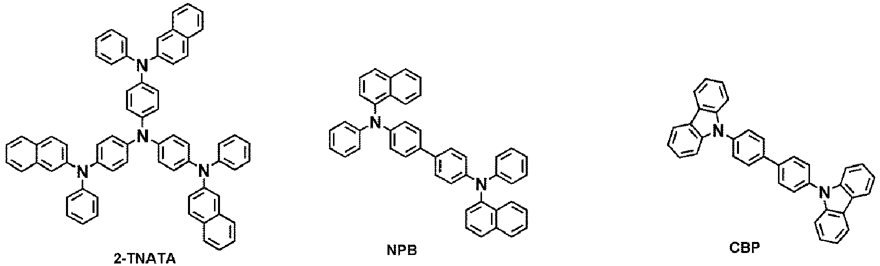

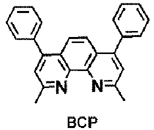

### Examples 2 to 10 and Comparative Examples A to C

Organic light-emitting devices were manufactured in the same manner as in Example 1, except that Compounds shown in Table 3 were each used instead of Compound 1 as a dopant in forming an emission layer.

### Evaluation Example 3: Evaluation of characteristics of organic light-emitting device

The driving voltage, FWHM of an EL spectrum, lifespan (T₉₇) and conversion luminescence efficiency, of the organic light-emitting devices manufactured according to Examples 1 to 10 and Comparative Examples A to C were evaluated, and results thereof are shown in Table 3. A current-voltage meter (Keithley 2400) and a luminance meter (Minolta Cs-1000A) were used as evaluation devices, and the lifespan (LT₉₇) (at 3,500 cd/m² (nit)) indicates an amount of time that lapsed when luminance was 97 % of initial luminance (100 %). Each of lifespan (LT₉₇) and conversion luminescence efficiency were shown in a relative value(%) compared to lifespan (LT₉₇) and conversion luminescence efficiency of Comparative Example A.

**[Table 3]**

| | Dopant in emission layer | Driving voltage (V) | FWHM (nm) | LT₉₇ (relative value compared to Comparative Example A) (%) | Conversion efficiency at CIEx 0.680 |
|---|---|---|---|---|---|
| | | | | | (relative value compared to Comparative Example A) (%) |
| Example 1 | 1 | 4.1 | 50 | 197 | 130 |
| Example 2 | 3 | 4.2 | 52 | 220 | 140 |
| Example 3 | 7 | 4.2 | 45 | 231 | 148 |
| Example 4 | 8 | 4.3 | 58 | 200 | 135 |
| Example 5 | 13 | 4.2 | 57 | 220 | 130 |
| Example 6 | 14 | 4.2 | 50 | 200 | 130 |
| Example 7 | 15 | 4.1 | 60 | 150 | 125 |
| Example 8 | 17 | 4.4 | 60 | 180 | 130 |
| Example 9 | 18 | 4.2 | 58 | 190 | 150 |
| Example 10 | 21 | 4.3 | 58 | 170 | 125 |
| Comparative Example A | A | 4.3 | 70 | 100 | 100 |
| Comparative Example B | B | 4.4 | 68 | 105 | 103 |
| Comparative Example C | C | 4.4 | 70 | 115 | 106 |

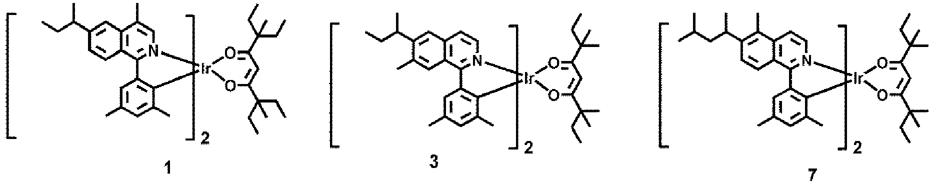

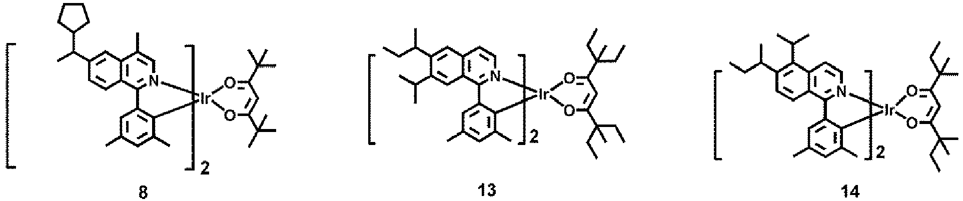

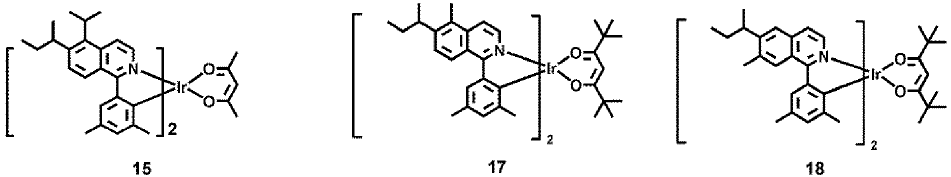

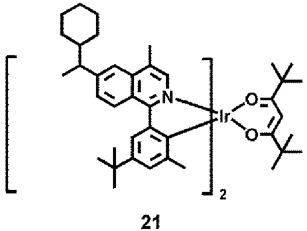

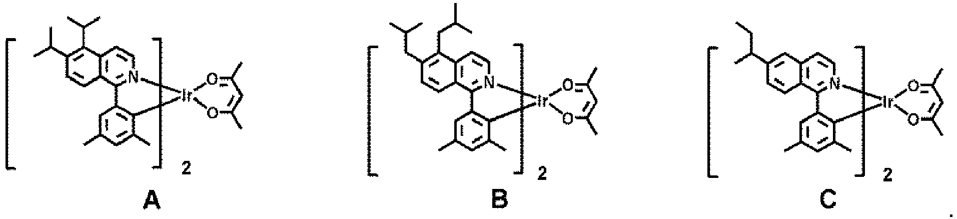

Referring to Table 3, it was confirmed that the FWHM of the light emitted from the organic light-emitting devices of Examples 1 to 10 was smaller than that of the light emitted from the organic light-emitting devices of Comparative Examples A to C, and that the organic light-emitting devices of Examples 1 to 10 had improved driving voltage, improved lifespan, and improved luminescence efficiency, as compared with the organic light-emitting devices of Comparative Examples A to C.

According to the one or more embodiments, an organometallic compound has excellent electric characteristics and thermal stability, and in this regard, an organic light-emitting device including the organometallic compound may have excellent driving voltage, relatively reduced FWHM, excellent lifespan and excellent luminescent efficiency characteristics.

It should be understood that embodiments described herein should be considered in a descriptive sense only and not for purposes of limitation. Descriptions of features or aspects within each embodiment should typically be considered as available for other similar features or aspects in other embodiments.

While one or more embodiments have been described with reference to the figures, it will be understood by those of ordinary skill in the art that various changes in form and details may be made therein without departing from the scope as defined by the following claims.

## Claims

1. An organometallic compound represented by Formula 1: 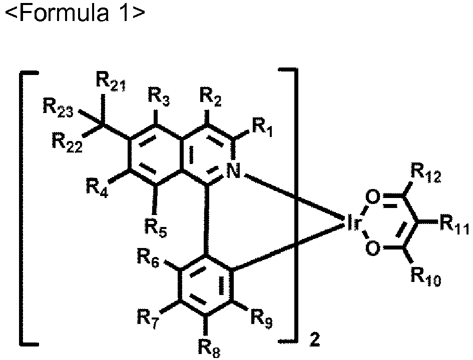
wherein, in Formula 1,
R₁ to R₁₂ and R₂₃ are each independently hydrogen, deuterium, -F, -Cl, -Br, -I, -SF₅, a hydroxyl group, a cyano group, a nitro group, an amino group, an amidino group, a hydrazino group, a hydrazono group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -N(Q₁)(Q₂), -Si(Q₃)(Q₄)(Q₅), -Ge(Q₃)(Q₄)(Q₅), -B(Q₆)(Q₇), -P(=O)(Q₈)(Q₉), or -P(Q₈)(Q₉),
at least one of R₁ to R₅ is each independently deuterium, -F, -Cl, -Br, -I, -SF₅, a hydroxyl group, a cyano group, a nitro group, an amino group, an amidino group, a hydrazino group, a hydrazono group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -N(Q₁)(Q₂), -Si(Q₃)(Q₄)(Q₅), -Ge(Q₃)(Q₄)(Q₅), -B(Q₆)(Q₇), -P(=O)(Q₈)(Q₉), or -P(Q₈)(Q₉),
R₂₁ is a C₂-C₂₀ alkyl group, a deuterium-containing C₂-C₂₀ alkyl group, a C₃-C₁₀ cycloalkyl group, or a deuterium-containing C₃-C₁₀ cycloalkyl group,
R₂₂ is a C₁-C₂₀ alkyl group, a deuterium-containing C₁-C₂₀ alkyl group, a C₃-C₁₀ cycloalkyl group, or a deuterium-containing C₃-C₁₀ cycloalkyl group,
two or more of R₁ to R₉ are optionally linked to each other to form a C₅-C₃₀ carbocyclic group unsubstituted or substituted with at least one R₁ₐ or a C₂-C₃₀ heterocyclic group unsubstituted or substituted with at least one R₁ₐ,
two or more of R₁₀ to R₁₂ are optionally linked to each other to form a C₅-C₃₀ carbocyclic group unsubstituted or substituted with at least one R₁ₐ or a C₂-C₃₀ heterocyclic group unsubstituted or substituted with at least one R₁ₐ,
R₁ₐ is the same as defined in connection with R₁,
a substituent(s) of the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₁-C₆₀ heteroaryl group, the substituted monovalent non-aromatic condensed polycyclic group, and the substituted monovalent non-aromatic condensed heteropolycyclic group is each independently:
deuterium, -F, -Cl, -Br, -I, -CD₃, -CD₂H, -CDH₂, -CF₃, -CF₂H, -CFH₂, a hydroxyl group, a cyano group, a nitro group, an amino group, an amidino group, a hydrazino group, a hydrazono group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, or a C₁-C₆₀ alkoxy group;
a C₁-C₆₀ alkyl group, a C₂-C₆₀ alkenyl group, a C₂-C₆₀ alkynyl group, or a C₁-C₆₀ alkoxy group, each substituted with deuterium, -F, -Cl, -Br, -I, -CD₃, -CD₂H, -CDH₂, -CF₃, - CF₂H, -CFH₂, a hydroxyl group, a cyano group, a nitro group, an amino group, an amidino group, a hydrazino group, a hydrazono group, a carboxylic acid group or a salt thereof, a sulfonic acid group or a salt thereof, a phosphoric acid group or a salt thereof, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₁₁)(Q₁₂), - Si(Q₁₃)(Q₁₄)(Q₁₅), -Ge(Q₁₃)(Q₁₄)(Q₁₅), -B(Q₁₆)(Q₁₇), -P(=O)(Q₁₈)(Q₁₉), -P(Q₁₈)(Q₁₉), or any combination thereof;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, or a monovalent non-aromatic condensed heteropolycyclic group, each unsubstituted or substituted with deuterium, -F, -Cl, -Br, -I, -CD₃, -CD₂H, -CDH₂, -CF₃, - CF₂H, -CFH₂, a hydroxyl group, a cyano group, a nitro group, an amino group, an amidino group, a hydrazino group, a hydrazono group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -N(Q₂₁)(Q₂₂), - Si(Q₂₃)(Q₂₄)(Q₂₅), -Ge(Q₂₃)(Q₂₄)(Q₂₅), -B(Q₂₆)(Q₂₇), -P(=O)(Q₂₈)(Q₂₉), -P(Q₂₈)(Q₂₉), or any combination thereof;
-N(Q₃₁)(Q₃₂), -Si(Q₃₃)(Q₃₄)(Q₃₅), -Ge(Q₃₃)(Q₃₄)(Q₃₅), -B(Q₃₆)(Q₃₇), -P(=O)(Q₃₈)(Q₃₉) or -P(Q₃₈)(Q₃₉); or
any combination thereof; and
Q₁ to Q₉, Q₁₁ to Q19, Q21 to Q29, and Q31 to Q39 are each independently hydrogen; deuterium; -F; -Cl; -Br; -I; a hydroxyl group; a cyano group; a nitro group; an amino group; an amidino group; a hydrazine group; a hydrazone group; a carboxylic acid group or a salt thereof; a sulfonic acid group or a salt thereof; a phosphoric acid group or a salt thereof; a C₁-C₆₀ alkyl group unsubstituted or substituted with deuterium, a C₁-C₆₀ alkyl group, a C₆-C₆₀ aryl group, or any combination thereof; a C₂-C₆₀ alkenyl group; a C₂-C₆₀ alkynyl group; a C₁-C₆₀ alkoxy group; a C₃-C₁₀ cycloalkyl group; a C₁-C₁₀ heterocycloalkyl group; a C₃-C₁₀ cycloalkenyl group; a C₁-C₁₀ heterocycloalkenyl group; a C₆-C₆₀ aryl group unsubstituted or substituted with a C₁-C₆₀ alkyl group, a C₆-C₆₀ aryl group, or any combination thereof; a C₆-C₆₀ aryloxy group; a C₆-C₆₀ arylthio group; a C₁-C₆₀ heteroaryl group; a monovalent non-aromatic condensed polycyclic group; or a monovalent non-aromatic condensed heteropolycyclic group.

2. The organometallic compound of claim 1, wherein
R₁ to R₁₂ and R₂₃ are each independently:
hydrogen, deuterium, -F, a cyano group, or a C₁-C₂₀ alkyl group;
a C₁-C₂₀ alkyl group substituted with deuterium, -F, -CD₃, -CD₂H, -CDH₂, -CF₃, - CF₂H, -CFH₂, a cyano group, a C₁-C₁₀ alkyl group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a phenyl group, a (C₁-C₂₀ alkyl)phenyl group, a biphenyl group, a terphenyl group, or a combination thereof;
a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a phenyl group, a (C₁-C₂₀ alkyl)phenyl group, a biphenyl group, or a terphenyl group, each unsubstituted or substituted with deuterium, -F, -CD₃, -CD₂H, -CDH₂, -CF₃, -CF₂H, -CFH₂, a cyano group, a C₁-C₁₀ alkyl group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a phenyl group, a (C₁-C₂₀ alkyl)phenyl group, a biphenyl group, a terphenyl group, or any combination thereof; or
-Si(Q₃)(Q₄)(Q₅), or -Ge(Q₃)(Q₄)(Q₅); and
each of at least one of R₁ to R₅ is independently:
deuterium, -F, a cyano group, or a C₁-C₂₀ alkyl group;
a C₁-C₂₀ alkyl group substituted with deuterium, -F, -CD₃, -CD₂H, -CDH₂, -CF₃, - CF₂H, -CFH₂, a cyano group, a C₁-C₁₀ alkyl group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a phenyl group, a (C₁-C₂₀ alkyl)phenyl group, a biphenyl group, a terphenyl group, or a combination thereof;
a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a phenyl group, a (C₁-C₂₀ alkyl)phenyl group, a biphenyl group, or a terphenyl group, each unsubstituted or substituted with deuterium, -F, -CD₃, -CD₂H, -CDH₂, -CF₃, -CF₂H, -CFH₂, a cyano group, a C₁-C₁₀ alkyl group, a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, a bicyclo[2.2.2]octyl group, a phenyl group, a (C₁-C₂₀ alkyl)phenyl group, a biphenyl group, a terphenyl group, or any combination thereof; or
-Si(Q₃)(Q₄)(Q₅), or -Ge(Q₃)(Q₄)(Q₅);
wherein, Q₃ to Q₅ are each independently:
-CH₃, -CD₃, -CD₂H, -CDH₂, -CH₂CH₃, -CH₂CD₃, -CH₂CD₂H, -CH₂CDH₂, -CHDCH₃, -CHDCD₂H, -CHDCDH₂, -CHDCD₃, -CD₂CD₃, -CD₂CD₂H, or -CD₂CDH₂; or
an n-propyl group, an isopropyl group, an n-butyl group, a sec-butyl group, an isobutyl group, a tert-butyl group, an n-pentyl group, a tert-pentyl group, a neopentyl group, an isopentyl group, a sec-pentyl group, a 3-pentyl group, a sec-isopentyl group, a phenyl group, a biphenyl group, or a naphthyl group, each unsubstituted or substituted with deuterium, a C₁-C₁₀ alkyl group, a phenyl group, or any combination thereof.

3. The organometallic compound of claims 1 or 2, wherein
R₂₁ is an ethyl group, an n-propyl group, an isopropyl group, an n-butyl group, a sec-butyl group, an isobutyl group, a tert-butyl group, an n-pentyl group, a tert-pentyl group, a neopentyl group, an isopentyl group, a sec-pentyl group, a 3-pentyl group, a sec-isopentyl group, an n-hexyl group, an isohexyl group, a sec-hexyl group, a tert-hexyl group, an n-heptyl group, an isoheptyl group, a sec-heptyl group, a tert-heptyl group, an n-octyl group, an isooctyl group, a sec-octyl group, a tert-octyl group, an n-nonyl group, an isononyl group, a sec-nonyl group, a tert-nonyl group, an n-decyl group, an isodecyl group, a sec-decyl group, a tert-decyl group, a cyclopentyl group, a cyclohexyl group, a cycloheptyl group, a cyclooctyl group, a bicyclo[1.1.1]pentyl group, a bicyclo[2.1.1]hexyl group, a bicyclo[2.2.1]heptyl group, or a bicyclo[2.2.2]octyl group, each unsubstituted or substituted with at least one deuterium, and
R₂₂ is a methyl group, an ethyl group, an n-propyl group, an isopropyl group, an n-butyl group, a sec-butyl group, an isobutyl group, a tert-butyl group, an n-pentyl group, a tert-pentyl group, a neopentyl group, an isopentyl group, a sec-pentyl group, a 3-pentyl group, a sec-isopentyl group, an n-hexyl group, an isohexyl group, a sec-hexyl group, a tert-hexyl group, an n-heptyl group, an isoheptyl group, a sec-heptyl group, a tert-heptyl group, an n-octyl group, an isooctyl group, a sec-octyl group, a tert-octyl group, an n-nonyl group, an isononyl group, a sec-nonyl group, a tert-nonyl group, an n-decyl group, an isodecyl group, a sec-decyl group, a tert-decyl group, a cyclopentyl group, a cyclohexyl group, a cycloheptyl group, a cyclooctyl group, a bicyclo[1.1.1]pentyl group, a bicyclo[2.1.1]hexyl group, a bicyclo[2.2.1]heptyl group, or a bicyclo[2.2.2]octyl group, each unsubstituted or substituted with at least one deuterium.

4. The organometallic compound of any of claims 1-3, wherein
R₆ and R₈ are each independently hydrogen or deuterium, and
R₇ and R₉ are each independently a substituted or unsubstituted C₁-C₆₀ alkyl group, a substituted or unsubstituted C₃-C₁₀ cycloalkyl group, -Si(Q₃)(Q₄)(Q₅), or -Ge(Q₃)(Q₄)(Q₅).

5. The organometallic compound of any of claims 1-4, wherein R₇ and R₉ are each independently a substituted or unsubstituted C₁-C₆₀ alkyl group and R₇ and R₉ are different from each other;
preferably wherein the number of carbon atoms included in R₇ is greater than the number of carbon atoms included in R₉.

6. The organometallic compound of any of claims 1-5, wherein R₂₁ is a C₃-C₁₀ cycloalkyl group, or a deuterium-containing C₃-C₁₀ cycloalkyl group; and/or
wherein R₂₂ is a methyl group.

7. The organometallic compound of any of claims 1-6, wherein R₂₂ is a C₂-C₂₀ alkyl group, a deuterium-containing C₂-C₂₀ alkyl group, a C₃-C₁₀ cycloalkyl group, or a deuterium-containing C₃-C₁₀ cycloalkyl group; and/or
wherein R₂₃ is hydrogen, or deuterium.

8. The organometallic compound of any of claims 1-7, wherein
the organometallic compound satisfies <Condition 3>, <Condition 4>, or combination thereof:
<Condition 3>
R₁₀ in Formula 1 is represented by Formula 3.
<Condition 4>
R₁₂ in Formula 1 is represented by Formula 4. 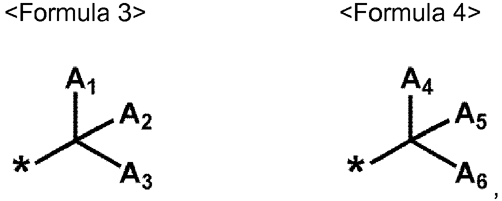
wherein, in Formulae 3 and 4,
A₁ to A₆ are each independently:
hydrogen, or deuterium, or
a C₁-C₂₀ alkyl group, a C₃-C₁₀ cycloalkyl group, a C₆-C₆₀ aryl group, or a C₁-C₆₀ heteroaryl group, each unsubstituted or substituted with deuterium, a C₁-C₂₀ alkyl group, a C₃-C₁₀ cycloalkyl group, or any combination thereof, and
* indicates a binding site to a neighboring atom;
preferably wherein
Formula 3 satisfies one of <Condition 3-1> to <Condition 3-5> and
Formula 4 satisfies one of <Condition 4-1> to <Condition 4-5>:
<Condition 3-1>
A₁ to A₃ are not hydrogen, simultaneously.
<Condition 3-2>
A₁ to A₃ are each independently a C₁-C₂₀ alkyl group, or a C₃-C₁₀ cycloalkyl group, each unsubstituted or substituted with deuterium, a C₁-C₂₀ alkyl group, a C₃-C₁₀ cycloalkyl group, or any combination thereof.
<Condition 3-3>
A₁ and A₂ are each independently a C₁-C₂₀ alkyl group, or a C₃-C₁₀ cycloalkyl group, each unsubstituted or substituted with deuterium, a C₁-C₂₀ alkyl group, a C₃-C₁₀ cycloalkyl group, or any combination thereof, and
A₃ is a C₂-C₂₀ alkyl group, or a C₃-C₁₀ cycloalkyl group, each unsubstituted or substituted with deuterium, a C₁-C₂₀ alkyl group, a C₃-C₁₀ cycloalkyl group, or any combination thereof.
<Condition 3-4>
A₁ is a C₁-C₂₀ alkyl group, or a C₃-C₁₀ cycloalkyl group, each unsubstituted or substituted with deuterium, a C₁-C₂₀ alkyl group, a C₃-C₁₀ cycloalkyl group, or any combination thereof, and
A₂ and A₃ are each independently a C₂-C₂₀ alkyl group, or a C₃-C₁₀ cycloalkyl group, each unsubstituted or substituted with deuterium, a C₁-C₂₀ alkyl group, a C₃-C₁₀ cycloalkyl group, or any combination thereof.
<Condition 3-5>
A₁ is hydrogen, or deuterium,
A₂ is a C₁-C₂₀ alkyl group, or a C₃-C₁₀ cycloalkyl group, each unsubstituted or substituted with deuterium, a C₁-C₂₀ alkyl group, a C₃-C₁₀ cycloalkyl group, or any combination thereof, and
A₃ is a C₂-C₂₀ alkyl group, or a C₃-C₁₀ cycloalkyl group, each unsubstituted or substituted with deuterium, a C₁-C₂₀ alkyl group, a C₃-C₁₀ cycloalkyl group, or any combination thereof.
<Condition 4-1>
A₄ to A₆ are not hydrogen, simultaneously.
<Condition 4-2>
A₄ to A₆ are each independently a C₁-C₂₀ alkyl group, or a C₃-C₁₀ cycloalkyl group, each unsubstituted or substituted with deuterium, a C₁-C₂₀ alkyl group, a C₃-C₁₀ cycloalkyl group, or any combination thereof.
<Condition 4-3>
A₄ and A₅ are each independently a C₁-C₂₀ alkyl group, or a C₃-C₁₀ cycloalkyl group, each unsubstituted or substituted with deuterium, a C₁-C₂₀ alkyl group, a C₃-C₁₀ cycloalkyl group, or any combination thereof, and
A₆ is a C₂-C₂₀ alkyl group, or a C₃-C₁₀ cycloalkyl group, each unsubstituted or substituted with deuterium, a C₁-C₂₀ alkyl group, a C₃-C₁₀ cycloalkyl group, or any combination thereof.
<Condition 4-4>
A₄ is a C₁-C₂₀ alkyl group, or a C₃-C₁₀ cycloalkyl group, each unsubstituted or substituted with deuterium, a C₁-C₂₀ alkyl group, a C₃-C₁₀ cycloalkyl group, or any combination thereof, and
A₅ and A₆ are each independently a C₂-C₂₀ alkyl group, or a C₃-C₁₀ cycloalkyl group, each unsubstituted or substituted with deuterium, a C₁-C₂₀ alkyl group, a C₃-C₁₀ cycloalkyl group, or any combination thereof.
<Condition 4-5>
A₄ is hydrogen, or deuterium,
A₅ is a C₁-C₂₀ alkyl group, or a C₃-C₁₀ cycloalkyl group, each unsubstituted or substituted with deuterium, a C₁-C₂₀ alkyl group, a C₃-C₁₀ cycloalkyl group, or any combination thereof, and
A₆ is a C₂-C₂₀ alkyl group, or a C₃-C₁₀ cycloalkyl group, each unsubstituted or substituted with deuterium, a C₁-C₂₀ alkyl group, a C₃-C₁₀ cycloalkyl group, or any combination thereof.

9. The organometallic compound of any of claims 1-8, wherein
at least one of R₁₀ and R₁₂ are each independently a cyclopentyl group, a cyclohexyl group, a cycloheptyl group, a cyclooctyl group, an adamantanyl group, a norbornanyl group, a norbornenyl group, a cyclopentenyl group, a cyclohexenyl group, a cycloheptenyl group, a bicyclo[1.1.1]pentyl group, a bicyclo[2.1.1]hexyl group, a bicyclo[2.2.1]heptyl group, or a bicyclo[2.2.2]octyl group, each unsubstituted or substituted with deuterium, -F, -CD₃, -CD₂H, -CDH₂, -CF₃, -CF₂H, -CFH₂, a cyano group, a C₁-C₁₀ alkyl group, or any combination thereof.

10. The organometallic compound of claim 1, wherein
the organometallic compound is one of Compounds 1 to 22:

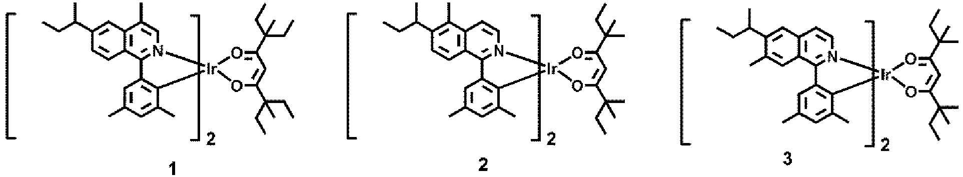

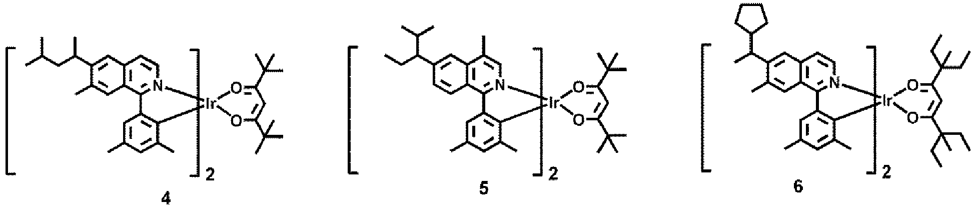

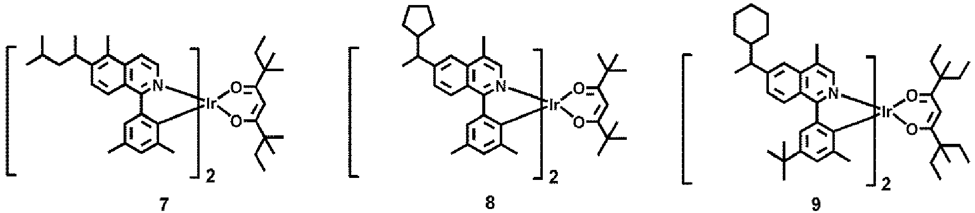

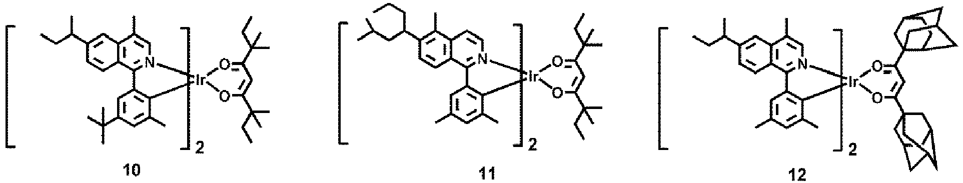

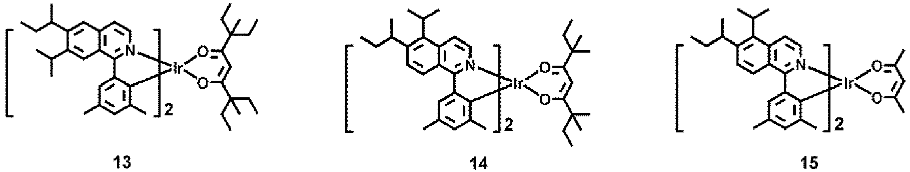

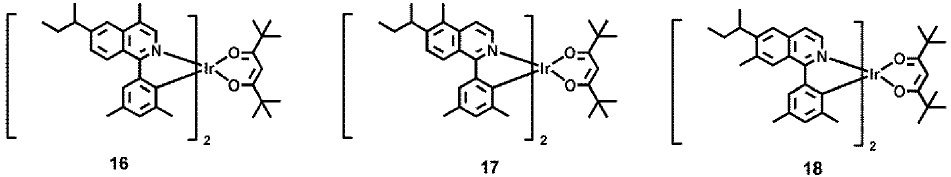

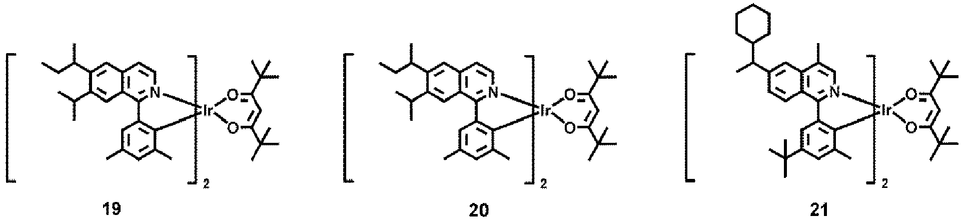

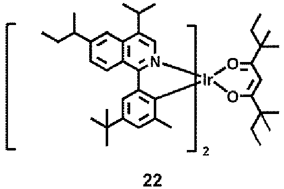

11. An organic light-emitting device comprising:
a first electrode;
a second electrode; and
an organic layer disposed between the first electrode and the second electrode and comprising an emission layer,
wherein the organic layer comprises at least one organometallic compound of any of claims 1-10.

12. The organic light-emitting device of claim 11, wherein
the first electrode is an anode,
the second electrode is a cathode,
the organic layer further comprises a hole transport region disposed between the first electrode and the emission layer and an electron transport region disposed between the emission layer and the second electrode,
the hole transport region comprises a hole injection layer, a hole transport layer, an electron blocking layer, a buffer layer, or any combination thereof, and
the electron transport region comprises a hole blocking layer, an electron transport layer, an electron injection layer, or any combination thereof.

13. The organic light-emitting device of claims 11 or 12, wherein
the emission layer comprises the organometallic compound.

14. The organic light-emitting device of claim 13, wherein the emission layer emits red light; and/or
wherein the emission layer further comprises a host, and an amount of the host in the emission layer is larger than an amount of the organometallic compound in the emission layer.

15. A diagnostic composition comprising at least one organometallic compound of any of claims 1-10.

## Patentansprüche

1. Metallorganische Verbindung, dargestellt durch Formel 1: 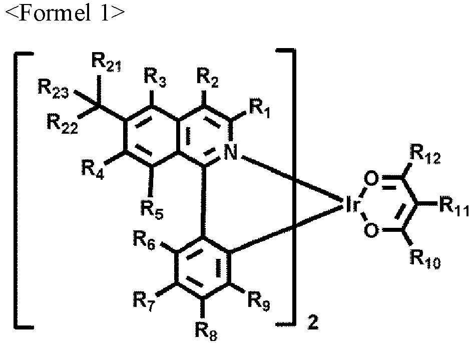
wobei in Formel 1
R₁ bis R₁₂ und R₂₃ jeweils unabhängig Wasserstoff, Deuterium, -F, -Cl, -Br, -I, -SF₅, eine Hydroxylgruppe, eine Cyanogruppe, eine Nitrogruppe, eine Aminogruppe, eine Amidinogruppe, eine Hydrazinogruppe, eine Hydrazonogruppe, eine Carbonsäuregruppe oder ein Salz davon, eine Sulfonsäuregruppe oder ein Salz davon, eine Phosphorsäuregruppe oder ein Salz davon, eine substituierte oder unsubstituierte C₁-C₆₀-Alkylgruppe, eine substituierte oder unsubstituierte C₂-C₆₀-Alkenylgruppe, eine substituierte oder unsubstituierte C₂-C₆₀-Alkynylgruppe, eine substituierte oder unsubstituierte C₁-C₆₀-Alkoxygruppe, eine substituierte oder unsubstituierte C₃-C₁₀-Cycloalkylgruppe, eine substituierte oder unsubstituierte C₁-C₁₀-Heterocycloalkylgruppe, eine substituierte oder unsubstituierte C₃-C₁₀-Cycloalkenylgruppe, eine substituierte oder unsubstituierte C₁-C₁₀-Heterocycloalkenylgruppe, eine substituierte oder unsubstituierte C₆-C₆₀-Arylgruppe, eine substituierte oder unsubstituierte C₆-C₆₀-Aryloxygruppe, eine substituierte oder unsubstituierte C₆-C₆₀-Arylthiogruppe, eine substituierte oder unsubstituierte C₁-C₆₀-Heteroarylgruppe, eine substituierte oder unsubstituierte monovalente nichtaromatische kondensierte polycyclische Gruppe, eine substituierte oder unsubstituierte monovalente nichtaromatische kondensierte heteropolycyclische Gruppe, -N(Q₁)(Q₂), -Si(Q₃)(Q₄)(Q₅), - Ge(Q₃)(Q₄)(Q₅), -B(Q₆)(Q₇), -P(=O)(Q₈)(Q₉) oder -P(Q₈)(Q₉) sind,
mindestens eines von R₁ bis R₅ jeweils unabhängig Deuterium, -F, -Cl, -Br, -I, -SF₅, eine Hydroxylgruppe, eine Cyanogruppe, eine Nitrogruppe, eine Aminogruppe, eine Amidinogruppe, eine Hydrazinogruppe, eine Hydrazonogruppe, eine Carbonsäuregruppe oder ein Salz davon, eine Sulfonsäuregruppe oder ein Salz davon, eine Phosphorsäuregruppe oder ein Salz davon, eine substituierte oder unsubstituierte C₁-C₆₀-Alkylgruppe, eine substituierte oder unsubstituierte C₂-C₆₀-Alkenylgruppe, eine substituierte oder unsubstituierte C₂-C₆₀-Alkynylgruppe, eine substituierte oder unsubstituierte C₁-C₆₀-Alkoxygruppe, eine substituierte oder unsubstituierte C₃-C₁₀-Cycloalkylgruppe, eine substituierte oder unsubstituierte C₁-C₁₀-Heterocycloalkylgruppe, eine substituierte oder unsubstituierte C₃-C₁₀-Cycloalkenylgruppe, eine substituierte oder unsubstituierte C₁-C₁₀-Heterocycloalkenylgruppe, eine substituierte oder unsubstituierte C₆-C₆₀-Arylgruppe, eine substituierte oder unsubstituierte C₆-C₆₀-Aryloxygruppe, eine substituierte oder unsubstituierte C₆-C₆₀-Arylthiogruppe, eine substituierte oder unsubstituierte C₁-C₆₀-Heteroarylgruppe, eine substituierte oder unsubstituierte monovalente nichtaromatische kondensierte polycyclische Gruppe, eine substituierte oder unsubstituierte monovalente nichtaromatische kondensierte heteropolycyclische Gruppe, -N(Q₁)(Q₂), -Si(Q₃)(Q₄)(Q₅), - Ge(Q₃)(Q₄)(Q₅), -B(Q₆)(Q₇), -P(=O)(Q₈)(Q₉) oder -P(Q₈)(Q₉) sind,
R₂₁ eine C₂-C₂₀-Alkylgruppe, eine Deuterium-haltige C₂-C₂₀-Alkylgruppe, eine C₃-C₁₀-Cycloalkylgruppe oder eine Deuterium-haltige C₃-C₁₀-Cycloalkylgruppe ist,
R₂₂ eine C₁-C₂₀-Alkylgruppe, eine Deuterium-haltige C₁-C₂₀-Alkylgruppe, eine C₃-C₁₀-Cycloalkylgruppe oder eine Deuterium-haltige C₃-C₁₀-Cycloalkylgruppe ist,
zwei oder mehrere von R₁ bis R₉ optional miteinander verbunden sind, um eine carbocyclische C₅-C₃₀-Gruppe, die unsubstituiert oder mit mindestens einem R₁ₐ substituiert ist, oder eine heterocyclische C₂-C₃₀-Gruppe, die unsubstituiert oder mit mindestens einem R₁ₐ substituiert ist, zu bilden,
zwei oder mehrere von R₁₀ bis R₁₂ optional miteinander verbunden sind, um eine carbocyclische C₅-C₃₀-Gruppe, die unsubstituiert oder mit mindestens einem R₁ₐ substituiert ist, oder eine heterocyclische C₂-C₃₀ Gruppe, die unsubstituiert oder mit mindestens einem R₁ₐ substituiert ist, zu bilden,
R₁ₐ gleich wie definiert in Verbindung mit R₁ ist,
ein Substituent(en) der substituierten C₁-C₆₀-Alkylgruppe, der substituierten C₂-C₆₀-Alkenylgruppe, der substituierten C₂-C₆₀-Alkinylgruppe, der substituierten C₁-C₆₀-Alkoxygruppe, der substituierten C₃-C₁₀-Cycloalkylgruppe, der substituierten C₁-C₁₀-Heterocycloalkylgruppe, der substituierten C₃-C₁₀-Cycloalkenylgruppe, der substituierten C₁-C₁₀-Heterocycloalkenylgruppe, der substituierten C₆-C₆₀-Arylgruppe, der substituierten C₆-C₆₀-Aryloxygruppe, der substituierten C₆-C₆₀-Arylthiogruppe, der substituierten C₁-C₆₀-Heteroarylgruppe, der substituierten monovalenten nicht-aromatischen kondensierten polycyclischen Gruppe, und der substituierten monovalenten nicht-aromatischen kondensierten heteropolycyclischen Gruppe, jeweils unabhängig wie folgt ist:
Deuterium, -F, -Cl, -Br, -I, -CD₃, -CD₂H, -CDH₂, -CF₃, -CF₂H, -CFH₂, eine Hydroxylgruppe, eine Cyanogruppe, eine Nitrogruppe, eine Aminogruppe, eine Amidinogruppe, eine Hydrazingruppe, eine Hydrazongruppe, eine Carbonsäuregruppe oder ein Salz davon, eine Sulfonsäuregruppe oder ein Salz davon, eine Phosphorsäuregruppe oder ein Salz davon, eine C₁-C₆₀-Alkylgruppe, eine C₂-C₆₀-Alkenylgruppe, eine C₂-C₆₀-Alkinylgruppe oder eine C₁-C₆₀-Alkoxygruppe;
eine C₁-C₆₀-Alkylgruppe, eine C₂-C₆₀-Alkenylgruppe, eine C₂-C₆₀-Alkinylgruppe oder eine C₁-C₆₀-Alkoxygruppe, jeweils substituiert mit Deuterium, -F, -Cl, -Br, -I, -CD₃, -CD₂H, - CDH₂, -CF₃, - CF₂H, -CFH₂, eine Hydroxylgruppe, eine Cyanogruppe, eine Nitrogruppe, eine Aminogruppe, Amidinogruppe, eine Hydrazingruppe, eine Hydrazongruppe, eine Carbonsäuregruppe oder ein Salz davon, eine Sulfonsäuregruppe oder ein Salz davon, eine Phosphorsäuregruppe oder ein Salz davon, eine C₃-C₁₀-Cycloalkylgruppe, eine C₁-C₁₀-Heterocycloalkylgruppe, eine C₃-C₁₀-Cycloalkenylgruppe, eine C₁-C₁₀-Heterocycloalkenylgruppe, eine C₆-C₆₀-Arylgruppe, eine C₆-C₆₀-Aryloxygruppe, eine C₆-C₆₀-Arylthiogruppe, eine C₁-C₆₀-Heteroarylgruppe, eine einwertige nicht-aromatische kondensierte polycyclische Gruppe, eine einwertige nicht-aromatische kondensierte heteropolycyclische Gruppe, -N(Qn)(Q₁₂), -Si(Q₁₃)(Q₁₄)(Q₁₅), -Ge(Q₁₃)(Q₁₄)(Q₁₅), -B(Q₁₆)(Q₁₇), -P(=O)(Q₁₈)(Q₁₉), - P(Q₁₈)(Q₁₉) oder eine beliebige Kombination davon;
eine C₃-C₁₀-Cycloalkylgruppe, eine C₁-C₁₀-Heterocycloalkylgruppe, eine C₃-C₁₀-Cycloalkenylgruppe, eine C₁-C₁₀-Heterocycloalkenylgruppe, eine C₆-C₆₀-Arylgruppe, eine C₆-C₆₀-Aryloxygruppe, eine C₆-C₆₀-Arylthiogruppe, eine C₁-C₆₀-Heteroarylgruppe, eine monovalenten nichtaromatischen kondensierten polycyclischen Gruppe oder eine monovalenten nichtaromatischen kondensierten heteropolycyclischen Gruppe, jeweils unsubstituiert oder substituiert mit Deuterium, -F, -Cl, -Br, -I, -CD₃, -CD₂H, -CDH₂, -CF₃, -CF₂H, -CFH₂, eine Hydroxylgruppe, eine Cyanogruppe, eine Nitrogruppe, eine Aminogruppe, eine Amidinogruppe, eine Hydrazingruppe, eine Hydrazongruppe, eine Carbonsäuregruppe oder einem Salz davon, eine Sulfonsäuregruppe oder einem Salz davon, eine Phosphorsäuregruppe oder einem Salz davon, eine C₁-C₆₀-Alkylgruppe, eine C₂-C₆₀-Alkenylgruppe, eine C₂-C₆₀-Alkynylgruppe, eine C₁-C₆₀-Alkoxygruppe, eine C₃-C₁₀-Cycloalkylgruppe, eine C₁-C₁₀-Heterocycloalkylgruppe, eine C₃-C₁₀-Cycloalkenylgruppe, eine C₁-C₁₀-Heterocycloalkenylgruppe, eine C₆-C₆₀-Arylgruppe, eine C₆-C₆₀-Aryloxygruppe, eine C₆-C₆₀-Arylthiogruppe, eine C₁-C₆₀-Heteroarylgruppe, eine monovalenten nichtaromatischen kondensierten polycyclischen Gruppe, eine monovalenten nichtaromatischen kondensierten heteropolycyclischen Gruppe, -N(Q₂₁)(Q₂₂), -Si(Q₂₃)(Q₂₄)(Q₂₅), -Ge(Q₂₃)(Q₂₄)(Q₂₅), -B(Q₂₆)(Q₂₇), -P(=O)(Q₂₈)(Q₂₉), -P(Q₂₈)(Q₂₉) oder eine beliebige Kombination davon;
-N(Q₃₁)(Q₃₂), -Si(Q₃₃)(Q₃₄)(Q₃₅), -Ge(Q₃₃)(Q₃₄)(Q₃₅), -B(Q₃₆)(Q₃₇), -P(=O)(Q₃₈)(Q₃₉) oder -P(Q₃₈)(Q₃₉); oder
eine beliebige Kombination davon; und
Q₁ bis Q₉, Q₁₁ bis Q₁₉, Q₂₁ bis Q₂₉ und Q₃₁ bis Q₃₉ sind jeweils unabhängig Wasserstoff; Deuterium; -F; -Cl; -Br; -I; eine Hydroxylgruppe; eine Cyanogruppe; eine Nitrogruppe; eine Aminogruppe; eine Amidinogruppe; eine Hydrazingruppe; eine Hydrazongruppe; eine Carbonsäuregruppe oder ein Salz davon; eine Sulfonsäuregruppe oder ein Salz davon; eine Phosphorsäuregruppe oder ein Salz davon; eine C₁-C₆₀-Alkylgruppe, die unsubstituiert oder mit Deuterium, einer C₁-C₆₀-Alkylgruppe, einer C₆-C₆₀-Arylgruppe oder einer beliebigen Kombination davon substituiert ist; eine C₂-C₆₀-Alkenylgruppe, eine C₂-C₆₀-Alkinylgruppe, eine C₁-C₆₀-Alkoxygruppe; eine C₃-C₁₀-Cycloalkylgruppe; eine C₁-C₁₀-Heterocycloalkylgruppe; eine C₃-C₁₀-Cycloalkenylgruppe, eine C₁-C₁₀-Heterocycloalkenylgruppe; eine C₆-C₆₀-Arylgruppe, die unsubstituiert oder mit einer C₁-C₆₀-Alkylgruppe, einer C₆-C₆₀-Arylgruppe oder einer beliebigen Kombination davon substituiert ist; eine C₆-C₆₀-Aryloxygruppe, eine C₆-C₆₀-Arylthiogruppe, eine C₁-C₆₀-Heteroarylgruppe; eine einwertige nicht-aromatische kondensierte polycyclische Gruppe; oder eine einwertige nicht-aromatische kondensierte heteropolycyclische Gruppe sind.

2. Metallorganische Verbindung nach Anspruch 1, wobei
R₁ bis R₁₂ und R₂₃ jeweils unabhängig wie folgt sind:
Wasserstoff, Deuterium, -F, eine Cyanogruppe oder eine C₁-C₂₀-Alkylgruppe,
eine C₁-C₂₀-Alkylgruppe, substituiert mit Deuterium, -F, -CD₃, -CD₂H, -CDH₂, -CF₃, - CF₂H, -CFH₂, eine Cyanogruppe, eine C₁-C₁₀-Alkylgruppe, eine Cyclopentylgruppe, einer Cyclohexylgruppe, eine Cycloheptylgruppe, eine Cyclooctylgruppe, eine Adamantanylgruppe, eine Norbomanylgruppe, eine Norbornenylgruppe, eine Cyclopentenylgruppe, eine Cyclohexenylgruppe, eine Cycloheptenylgruppe, eine Bicyclo[1.1.1]pentylgruppe, eine Bicyclo[2.1.1]hexylgruppe, eine Bicyclo[2.2.1]heptylgruppe, eine Bicyclo[2.2.2]octylgruppe, eine Phenylgruppe, eine (C₁-C₂₀-Alkyl)phenylgruppe, eine Biphenylgruppe, eine Terphenylgruppe oder eine Kombination davon;
eine Cyclopentylgruppe, einer Cyclohexylgruppe, eine Cycloheptylgruppe, eine Cyclooctylgruppe, eine Adamantanylgruppe, eine Norbomanylgruppe, eine Norbornenylgruppe, eine Cyclopentenylgruppe, eine Cyclohexenylgruppe, eine Cycloheptenylgruppe, eine Bicyclo[1.1.1]pentylgruppe, eine Bicyclo[2.1.1]hexylgruppe, eine Bicyclo[2.2.1]heptylgruppe, eine Bicyclo[2.2.2]octylgruppe, eine Phenylgruppe, eine (C₁-C₂₀-Alkyl)phenylgruppe, eine Biphenylgruppe, eine Terphenylgruppe, jeweils unsubstituiert oder substituiert mit Deuterium, - F, -CD₃, -CD₂H, -CDH₂, -CF₃, -CF₂H, -CFH₂, eine Cyanogruppe, eine C₁-C₁₀-Alkylgruppe, eine Cyclopentylgruppe, eine Cyclohexylgruppe, eine Cycloheptylgruppe, eine Cyclooctylgruppe, eine Adamantanylgruppe, eine Norbomanylgruppe, eine Norbornenylgruppe, eine Cyclopentenylgruppe, eine Cyclohexenylgruppe, eine Cycloheptenylgruppe, eine Bicyclo[1.1.1]Pentylgruppe, eine Bicyclo[2.1.1]hexylgruppe, eine Bicyclo[2.2.1]heptylgruppe, eine Bicyclo[2.2.2]octylgruppe, eine Phenylgruppe, eine (C₁-C₂₀-Alkyl)phenylgruppe, eine Biphenylgruppe, eine Terphenylgruppe oder eine beliebige Kombination davon; oder - Si(Q₃)(Q₄)(Q₅) oder -Ge(Q₃)(Q₄)(Q₅); und
mindestens eines von R₁ bis R₅ ist jeweils unabhängig:
Deuterium, -F, eine Cyanogruppe oder eine C₁-C₂₀-Alkylgruppe,
eine C₁-C₂₀-Alkylgruppe, substituiert mit Deuterium, -F, -CD₃, -CD₂H, -CDH₂, -CF₃, - CF₂H, -CFH₂, eine Cyanogruppe, eine C₁-C₁₀-Alkylgruppe, eine Cyclopentylgruppe, einer Cyclohexylgruppe, eine Cycloheptylgruppe, eine Cyclooctylgruppe, eine Adamantanylgruppe, eine Norbomanylgruppe, eine Norbornenylgruppe, eine Cyclopentenylgruppe, eine Cyclohexenylgruppe, eine Cycloheptenylgruppe, eine Bicyclo[1.1.1]pentylgruppe, eine Bicyclo[2.1.1]hexylgruppe, eine Bicyclo[2.2.1]heptylgruppe, eine Bicyclo[2.2.2]octylgruppe, eine Phenylgruppe, eine (C₁-C₂₀-Alkyl)phenylgruppe, eine Biphenylgruppe, eine Terphenylgruppe oder eine Kombination davon;
eine Cyclopentylgruppe, eine Cyclohexylgruppe, eine Cycloheptylgruppe, eine Cyclooctylgruppe, eine Adamantanylgruppe, eine Norbomanylgruppe, eine Norbornenylgruppe, eine Cyclopentenylgruppe, eine Cyclohexenylgruppe, eine Cycloheptenylgruppe, eine Bicyclo[1.1.1]pentylgruppe, eine Bicyclo[2.1.1]hexylgruppe, eine Bicyclo[2.2.1]heptylgruppe, eine Bicyclo[2.2.2]octylgruppe, eine Phenylgruppe, eine (C₁-C₂₀-Alkyl)Phenylgruppe, eine Biphenylgruppe oder eine Terphenylgruppe, jeweils unsubstituiert oder substituiert mit Deuterium, -F, -CD₃, -CD₂H, -CDH₂, -CF₃, -CF₂H, -CFH₂, eine Cyanogruppe, eine C₁-C₁₀-Alkylgruppe, eine Cyclopentylgruppe, einer Cyclohexylgruppe, eine Cycloheptylgruppe, eine Cyclooctylgruppe, eine Adamantanylgruppe, eine Norbomanylgruppe, eine Norbornenylgruppe, eine Cyclopentenylgruppe, eine Cyclohexenylgruppe, eine Cycloheptenylgruppe, eine Bicyclo[1.1.1]pentylgruppe, eine Bicyclo[2.1.1]hexylgruppe, eine Bicyclo[2.2.1]heptylgruppe, eine Bicyclo[2.2.2]octylgruppe, eine Phenylgruppe, eine (C₁-C₂₀-Alkyl)phenylgruppe, eine Biphenylgruppe, eine Terphenylgruppe oder eine beliebige Kombination davon; oder
-Si(Q₃)(Q₄)(Q₅) oder -Ge(Q₃)(Q₄)(Q₅);
wobei Q₃ bis Q₅ jeweils unabhängig wie folgt sind:
-CH₃, -CD₃, -CD₂H, -CDH₂, -CH₂CH₃, -CH₂CD₃, -CH₂CD₂H, -CH₂CDH₂, -CHDCH₃, - CHDCD₂H, -CHDCDH₂, -CHDCD₃, -CD₂CD₃, -CD₂CD₂H oder -CD₂CDH₂; oder
eine n-Propylgruppe, eine Isopropylgruppe, eine n-Butylgruppe, eine sec-Butylgruppe, eine Isobutylgruppe, eine tert-Butylgruppe, eine n-Pentylgruppe, eine tert-Pentylgruppe, eine neoPentylgruppe, eine Isopentylgruppe, eine sec-Pentylgruppe, eine 3-Pentylgruppe, eine sec-Isopentylgruppe, eine Phenylgruppe, eine Biphenylgruppe oder eine Naphthylgruppe, jeweils unsubstituiert oder substituiert mit Deuterium, einer C₁-C₁₀-Alkylgruppe, einer Phenylgruppe oder eine beliebige Kombination davon.

3. Metallorganische Verbindung nach Anspruch 1 oder 2, wobei
R₂₁ eine Ethylgruppe, eine n-Propylgruppe, eine Isopropylgruppe, eine n-Butylgruppe, eine sec-Butylgruppe, eine Isobutylgruppe, eine tert-Butylgruppe, eine n-Pentylgruppe, eine tert-Pentylgruppe, eine Neopentylgruppe, eine Isopentylgruppe, eine Sec-Pentylgruppe, eine 3-Pentylgruppe, eine Sec-Isopentylgruppe, eine n-Hexylgruppe, eine Isohexylgruppe, eine Sec-Hexylgruppe, eine Tert-Hexylgruppe, eine n-Heptylgruppe, eine Isoheptylgruppe, eine Sec-Heptylgruppe, eine Tert-Heptylgruppe, eine n-Octylgruppe, eine Isooctylgruppe, eine sec-Octylgruppe, eine tert-Octylgruppe, eine n-Nonylgruppe, eine Isononylgruppe, eine sec-Nonylgruppe, eine tert-Nonylgruppe, eine n-Decylgruppe, eine Isodecylgruppe, eine sec-Decylgruppe, eine tert-Decylgruppe, eine Cyclopentylgruppe, eine Cyclohexylgruppe, eine Cycloheptylgruppe, eine Cyclooctylgruppe, eine Bicyclo[1.1.1]pentylgruppe, eine Bicyclo[2.1.1]hexylgruppe, eine Bicyclo[2.2.1]heptylgruppe oder eine Bicyclo[2.2.2]octylgruppe ist, jeweils unsubstituiert oder substituiert mit mindestens einem Deuterium, und
R₂₂ eine Methylgruppe, eine Ethylgruppe, eine n-Propylgruppe, eine Isopropylgruppe, eine n-Butylgruppe, eine sec-Butylgruppe, eine Isobutylgruppe, eine tert-Butylgruppe, eine n-Pentylgruppe, eine tert-Pentylgruppe, eine Neopentylgruppe, eine Isopentylgruppe, eine sec-Pentylgruppe, eine 3-Pentylgruppe, eine sec-Isopentylgruppe, eine n-Hexylgruppe, eine Isohexylgruppe, eine sec-Hexylgruppe, eine tert-Hexylgruppe, eine n-Heptylgruppe, eine Isoheptylgruppe, eine sec-Heptylgruppe, eine tert-Heptylgruppe, eine n-Octylgruppe, eine Isooctylgruppe, eine sec-Octylgruppe, eine tert-Octylgruppe, eine n-Nonylgruppe, eine Isononylgruppe, eine sec-Nonylgruppe, eine tert-Nonylgruppe, eine n-Decylgruppe, eine Isodecylgruppe, eine sec-Decylgruppe, eine tert-Decylgruppe, eine Cyclopentylgruppe, eine Cyclohexylgruppe, eine Cycloheptylgruppe, eine Cyclooctylgruppe, eine Bicyclo[1.1.1]pentylgruppe, eine Bicyclo[2.1.1]hexylgruppe, eine Bicyclo[2.2.1]heptylgruppe oder eine Bicyclo[2.2.2]octylgruppe ist, jeweils unsubstituiert oder substituiert mit mindestens einem Deuterium.

4. Metallorganische Verbindung nach einem der Ansprüche 1-3, wobei
R₆ und R₈ jeweils unabhängig Wasserstoff oder Deuterium sind, und
R₇ und R₉ jeweils unabhängig eine substituierte oder unsubstituierte C₁-C₆₀-Alkylgruppe, eine substituierte oder unsubstituierte C₃-C₁₀-Cycloalkylgruppe, -Si(Q₃)(Q₄)(Q₅), oder - Ge(Q₃)(Q₄)(Q₅) sind.

5. Metallorganische Verbindung nach einem der Ansprüche 1-4, wobei R₇ und R₉ jeweils unabhängig eine substituierte oder unsubstituierte C₁-C₆₀-Alkylgruppe sind und R₇ und R₉ voneinander verschieden sind;
wobei vorzugsweise die Anzahl der in R₇ enthaltenen Kohlenstoffatome größer ist als die Anzahl der in R₉ enthaltenen Kohlenstoffatome.

6. Metallorganische Verbindung nach einem der Ansprüche 1-5, wobei R₂₁ eine C₃-C₁₀-Cycloalkylgruppe oder eine Deuterium-haltige C₃-C₁₀-Cycloalkylgruppe ist; und/oder
wobei R₂₂ eine Methylgruppe ist.

7. Metallorganische Verbindung nach einem der Ansprüche 1-6, wobei R₂₂ eine C₂-C₂₀-Alkylgruppe, eine Deuterium-haltige C₂-C₂₀-Alkylgruppe, eine C₃-C₁₀-Cycloalkylgruppe, oder eine Deuterium-haltige C₃-C₁₀-Cycloalkylgruppe ist; und/oder
wobei R₂₃ Wasserstoff oder Deuterium ist.

8. Metallorganische Verbindung nach einem der Ansprüche 1-7, wobei
die metallorganische Verbindung <Bedingung 3>, <Bedingung 4>, oder eine Kombination davon erfüllt:
<Bedingung 3>
R₁₀ in Formel 1 ist durch Formel 3 dargestellt
<Bedingung 4>
R₁₂ in Formel 1 ist durch Formel 4 dargestellt 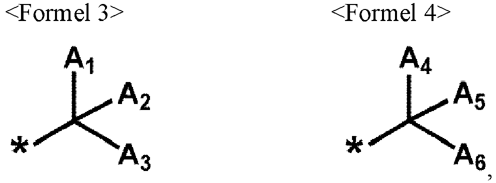
wobei in Formel 3 und 4
A₁ bis A₆ jeweils unabhängig wie folgt sind:
Wasserstoff oder Deuterium, oder
eine C₁-C₂₀-Alkylgruppe, eine C₃-C₁₀-Cycloalkylgruppe, eine C₆-C₆₀-Arylgruppe oder eine C₁-C₆₀-Heteroarylgruppe, jeweils unsubstituiert oder substituiert mit Deuterium, eine C₁-C₂₀-Alkylgruppe, eine C₃-C₁₀-Cycloalkylgruppe oder eine beliebige Kombination davon, und
* eine Bindungsstelle zu einem benachbarten Atom angibt;
wobei vorzugsweise
Formel 3 eine von <Bedingung 3-1> bis <Bedingung 3-5> erfüllt, und
Formel 4 eine von <Bedingung 4-1> bis <Bedingung 4-5> erfüllt:
<Bedingung 3-1>
A₁ bis A₃ sind nicht gleichzeitig Wasserstoff
<Bedingung 3-2>
A₁ bis A₃ sind jeweils unabhängig eine C₁-C₂₀-Alkylgruppe oder eine C₃-C₁₀-Cycloalkylgruppe, jeweils unsubstituiert oder substituiert mit Deuterium, eine C₁-C₂₀-Alkylgruppe, eine C₃-C₁₀-Cycloalkylgruppe oder eine Kombination davon
<Bedingung 3-3>
A₁ bis A₂ sind jeweils unabhängig eine C₁-C₂₀-Alkylgruppe oder eine C₃-C₁₀-Cycloalkylgruppe, jeweils unsubstituiert oder substituiert mit Deuterium, eine C₁-C₂₀-Alkylgruppe, eine C₃-C₁₀-Cycloalkylgruppe oder eine Kombination davon, und
A₃ ist eine C₂-C₂₀-Alkylgruppe oder eine C₃-C₁₀-Cycloalkylgruppe, jeweils unsubstituiert oder substituiert mit Deuterium, eine C₁-C₂₀-Alkylgruppe, eine C₃-C₁₀-Cycloalkylgruppe oder eine Kombination davon
<Bedingung 3-4>
A₁ ist eine C₁-C₂₀-Alkylgruppe oder eine C₃-C₁₀-Cycloalkylgruppe, jeweils unsubstituiert oder substituiert mit Deuterium, eine C₁-C₂₀-Alkylgruppe, eine C₃-C₁₀-Cycloalkylgruppe oder eine Kombination davon, und
A₂ und A₃ sind jeweils unabhängig eine C₂-C₂₀-Alkylgruppe oder eine C₃-C₁₀-Cycloalkylgruppe, jeweils unsubstituiert oder substituiert mit Deuterium, eine C₁-C₂₀-Alkylgruppe, eine C₃-C₁₀-Cycloalkylgruppe oder eine Kombination davon
<Bedingung 3-5>
A₁ ist Wasserstoff oder Deuterium,
A₂ ist eine C₁-C₂₀-Alkylgruppe oder eine C₃-C₁₀-Cycloalkylgruppe, jeweils unsubstituiert oder substituiert mit Deuterium, eine C₁-C₂₀-Alkylgruppe, eine C₃-C₁₀-Cycloalkylgruppe oder eine Kombination davon, und
A₃ ist eine C₂-C₂₀-Alkylgruppe oder eine C₃-C₁₀-Cycloalkylgruppe, jeweils unsubstituiert oder substituiert mit Deuterium, eine C₁-C₂₀-Alkylgruppe, eine C₃-C₁₀-Cycloalkylgruppe oder eine Kombination davon
<Bedingung 4-1>
A₄ bis A₆ sind nicht gleichzeitig Wasserstoff
<Bedingung 4-2>
A₄ bis A₆ sind jeweils unabhängig eine C₁-C₂₀-Alkylgruppe oder eine C₃-C₁₀-Cycloalkylgruppe, jeweils unsubstituiert oder substituiert mit Deuterium, eine C₁-C₂₀-Alkylgruppe, eine C₃-C₁₀-Cycloalkylgruppe oder eine Kombination davon
<Bedingung 4-3>
A₄ und A₅ sind jeweils unabhängig eine C₁-C₂₀-Alkylgruppe oder eine C₃-C₁₀-Cycloalkylgruppe, jeweils unsubstituiert oder substituiert mit Deuterium, eine C₁-C₂₀-Alkylgruppe, eine C₃-C₁₀-Cycloalkylgruppe oder eine Kombination davon, und
A₆ ist eine C₂-C₂₀-Alkylgruppe oder eine C₃-C₁₀-Cycloalkylgruppe, jeweils unsubstituiert oder substituiert mit Deuterium, eine C₁-C₂₀-Alkylgruppe, eine C₃-C₁₀-Cycloalkylgruppe oder eine Kombination davon
<Bedingung 4-4>
A₄ ist eine C₁-C₂₀-Alkylgruppe oder eine C₃-C₁₀-Cycloalkylgruppe, jeweils unsubstituiert oder substituiert mit Deuterium, eine C₁-C₂₀-Alkylgruppe, eine C₃-C₁₀-Cycloalkylgruppe oder eine Kombination davon, und
A₅ und A₆ sind jeweils unabhängig eine C₂-C₂₀-Alkylgruppe oder eine C₃-C₁₀-Cycloalkylgruppe, jeweils unsubstituiert oder substituiert mit Deuterium, eine C₁-C₂₀-Alkylgruppe, eine C₃-C₁₀-Cycloalkylgruppe oder eine Kombination davon
<Bedingung 4-5>
A₄ ist Wasserstoff oder Deuterium,
A₅ ist eine C₁-C₂₀-Alkylgruppe oder eine C₃-C₁₀-Cycloalkylgruppe, jeweils unsubstituiert oder substituiert mit Deuterium, eine C₁-C₂₀-Alkylgruppe, eine C₃-C₁₀-Cycloalkylgruppe oder eine Kombination davon, und
A₆ ist eine C₂-C₂₀-Alkylgruppe oder eine C₃-C₁₀-Cycloalkylgruppe, jeweils unsubstituiert oder substituiert mit Deuterium, eine C₁-C₂₀-Alkylgruppe, eine C₃-C₁₀-Cycloalkylgruppe oder eine Kombination davon.

9. Metallorganische Verbindung nach einem der Ansprüche 1-8, wobei
mindestens eines von R₁₀ und R₁₂ jeweils unabhängig eine Cyclopentylgruppe, eine Cyclohexylgruppe, eine Cycloheptylgruppe, eine Cyclooctylgruppe, eine Adamantanylgruppe, eine Norbomanylgruppe, eine Norbornenylgruppe, eine Cyclopentenylgruppe, eine Cyclohexenylgruppe, eine Cycloheptenylgruppe, eine Bicyclo[1.1.1]pentylgruppe, eine Bicyclo[2.1.1]hexylgruppe, eine Bicyclo[2.2.1]heptylgruppe oder eine Bicyclo[2.2.2]octylgruppe, jeweils unsubstituiert oder substituiert mit Deuterium, -F, -CD₃, -CD₂H, -CDH₂, -CF₃, -CF₂H, - CFH₂, eine Cyanogruppe, eine C₁-C₁₀-Alkylgruppe oder eine Kombination davon ist.

10. Metallorganische Verbindung nach Anspruch 1, wobei
die metallorganische Verbindung eine von Verbindung 1 bis 22 ist:

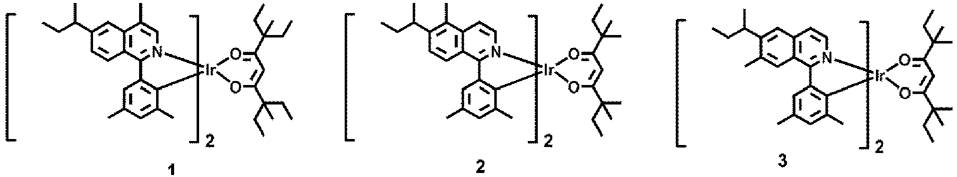

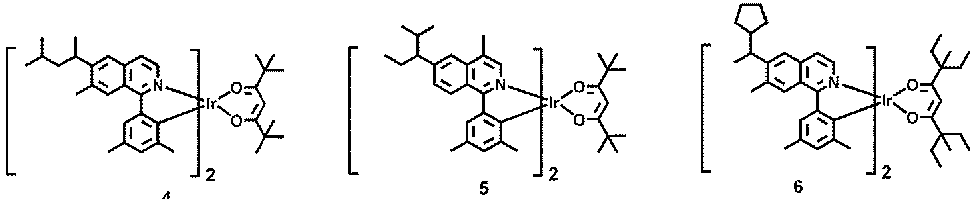

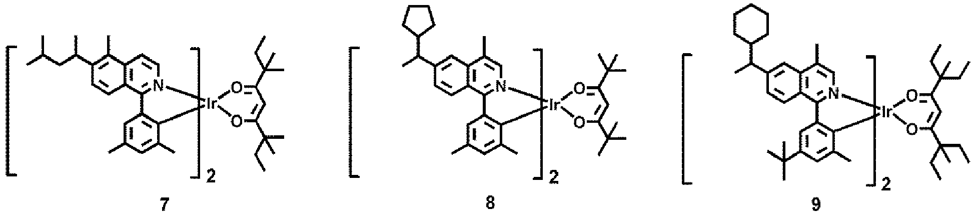

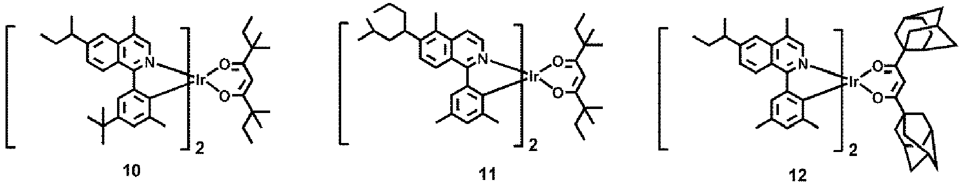

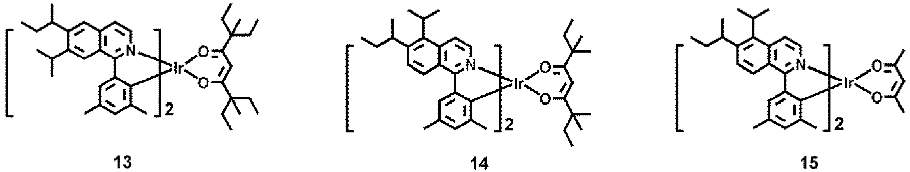

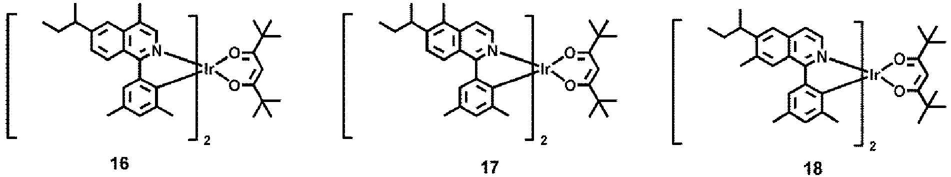

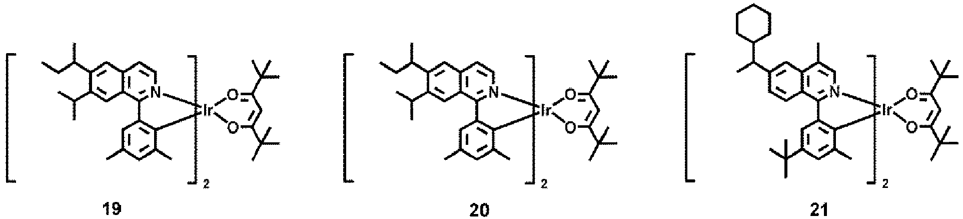

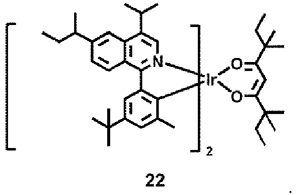

11. Organische lichtemittierende Vorrichtung, umfassend:
eine erste Elektrode;
eine zweite Elektrode; und
eine organische Schicht, die zwischen der ersten Elektrode und der zweiten Elektrode angeordnet ist und umfassend eine Emissionsschicht,
wobei die organische Schicht mindestens eine metallorganische Verbindung nach einem der Ansprüche 1-10 umfasst.

12. Organische lichtemittierende Vorrichtung nach Anspruch 11, wobei
die erste Elektrode eine Anode ist,
die zweite Elektrode eine Kathode ist,
die organische Schicht ferner einen Lochtransportbereich, angeordnet zwischen der ersten Elektrode und der Emissionsschicht, und einen Elektronentransportbereich, angeordnet zwischen der Emissionsschicht und der zweiten Elektrode, umfasst,
der Lochtransportbereich eine Lochinjektionsschicht, eine Lochtransportschicht, eine Elektronensperrschicht, eine Pufferschicht oder eine beliebige Kombination davon umfasst, und
der Elektronentransportbereich eine Lochsperrschicht, eine Elektronentransportschicht, eine Elektroneninjektionsschicht oder eine beliebige Kombination davon umfasst.

13. Organische lichtemittierende Vorrichtung nach Anspruch 11 oder 12, wobei
die Emissionsschicht die metallorganische Verbindung umfasst.

14. Organische lichtemittierende Vorrichtung nach Anspruch 13, wobei die Emissionsschicht rotes Licht emittiert; und/oder
wobei die Emissionsschicht ferner einen Wirt umfasst, und eine Menge des Wirts in der Emissionsschicht größer ist als eine Menge der metallorganischen Verbindung in der Emissionsschicht.

15. Diagnostische Zusammensetzung, umfassend mindestens eine metallorganische Verbindung nach einem der Ansprüche 1-10.

## Revendications

1. Composé organométallique représenté par la Formule 1 : 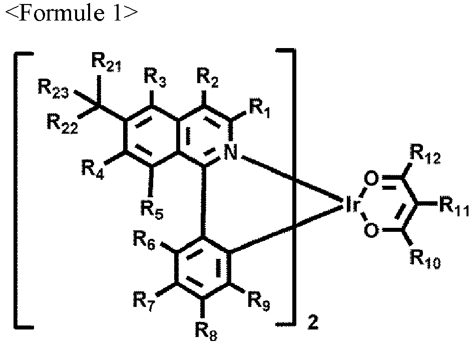
avec dans la Formule 1,
R₁ à R₁₂ et R₂₃ sont chacun indépendamment l'hydrogène, le deutérium, -F, -Cl, -Br, -I, - SF₅, un groupe hydroxyle, un groupe cyano, un groupe nitro, un groupe amino, un groupe amidino, un groupe hydrazino, un groupe hydrazono, un groupe acide carboxylique ou un de ses sels, un groupe acide sulfonique ou un de ses sels, un groupe acide phosphorique ou un de ses sels, un groupe C₁-C₆₀ alkyle substitué ou non substitué, un groupe C₂-C₆₀ alcényle substitué ou non substitué, un groupe C₂-C₆₀ alcynyle substitué ou non substitué, un groupe C₁-C₆₀ alcoxy substitué ou non substitué, un groupe C₃-C₁₀ cycloalkyle substitué ou non substitué, un groupe C₁-C₁₀ hétérocycloalkyle substitué ou non substitué, un groupe C₃-C₁₀ cycloalcényle substitué ou non substitué, un groupe C₁-C₁₀ hétérocycloalcényle substitué ou non substitué, un groupe C₆-C₆₀ aryle substitué ou non substitué, un groupe C₆-C₆₀ aryloxy substitué ou non substitué, un groupe C₆-C₆₀ arylthio substitué ou non substitué, un groupe C₁-C₆₀ hétéroaryle substitué ou non substitué, un groupe polycyclique monovalent non-aromatique condensé substitué ou non substitué, un groupe hétéropolycyclique monovalent non-aromatique condensé substitué ou non substitué, -N(Q₁)(Q₂), -Si(Q₃)(Q₄)(Q₅), -Ge(Q₃)(Q₄)(Q₅), -B(Q₆)(Q₇), -P(=O)(Q₈)(Q₉), ou -P(Q₈)(Q₉),
au moins un parmi R₁ à R₅ est chacun indépendamment le deutérium, -F, -Cl, -Br, -I, -SF₅, un groupe hydroxyle, un groupe cyano, un groupe nitro, un groupe amino, un groupe amidino, un groupe hydrazino, un groupe hydrazono, un groupe acide carboxylique ou un de ses sels, un groupe acide sulfonique ou un de ses sels, un groupe acide phosphorique ou un de ses sels, un groupe C₁-C₆₀ alkyle substitué ou non substitué, un groupe C₂-C₆₀ alcényle substitué ou non substitué, un groupe C₂-C₆₀ alcynyle substitué ou non substitué, un groupe C₁-C₆₀ alcoxy substitué ou non substitué, un groupe C₃-C₁₀ cycloalkyle substitué ou non substitué, un groupe C₁-C₁₀ hétérocycloalkyle substitué ou non substitué, un groupe C₃-C₁₀ cycloalcényle substitué ou non substitué, un groupe C₁-C₁₀ hétérocycloalcényle substitué ou non substitué, un groupe C₆-C₆₀ aryle substitué ou non substitué, un groupe C₆-C₆₀ aryloxy substitué ou non substitué, un groupe C₆-C₆₀ arylthio substitué ou non substitué, un groupe C₁-C₆₀ hétéroaryle substitué ou non substitué, un groupe polycyclique monovalent non-aromatique condensé substitué ou non substitué, un groupe hétéropolycyclique monovalent non-aromatique condensé substitué ou non substitué, -N(Q₁)(Q₂), -Si(Q₃)(Q₄)(Q₅), -Ge(Q₃)(Q₄)(Q₅), -B(Q₆)(Q₇), -P(=O)(Q₈)(Q₉), ou -P(Q₈)(Q₉),
R₂₁ est un groupe C₂-C₂₀ alkyle, un groupe C₂-C₂₀ alkyle contenant du deutérium, un groupe C₃-C₁₀ cycloalkyle, ou un groupe C₃-C₁₀ cycloalkyle contenant du deutérium,
R₂₂ est un groupe C₁-C₂₀ alkyle, un groupe C₁-C₂₀ alkyle contenant du deutérium, un groupe C₃-C₁₀ cycloalkyle, ou un groupe C₃-C₁₀ cycloalkyle contenant du deutérium,
deux ou plusieurs parmi R₁ à R₉ sont éventuellement liés les uns aux autres pour former un groupe C₅-C₃₀ carbocyclique non substitué ou substitué par au moins un R₁ₐ ou un groupe C₂-C₃₀ hétérocyclique non substitué ou substitué par au moins un R₁ₐ,
deux ou plusieurs parmi R₁₀ à R₁₂ sont éventuellement liés les uns aux autres pour former un groupe C₅-C₃₀ carbocyclique non substitué ou substitué par au moins un R₁ₐ ou un groupe C₂-C₃₀ hétérocyclique non substitué ou substitué par au moins un R₁ₐ,
R₁ₐ est le même que celui défini en relation avec R₁,
un ou des substituants du groupe C₁-C₆₀ alkyle substitué, du groupe C₂-C₆₀ alcényle substitué, du groupe C₂-C₆₀ alcynyle substitué, du groupe C₁-C₆₀ alcoxy substitué, du groupe C₃-C₁₀ cycloalkyle substitué, du groupe C₁-C₁₀ hétérocycloalkyle substitué, du groupe C₃-C₁₀ cycloalcényle substitué, du groupe C₁-C₁₀ hétérocycloalcényle substitué, du groupe C₆-C₆₀ aryle substitué, du groupe C₆-C₆₀ aryloxy substitué, du groupe C₆-C₆₀ arylthio substitué, du groupe C₁-C₆₀ hétéroaryle substitué, du groupe polycyclique monovalent non-aromatique condensé substitué, et du groupe hétéropolycyclique monovalent non-aromatique condensé substitué sont chacun indépendamment :
deutérium, -F, -Cl, -Br, -I, -CD₃, -CD₂H, -CDH₂, -CF₃, -CF₂H, -CFH₂, un groupe hydroxyle, un groupe cyano, un groupe nitro, un groupe amino, un groupe amidino, un groupe hydrazino, un groupe hydrazono, un groupe acide carboxylique ou un de ses sels, un groupe acide sulfonique ou un de ses sels, un groupe acide phosphorique ou un de ses sels, un groupe C₁-C₆₀ alkyle, un groupe C₂-C₆₀ alcényle, un groupe C₂-C₆₀ alcynyle, ou un groupe C₁-C₆₀ alcoxy ;
un groupe C₁-C₆₀ alkyle, un groupe C₂-C₆₀ alcényle, un groupe C₂-C₆₀ alcynyle, ou un groupe C₁-C₆₀ alcoxy, chacun substitué par le deutérium, -F, -Cl, -Br, -I, -CD₃, -CD₂H, -CDH₂, - CF₃, -CF₂H, -CFH₂, un groupe hydroxyle, un groupe cyano, un groupe nitro, un groupe amino, un groupe amidino, un groupe hydrazino, un groupe hydrazono, un groupe acide carboxylique ou un de ses sels, un groupe acide sulfonique ou un de ses sels, un groupe acide phosphorique ou un de ses sels, un groupe C₃-C₁₀ cycloalkyle, un groupe C₁-C₁₀ hétérocycloalkyle, un groupe C₃-C₁₀ cycloalcényle, un groupe C₁-C₁₀ hétérocycloalcényle, un groupe C₆-C₆₀ aryle, un groupe C₆-C₆₀ aryloxy, un groupe C₆-C₆₀ arylthio, un groupe C₁-C₆₀ hétéroaryle, un groupe polycyclique monovalent non-aromatique condensé, un groupe hétéropolycyclique monovalent non-aromatique condensé, -N(Q₁₁)(Q₁₂), -Si(Q₁₃)(Q₁₄)(Q₁₅), -Ge(Q₁₃)(Q₁₄)(Q₁₅), -B(Q₁₆)(Q₁₇), -P(=O)(Q₁₈)(Q₁₉), -P(Q₁₈)(Q₁₉), ou une combinaison quelconque de ceux-ci ;
un groupe C₃-C₁₀ cycloalkyle, un groupe C₁-C₁₀ hétérocycloalkyle, un groupe C₃-C₁₀ cycloalcényle, un groupe C₁-C₁₀ hétérocycloalcényle, un groupe C₆-C₆₀ aryle, un groupe C₆-C₆₀ aryloxy, un groupe C₆-C₆₀ arylthio, un groupe C₁-C₆₀ hétéroaryle, un groupe polycyclique monovalent non-aromatique condensé, ou un groupe hétéropolycyclique monovalent non-aromatique condensé, chacun non substitué ou substitué par le deutérium, -F, -Cl, -Br, -I, -CD₃, - CD₂H, -CDH₂, -CF₃, -CF₂H, -CFH₂, un groupe hydroxyle, un groupe cyano, un groupe nitro, un groupe amino, un groupe amidino, un groupe hydrazino, un groupe hydrazono, un groupe acide carboxylique ou un de ses sels, un groupe acide sulfonique ou un de ses sels, un groupe acide phosphorique ou un de ses sels, un groupe C₁-C₆₀ alkyle, un groupe C₂-C₆₀ alcényle, un groupe C₂-C₆₀ alcynyle, un groupe C₁-C₆₀ alcoxy, un groupe C₃-C₁₀ cycloalkyle, un groupe C₁-C₁₀ hétérocycloalkyle, un groupe C₃-C₁₀ cycloalcényle, un groupe C₁-C₁₀ hétérocycloalcényle, un groupe C₆-C₆₀ aryle, un groupe C₆-C₆₀ aryloxy, un groupe C₆-C₆₀ arylthio, un groupe C₁-C₆₀ hétéroaryle, un groupe polycyclique monovalent non-aromatique condensé, un groupe hétéropolycyclique monovalent non-aromatique condensé, -N(Q₂₁)(Q₂₂), -Si(Q₂₃)(Q₂₄)(Q₂₅), - Ge(Q₂₃)(Q₂₄)(Q₂₅), -B(Q₂₆)(Q₂₇), -P(=O)(Q₂₈)(Q₂₉), -P(Q₂₈)(Q₂₉), ou une combinaison quelconque de ceux-ci ;
-N(Q₃₁)(Q₃₂), -Si(Q₃₃)(Q₃₄)(Q₃₅), -Ge(Q₃₃)(Q₃₄)(Q₃₅), -B(Q₃₆)(Q₃₇), -P(=O)(Q₃₈)(Q₃₉) ou - P(Q₃₈)(Q₃₉) ; ou
une combinaison quelconque de ceux-ci ; et
Q₁ à Q₉, Q₁₁ à Q₁₉, Q₂₁ à Q₂₉, et Q₃₁ à Q₃₉ sont chacun indépendamment l'hydrogène ; le deutérium ; -F ; -Cl ; -Br ; -I ; un groupe hydroxyle ; un groupe cyano ; un groupe nitro ; un groupe amino ; un groupe amidino ; un groupe hydrazine ; un groupe hydrazone ; un groupe acide carboxylique ou un de ses sels ; un groupe acide sulfonique ou un de ses sels ; un groupe acide phosphorique ou un de ses sels ; un groupe C₁-C₆₀ alkyle non substitué ou substitué par le deutérium, un groupe C₁-C₆₀ alkyle, un groupe C₆-C₆₀ aryle ou une combinaison quelconque de ceux-ci ; un groupe C₂-C₆₀ alcényle ; un groupe C₂-C₆₀ alcynyle ; un groupe C₁-C₆₀ alcoxy ; un groupe C₃-C₁₀ cycloalkyle ; un groupe C₁-C₁₀ hétérocycloalkyle ; un groupe C₃-C₁₀ cycloalcényle ; un groupe C₁-C₁₀ hétérocycloalcényle ; un groupe C₆-C₆₀ aryle non substitué ou substitué par un groupe C₁-C₆₀ alkyle, un groupe C₆-C₆₀ aryle, ou une combinaison quelconque de ceux-ci ; un groupe C₆-C₆₀ aryloxy ; un groupe C₆-C₆₀ arylthio ; un groupe C₁-C₆₀ hétéroaryle ; un groupe polycyclique monovalent non-aromatique condensé ; ou un groupe hétéropolycyclique monovalent non-aromatique condensé.

2. Composé organométallique de la revendication 1, dans lequel
R₁ à R₁₂ et R₂₃ sont chacun indépendamment :
hydrogène, deutérium, -F, un groupe cyano, ou un groupe C₁-C₂₀ alkyle ;
un groupe C₁-C₂₀ alkyle substitué par le deutérium, -F, -CD₃, -CD₂H, -CDH₂, -CF₃, -CF₂H, -CFH₂, un groupe cyano, un groupe C₁-C₁₀ alkyle, un groupe cyclopentyle, un groupe cyclohexyle, un groupe cycloheptyle, un groupe cyclooctyle, un groupe adamantanyle, un groupe norbornanyle, un groupe norbornényle, un groupe cyclopentényle, un groupe cyclohexényle, un groupe cycloheptenyle, un groupe bicyclo[1.1.1]pentyle, un groupe bicyclo[2.1.1]hexyle, un groupe bicyclo[2.2.1]heptyle, un groupe bicyclo[2.2.2]octyle, un groupe phényle, un groupe (C₁-C₂₀ alkyl)phényle, un groupe biphényle, un groupe terphényle, ou une combinaison de ceux-ci ;
un groupe cyclopentyle, un groupe cyclohexyle, un groupe cycloheptyle, un groupe cyclooctyle, un groupe adamantanyle, un groupe norbornanyle, un groupe norbornényle, un groupe cyclopentényle, un groupe cyclohexényle, un groupe cycloheptényle, un groupe bicyclo[1.1.1]pentyle, un groupe bicyclo[2.1.1]hexyle, un groupe bicyclo[2.2.1]heptyle, un groupe bicyclo[2.2.2]octyle, un groupe phényle, un groupe (C₁-C₂₀ alkyl)phényle, un groupe biphényle, ou un groupe terphényle, chacun non substitué ou substitué par le deutérium, -F, -CD₃, -CD₂H, -CDH₂, -CF₃, -CF₂H, -CFH₂, un groupe cyano, un groupe C₁-C₁₀ alkyle, un groupe cyclopentyle, un groupe cyclohexyle, un groupe cycloheptyle, un groupe cyclooctyle, un groupe adamantanyle, un groupe norbornanyle, un groupe norbornényle, un groupe cyclopentényle, un groupe cyclohexényle, un groupe cycloheptényle, un groupe bicyclo[1.1.1]pentyle, un groupe bicyclo[2.1.1]hexyle, un groupe bicyclo[2.2.1]heptyle, un groupe bicyclo[2.2.2]octyle, un groupe phényle, un groupe (C₁-C₂₀ alkyl)phényle, un groupe biphényle, un groupe terphényle, ou une combinaison quelconque de ceux-ci ; ou
-Si(Q₃)(Q₄)(Q₅), ou -Ge(Q₃)(Q₄)(Q₅) ; et
chacun d'au moins un parmi R₁ à R₅ est indépendamment :
deutérium, -F, un groupe cyano, ou un groupe C₁-C₂₀ alkyle ;
un groupe C₁-C₂₀ alkyle substitué par le deutérium, -F, -CD₃, -CD₂H, -CDH₂, -CF₃, -CF₂H, -CFH₂, un groupe cyano, un groupe C₁-C₁₀ alkyle, un groupe cyclopentyle, un groupe cyclohexyle, un groupe cycloheptyle, un groupe cyclooctyle, un groupe adamantanyle, un groupe norbornanyle, un groupe norbornényle, un groupe cyclopentényle, un groupe cyclohexényle, un groupe cycloheptényle, un groupe bicyclo[1.1.1]pentyle, un groupe bicyclo[2.1.1]hexyle, un groupe bicyclo[2.2.1]heptyle, un groupe bicyclo[2.2.2]octyle, un groupe phényle, un groupe (C₁-C₂₀ alkyl)phényle, un groupe biphényle, un groupe terphényle, ou une combinaison de ceux-ci ;
un groupe cyclopentyle, un groupe cyclohexyle, un groupe cycloheptyle, un groupe cyclooctyle, un groupe adamantanyle, un groupe norbornanyle, un groupe norbornényle, un groupe cyclopentényle, un groupe cyclohexényle, un groupe cycloheptényle, un groupe bicyclo[1.1.1]pentyle, un groupe bicyclo[2.1.1]hexyle, un groupe bicyclo[2.2.1]heptyle, un groupe bicyclo[2.2.2]octyle, un groupe phényle, un groupe (C₁-C₂₀ alkyl)phényle, un groupe biphényle, ou un groupe terphényle, chacun non substitué ou substitué par le deutérium, -F, -CD₃, -CD₂H, -CDH₂, -CF₃, -CF₂H, -CFH₂, un groupe cyano, un groupe C₁-C₁₀ alkyle, un groupe cyclopentyle, un groupe cyclohexyle, un groupe cycloheptyle, un groupe cyclooctyle, un groupe adamantanyle, un groupe norbornanyle, un groupe norbornényle, un groupe cyclopentényle, un groupe cyclohexényle, un groupe cycloheptényle, un groupe bicyclo[1.1.1]pentyle, un groupe bicyclo[2.1.1]hexyle, un groupe bicyclo[2.2.1]heptyle, un groupe bicyclo[2.2.2]octyle, un groupe phényle, un groupe (C₁-C₂₀ alkyl)phényle, un groupe biphényle, un groupe terphényle, ou une combinaison quelconque de ceux-ci ; ou
-Si(Q₃)(Q₄)(Q₅), ou -Ge(Q₃)(Q₄)(Q₅),
Q₃ à Q₅ étant chacun indépendamment :
-CH₃, -CD₃, -CD₂H, -CDH₂, -CH₂CH₃, -CH₂CD₃, -CH₂CD₂H, -CH₂CDH₂, -CHDCH₃, - CHDCD₂H, -CHDCDH₂, -CHDCD₃, -CD₂CD₃, -CD₂CD₂H, ou -CD₂CDH₂ ; ou
un groupe n-propyle, un groupe isopropyle, un groupe n-butyle, un groupe sec-butyle, un groupe isobutyle, un groupe tert-butyle, un groupe n-pentyle, un groupe tert-pentyle, un groupe néopentyle, un groupe isopentyle, un groupe sec-pentyle, un groupe 3-pentyle, un groupe sec-isopentyle, un groupe phényle, un groupe biphényle, ou un groupe naphthyle, chacun non substitué ou substitué par le deutérium, un groupe C₁-C₁₀ alkyle, un groupe phényle, ou une combinaison quelconque de ceux-ci.

3. Composé organométallique de la revendication 1 ou 2, dans lequel
R₂₁ est un groupe éthyle, un groupe n-propyle, un groupe isopropyle, un groupe n-butyle, un groupe sec-butyle, un groupe isobutyle, un groupe tert-butyle, un groupe n-pentyle, un groupe tert-pentyle,
un groupe néopentyle, un groupe isopentyle, un groupe sec-pentyle, un groupe 3-pentyle, un groupe sec-isopentyle, un groupe n-hexyle, un groupe isohexyle, un groupe sec-hexyle, un groupe tert-hexyle, un groupe n-heptyle, un groupe isoheptyle, un groupe sec-heptyle, un groupe tert-heptyle, un groupe n-octyle, un groupe isooctyle, un groupe sec-octyle, un groupe tert-octyle, un groupe n-nonyle, un groupe isononyle, un groupe sec-nonyle, un groupe tert-nonyle, un groupe n-décyle, un groupe isodécyle, un groupe sec-décyle, un groupe tert-décyle, un groupe cyclopentyle, un groupe cyclohexyle, un groupe cycloheptyle, un groupe cyclooctyle, un groupe bicyclo[1.1.1]pentyle, un groupe bicyclo[2.1.1]hexyle, un groupe bicyclo[2.2.1]heptyle, ou un groupe bicyclo[2.2.2]octyle, chacun non substitué ou substitué par au moins un deutérium, et
R₂₂ est un groupe méthyle, un groupe éthyle, un groupe n-propyle, un groupe isopropyle, un groupe n-butyle, un groupe sec-butyle, un groupe isobutyle, un groupe tert-butyle, un groupe n-pentyle, un groupe tert-pentyle, un groupe néopentyle, un groupe isopentyle, un groupe sec-pentyle, un groupe 3-pentyle, un groupe sec-isopentyle, un groupe n-hexyle, un groupe isohexyle, un groupe sec-hexyle, un groupe tert-hexyle, un groupe n-heptyle, un groupe isoheptyle, un groupe sec-heptyle, un groupe tert-heptyle, un groupe n-octyle, un groupe isooctyle, un groupe sec-octyle, un groupe tert-octyle, un groupe n-nonyle, un groupe isononyle, un groupe sec-nonyle, un groupe tert-nonyle, un groupe n-décyle, un groupe isodécyle, un groupe sec-décyle, un groupe tert-décyle, un groupe cyclopentyle, un groupe cyclohexyle, un groupe cycloheptyle, un groupe cyclooctyle, un groupe bicyclo[1.1.1]pentyle, un groupe bicyclo[2.1.1]hexyle, un groupe bicyclo[2.2.1]heptyle, ou un groupe bicyclo[2.2.2]octyle, chacun non substitué ou substitué par au moins un deutérium.

4. Composé organométallique de l'une quelconque des revendications 1 à 3, dans lequel
R₆ et R₈ sont chacun indépendamment l'hydrogène ou le deutérium, et
R₇ et R₉ sont chacun indépendamment un groupe C₁-C₆₀ alkyle substitué ou non substitué, un groupe C₃-C₁₀ cycloalkyle substitué ou non substitué, -Si(Q₃)(Q₄)(Q₅), ou -Ge(Q₃)(Q₄)(Q₅).

5. Composé organométallique de l'une quelconque des revendications 1 à 4, dans lequel R₇ et R₉ sont chacun indépendamment un groupe C₁-C₆₀ alkyle substitué ou non substitué et R₇ et R₉ sont différents l'un de l'autre ;
de préférence dans lequel le nombre d'atomes de carbone inclus dans R₇ est supérieur au nombre d'atomes de carbone inclus dans R₉.

6. Composé organométallique de l'une quelconque des revendications 1 à 5, dans lequel R₂₁ est un groupe C₃-C₁₀ cycloalkyle, ou un groupe C₃-C₁₀ cycloalkyle contenant du deutérium ; et/ou
dans lequel R₂₂ est un groupe méthyle.

7. Composé organométallique de l'une quelconque des revendications 1 à 6, dans lequel R₂₂ est un groupe C₂-C₂₀ alkyle, un groupe C₂-C₂₀ alkyle contenant du deutérium, un groupe C₃-C₁₀ cycloalkyle, ou un groupe C₃-C₁₀ cycloalkyle contenant du deutérium ; et/ou
dans lequel R₂₃ est l'hydrogène ou le deutérium.

8. Composé organométallique de l'une quelconque des revendications 1 à 7,
le composé organométallique satisfaisant à la <Condition 3>, à la <Condition 4> ou à leur combinaison :
<Condition 3>
R₁₀ dans la Formule 1 est représenté par la Formule 3,
<Condition 4>
R₁₂ dans la Formule 1 est représenté par la Formule 4, 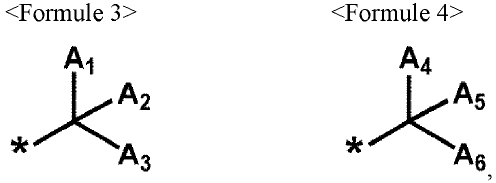
avec dans les Formules 3 et 4,
A₁ à A₆ sont chacun indépendamment :
l'hydrogène, ou le deutérium, ou
un groupe C₁-C₂₀ alkyle, un groupe C₃-C₁₀ cycloalkyle, un groupe C₆-C₆₀ aryle, ou un groupe C₁-C₆₀ hétéroaryle, chacun non substitué ou substitué par le deutérium, un groupe C₁-C₂₀ alkyle, un groupe C₃-C₁₀ cycloalkyle, ou une combinaison quelconque de ceux-ci, et
* indique un site de liaison à un atome voisin ;
de préférence dans lequel
la Formule 3 satisfait à l'une des <Condition 3-1> à <Condition 3-5> et
la Formule 4 satisfait à l'une des <Condition 4-1> à <Condition 4-5> :
<Condition 3-1>
A₁ à A₃ ne sont pas l'hydrogène, simultanément,
<Condition 3-2>
A₁ à A₃ sont chacun indépendamment un groupe C₁-C₂₀ alkyle, ou un groupe C₃-C₁₀ cycloalkyle, chacun non substitué ou substitué par le deutérium, un groupe C₁-C₂₀ alkyle, un groupe C₃-C₁₀ cycloalkyle, ou une combinaison quelconque de ceux-ci,
<Condition 3-3>
A₁ et A₂ sont chacun indépendamment un groupe C₁-C₂₀ alkyle, ou un groupe C₃-C₁₀ cycloalkyle, chacun non substitué ou substitué par le deutérium, un groupe C₁-C₂₀ alkyle, un groupe C₃-C₁₀ cycloalkyle, ou une combinaison quelconque de ceux-ci, et
A₃ est un groupe C₂-C₂₀ alkyle, ou un groupe C₃-C₁₀ cycloalkyle, chacun non substitué ou substitué par le deutérium, un groupe C₁-C₂₀ alkyle, un groupe C₃-C₁₀ cycloalkyle, ou une combinaison quelconque de ceux-ci,
<Condition 3-4>
A₁ est un groupe C₁-C₂₀ alkyle, ou un groupe C₃-C₁₀ cycloalkyle, chacun non substitué ou substitué par le deutérium, un groupe C₁-C₂₀ alkyle, un groupe C₃-C₁₀ cycloalkyle, ou une combinaison quelconque de ceux-ci, et
A₂ et A₃ sont chacun indépendamment un groupe C₂-C₂₀ alkyle, ou un groupe C₃-C₁₀ cycloalkyle, chacun non substitué ou substitué par le deutérium, un groupe C₁-C₂₀ alkyle, un groupe C₃-C₁₀ cycloalkyle, ou une combinaison quelconque de ceux-ci,
<Condition 3-5>
A₁ est l'hydrogène, ou le deutérium,
A₂ est un groupe C₁-C₂₀ alkyle, ou un groupe C₃-C₁₀ cycloalkyle, chacun non substitué ou substitué par le deutérium, un groupe C₁-C₂₀ alkyle, un groupe C₃-C₁₀ cycloalkyle, ou une combinaison quelconque de ceux-ci, et
A₃ est un groupe C₂-C₂₀ alkyle, ou un groupe C₃-C₁₀ cycloalkyle, chacun non substitué ou substitué par le deutérium, un groupe C₁-C₂₀ alkyle, un groupe C₃-C₁₀ cycloalkyle, ou une combinaison quelconque de ceux-ci,
<Condition 4-1>
A₄ à A₆ ne sont pas l'hydrogène, simultanément,
<Condition 4-2>
A₄ à A₆ sont chacun indépendamment un groupe C₁-C₂₀ alkyle, ou un groupe C₃-C₁₀ cycloalkyle, chacun non substitué ou substitué par le deutérium, un groupe C₁-C₂₀ alkyle, un groupe C₃-C₁₀ cycloalkyle, ou une combinaison quelconque de ceux-ci,
<Condition 4-3>
A₄ et A₅ sont chacun indépendamment un groupe C₁-C₂₀ alkyle, ou un groupe C₃-C₁₀ cycloalkyle, chacun non substitué ou substitué par le deutérium, un groupe C₁-C₂₀ alkyle, un groupe C₃-C₁₀ cycloalkyle, ou une combinaison quelconque de ceux-ci, et
A₆ est un groupe C₂-C₂₀ alkyle, ou un groupe C₃-C₁₀ cycloalkyle, chacun non substitué ou
substitué par le deutérium, un groupe C₁-C₂₀ alkyle, un groupe C₃-C₁₀ cycloalkyle, ou une combinaison quelconque de ceux-ci,
<Condition 4-4>
A₄ est un groupe C₁-C₂₀ alkyle, ou un groupe C₃-C₁₀ cycloalkyle, chacun non substitué ou substitué par le deutérium, un groupe C₁-C₂₀ alkyle, un groupe C₃-C₁₀ cycloalkyle, ou une combinaison quelconque de ceux-ci, et
A₅ et A₆ sont chacun indépendamment un groupe C₂-C₂₀ alkyle, ou un groupe C₃-C₁₀ cycloalkyle, chacun non substitué ou substitué par le deutérium, un groupe C₁-C₂₀ alkyle, un groupe C₃-C₁₀ cycloalkyle, ou une combinaison quelconque de ceux-ci,
<Condition 4-5>
A₄ est l'hydrogène ou le deutérium,
A₅ est un groupe C₁-C₂₀ alkyle, ou un groupe C₃-C₁₀ cycloalkyle, chacun non substitué ou substitué par le deutérium, un groupe C₁-C₂₀ alkyle, un groupe C₃-C₁₀ cycloalkyle, ou une combinaison quelconque de ceux-ci, et
A₆ est un groupe C₂-C₂₀ alkyle, ou un groupe C₃-C₁₀ cycloalkyle, chacun non substitué ou substitué par le deutérium, un groupe C₁-C₂₀ alkyle, un groupe C₃-C₁₀ cycloalkyle, ou une combinaison quelconque de ceux-ci.

9. Composé organométallique de l'une quelconque des revendications 1 à 8, dans lequel
au moins un parmi R₁₀ et R₁₂ sont chacun indépendamment un groupe cyclopentyle, un groupe cyclohexyle, un groupe cycloheptyle, un groupe cyclooctyle, un groupe adamantanyle, un groupe norbornanyle, un groupe norbornényle, un groupe cyclopentényle, un groupe cyclohexényle, un groupe cycloheptényle, un groupe bicyclo[1.1.1]pentyle, un groupe bicyclo[2.1.1]hexyle, un groupe bicyclo[2.2.1]heptyle, ou un groupe bicyclo[2.2.2]octyle, chacun non substitué ou substitué par le deutérium, -F, -CD₃, -CD₂H, -CDH₂, -CF₃, -CF₂H, -CFH₂, un groupe cyano, un groupe C₁-C₁₀ alkyle, ou une combinaison quelconque de ceux-ci.

10. Composé organométallique de la revendication 1, le composé organométallique étant l'un des Composés 1 à 22 :

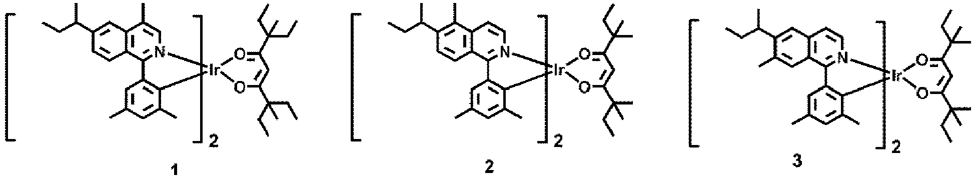

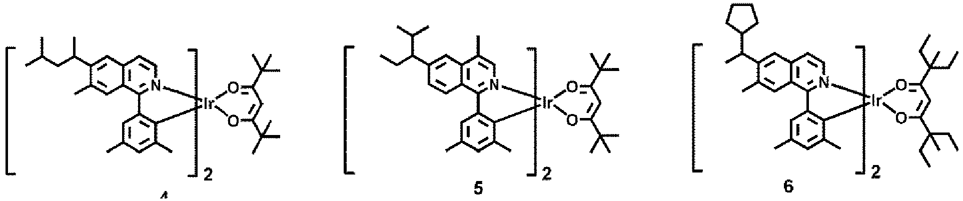

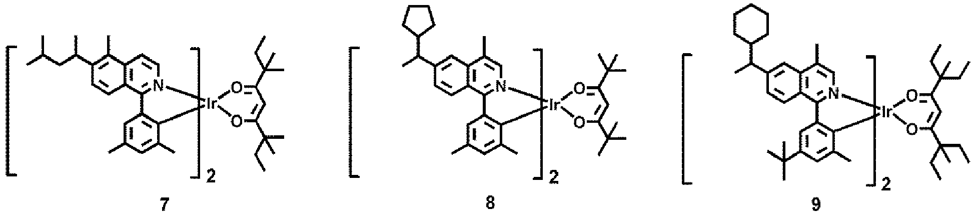

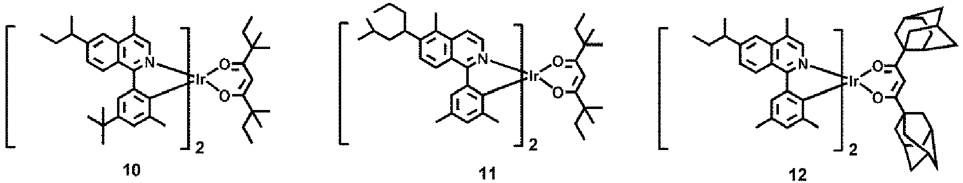

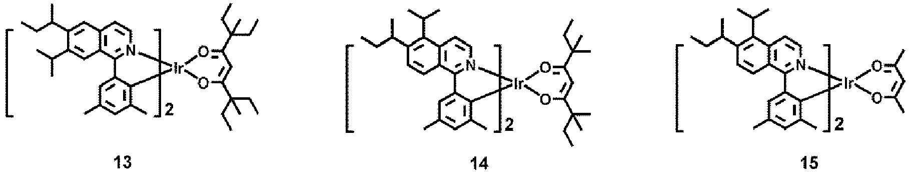

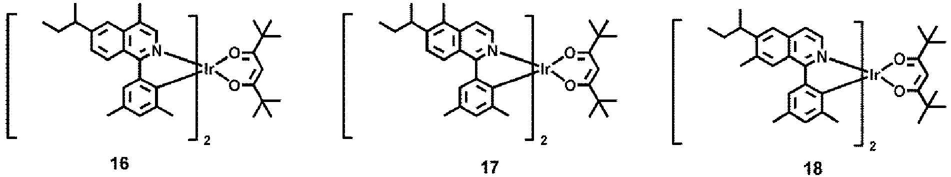

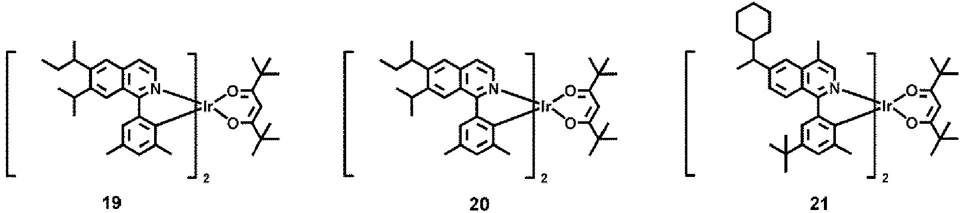

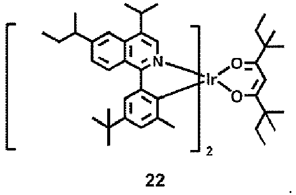

11. Dispositif organique émetteur de lumière comprenant :
une première électrode ;
une seconde électrode ; et
une couche organique disposée entre la première électrode et la seconde électrode et comprenant une couche d'émission,
la couche organique comprenant au moins un composé organométallique de l'une quelconque des revendications 1 à 10.

12. Dispositif organique émetteur de lumière de la revendication 11, dans lequel
la première électrode est une anode,
la seconde électrode est une cathode,
la couche organique comprend en outre une région de transport de trous disposée entre la première électrode et la couche d'émission et une région de transport d'électrons disposée entre la couche d'émission et la seconde électrode,
la région de transport par trous comprend une couche d'injection de trous, une couche de transport de trous, une couche de blocage d'électrons, une couche tampon ou une combinaison quelconque de celles-ci, et
la région de transport d'électrons comprend une couche de blocage de trous, une couche de transport d'électrons, une couche d'injection d'électrons, ou une combinaison quelconque de celles-ci.

13. Dispositif organique émetteur de lumière des revendications 11 ou 12, dans lequel la
couche d'émission comprend le composé organométallique.

14. Dispositif organique émetteur de lumière de la revendication 13, dans lequel la couche d'émission émet de la lumière rouge ; et/ou
dans lequel la couche d'émission comprend en outre un hôte, et une quantité de l'hôte dans la couche d'émission est plus grande qu'une quantité du composé organométallique dans la couche d'émission.

15. Composition diagnostique comprenant au moins un composé organométallique de l'une quelconque des revendications 1 à 10.
